(19) **Europäisches Patentamt** / **European Patent Office** / **Office européen des brevets**

(11) **EP 4 246 123 B1**

(12) **EUROPEAN PATENT SPECIFICATION**

(45) Date of publication and mention
of the grant of the patent:
**19.11.2025 Bulletin 2025/47**

(21) Application number: **23162048.5**

(22) Date of filing: **15.03.2023**

(51) International Patent Classification (IPC):
**G01N 15/14** (2024.01)   *G01N 15/00* (2024.01)

(52) Cooperative Patent Classification (CPC):
**G01N 15/1429; G01N 15/1459;** G01N 15/149;
G01N 2015/012; G01N 2015/016; G01N 2015/018;
G01N 2015/1402; G01N 2015/1486;
G01N 2015/1488

(54) **SPECIMEN ANALYZER, SPECIMEN ANALYSIS METHOD, AND PROGRAM**

PROBENANALYSEGERÄT, PROBENANALYSEVERFAHREN UND PROGRAMM

ANALYSEUR D'ÉCHANTILLON, PROCÉDÉ D'ANALYSE D'ÉCHANTILLON ET PROGRAMME

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **17.03.2022 JP 2022042964**
**17.03.2022 JP 2022042965**

(43) Date of publication of application:
**20.09.2023 Bulletin 2023/38**

(73) Proprietor: **SYSMEX CORPORATION**
**Kobe-shi**
**Hyogo 651-0073 (JP)**

(72) Inventors:
• **KIMURA, Konobu**
**Kobe-shi, Hyogo, 651-0073 (JP)**
• **SUZUKI, Kenichiro**
**Kobe-shi, Hyogo, 651-0073 (JP)**
• **NAKANISHI, Noriyuki**
**Kobe-shi, Hyogo, 651-0073 (JP)**

(74) Representative: **Hoffmann Eitle**
**Patent- und Rechtsanwälte PartmbB**
**Arabellastraße 30**
**81925 München (DE)**

(56) References cited:
**EP-A1- 3 441 762**      **EP-A2- 2 902 769**
**US-A1- 2022 003 745**

**Description**

FIELD OF THE INVENTION

The present invention relates to a specimen analyzer, a specimen analysis method, and a program that analyze a specimen.

BACKGROUND OF THE INVENTION

International Publication No. WO2018/203568 discloses a method in which: signals obtained by measuring cells by a flow cytometer are analyzed by an artificial intelligence algorithm; and the cells are classified according to the types.

SUMMARY OF THE INVENTION

When data is processed by using an artificial intelligence algorithm, the load on a computer that processes the data is increased in accordance with increase in the volume of the data. For example, when the amount of information to be used in classification of components (e.g., cells or particles) in a specimen is increased in order to improve classification accuracy, in a case of a specimen such as blood or urine that contains a plurality of components, the data volume per specimen increases due to increase in the amount of information obtained from a single component. When the number of specimens to be tested has been increased as well, the data volume increases. International Publication No. WO2018/203568 does not disclose any technology that can reduce the load on a computer at the time when data is processed by using an artificial intelligence algorithm. Further, US 2022/003745 A1 relates to a cell analysis method using a trained deep learning algorithm.

In view of the above problem, an object of the present invention is to provide a specimen analyzer, a specimen analysis method, and a program that can reduce the load on a computer that analyzes, according to an artificial intelligence algorithm, data obtained through measurement of a specimen. This problem is solved by the subject-matter of the independent claims. The dependent claims describe further preferred embodiments, and this description exemplifies how the present invention may be carried out.

A specimen analyzer (4000) of the present invention is defined in claim 1.

According to the specimen analyzer of the present invention, the analysis process performed on data that corresponds to the optical signal obtained from the specimen is apportioned between the first analysis operation according to the artificial intelligence algorithm and the second and third analysis operations of processing the first and second representative values that each correspond to a feature of the analyte. Therefore, when compared with a case where the data that corresponds to the optical signal is all analyzed by using only the artificial intelligence algorithm, the load on the analysis unit being a computer that processes the data can be reduced.

In addition, analysis of the second measurement item with respect to the first measurement sample is executed through at least one of the first analysis operation according to the artificial intelligence algorithm, and the second analysis operation of processing the first representative value that corresponds to a feature of the analyte. Accordingly, for example, even when analysis of the second measurement item is difficult to be performed through the second analysis operation, a highly accurate analysis can be performed through the first analysis operation as appropriate.

Since the plurality of first sample preparation parts are provided, a plurality of first measurement samples can be prepared in parallel on the basis of a plurality of specimens. Accordingly, the throughput of the specimen analysis can be improved.

A specimen analysis method of the present invention is defined in claim 23.

According to the specimen analysis method of the present invention, the analysis process performed on data that corresponds to the optical signal obtained from the specimen is apportioned between the first analysis operation according to the artificial intelligence algorithm and the second and third analysis operations of processing the first and second representative values that each correspond to a feature of the analyte. Therefore, when compared with a case where the data that corresponds to the optical signal is all analyzed by using only the artificial intelligence algorithm, the load on the computer that processes the data can be reduced.

In addition, analysis of the second measurement item with respect to the first measurement sample is executed through at least one of the first analysis operation according to the artificial intelligence algorithm, and the second analysis operation of processing the first representative value that corresponds to a feature of the analyte. Accordingly, for example, even when analysis of the second measurement item is difficult to be performed through the second analysis operation, a highly accurate analysis can be performed through the first analysis operation as appropriate.

A program of the present invention is defined in claim 24.

According to the program of the present invention, the analysis process performed on data that corresponds to the optical signal obtained from the specimen is apportioned between the first analysis operation according to the artificial intelligence algorithm and the second and third analysis operations of processing the first and second representative values that each correspond to a feature of the analyte. Therefore, when compared with a case where the data that corresponds to the optical signal is all analyzed by using only the artificial intelligence algorithm, the load on the computer that processes the data can be reduced.

In addition, analysis of the second measurement item with respect to the first measurement sample is executed through at least one of the first analysis operation according to the artificial intelligence algorithm, and the second analysis operation of processing the first representative value that corresponds to a feature of the analyte. Accordingly, for example, even when analysis of the second measurement item is difficult to be performed through the second analysis operation, a highly accurate analysis can be performed through the first analysis operation as appropriate.

According to the present invention, the load on the computer that analyzes, according to an artificial intelligence algorithm, data obtained through measurement of a specimen can be reduced.

BRIEF DESCRIPTION OF THE DRAWINGS

FIG. 1 schematically shows a configuration example of a specimen analyzer according to Embodiment 1;
FIG. 2 shows an outline of an analysis in a case where an optical detection part is a detection part based on flow cytometry, according to Embodiment 1;
FIG. 3 schematically shows waveform data and representative values according to Embodiment 1;
FIG. 4 shows an outline of an analysis in a case where an optical detection part is a detection part that detects transmitted light or scattered light from a measurement sample, according to Embodiment 1;
FIG. 5 is a flowchart showing an example of a specimen analysis method according to Embodiment 1;
FIG. 6 is a flowchart showing an example in which an analysis operation is set on the basis of a rule set to an analysis unit, according to Embodiment 2;
FIG. 7 is a flowchart showing an example in which analysis is executed in accordance with a measurement item, according to Embodiment 3;
FIG. 8 is an exemplary drawing schematically showing a screen for setting an AI analysis or a calculation processing analysis for each measurement item, according to Embodiment 3;
FIG. 9 is a flowchart showing an example in which analysis is executed in accordance with a measurement order, according to Embodiment 3;
FIG. 10 is an exemplary drawing schematically showing a screen for setting an analysis mode for a measurement order, according to Embodiment 3;
FIG. 11 is a flowchart showing an example in which analysis is executed in accordance with an analysis mode of the apparatus, according to Embodiment 3;
FIG. 12 is an exemplary drawing schematically showing a screen for setting an analysis mode of the analysis unit, according to Embodiment 3;
FIG. 13 is a flowchart showing an example in which analysis is executed in accordance with the type of a measurement order, according to Embodiment 3;
FIG. 14 is an exemplary drawing schematically showing a screen for setting an analysis mode for each type of measurement order, according to Embodiment 3;
FIG. 15 is a flowchart showing an example in which analysis is executed in accordance with a measurement item and the type of a measurement order, according to Embodiment 3;
FIG. 16 is an exemplary drawing schematically showing a screen for setting the AI analysis or the calculation processing analysis for each type of measurement item and a measurement order, according to Embodiment 3;
FIG. 17 is a flowchart showing an example in which whether or not the AI analysis is necessary is determined on the basis of a flag provided through the calculation processing analysis, according to Embodiment 3;
FIG. 18 is an exemplary drawing schematically showing a screen for setting the AI analysis for each flag of an analysis result, according to Embodiment 3;
FIG. 19 is a flowchart showing an example in which the AI analysis is executed with respect to a specific analyte classified through the calculation processing analysis, according to Embodiment 3;
FIG. 20 is an exemplary drawing schematically showing a screen for setting whether or not to perform the AI analysis for each type of analyte, according to Embodiment 3;
FIG. 21 is a diagram describing a classification method according to the calculation processing analysis and the AI analysis executed in the process shown in FIG. 19, according to Embodiment 3;
FIG. 22 is a flowchart showing an example in which the AI analysis is executed when a specific classification has been performed in the calculation processing analysis, according to Embodiment 3;
FIG. 23 is an exemplary drawing schematically showing a screen for setting whether or not to perform the AI analysis with respect to an analyte of a specific type, according to Embodiment 3;

FIG. 24 is a block diagram showing a configuration of a measurement unit according to Embodiment 4;

FIG. 25 schematically shows a configuration of an optical system of an FCM detection part, according to Embodiment 4;

FIG. 26 is a block diagram showing a configuration of the analysis unit according to Embodiment 4;

FIG. 27 is a block diagram showing a configuration of the measurement unit in a case where the specimen analyzer executes counting and classification of blood cells in a blood specimen, according to Embodiment 4;

FIG. 28 is a block diagram showing a configuration of a specimen suction part and a sample preparation part in the measurement unit in FIG. 27, according to Embodiment 4;

FIG. 29 is a block diagram showing another configuration of the sample preparation part shown in FIG. 28, according to Embodiment 4;

FIG. 30 is a flowchart showing an example in which analysis is executed in accordance with a measurement channel, according to Embodiment 4;

FIG. 31 is an exemplary drawing schematically showing a screen for setting the AI analysis or the calculation processing analysis for each measurement channel, according to Embodiment 4;

FIG. 32 is a schematic diagram for describing waveform data to be used in an analysis method, according to Embodiment 4;

FIG. 33 is a schematic diagram showing an example of a generation method of training data to be used for training an AI algorithm for determining the type of an analyte in a specimen, according to Embodiment 4;

FIG. 34 shows a label value that corresponds to a cell type, according to Embodiment 4;

FIG. 35 schematically shows a method for analyzing waveform data of an analyte in a specimen by the AI algorithm, according to Embodiment 4;

FIG. 36 is a flowchart showing an example in which the AI analysis is executed on waveform data obtained in a WDF channel, according to Embodiment 4;

FIG. 37 is a flowchart showing an example in which, on the basis of waveform data obtained in the WDF channel, nucleated red blood cells and basophils are classified through the AI analysis, and the others are classified through the calculation processing analysis, according to Embodiment 4;

FIG. 38 is a flowchart showing an example in which the AI analysis is executed with respect to neutrophils/basophils specified through the calculation processing analysis in the WDF channel, according to Embodiment 4;

FIG. 39 is a block diagram schematically showing a configuration of the measurement unit, according to Embodiment 5;

FIG. 40 is a side view schematically showing measurement performed by a detection block, according to Embodiment 5;

FIG. 41 is a flowchart showing an analysis example according to Embodiment 5;

FIG. 42 is a block diagram showing a configuration of the specimen analyzer according to Embodiment 6;

FIG. 43 is a block diagram showing a configuration of the analysis unit according to Embodiment 6;

FIG. 44 is a block diagram showing another configuration of the specimen analyzer according to Embodiment 6;

FIG. 45 shows a configuration example of a parallel-processing processor according to Embodiment 6;

FIG. 46 schematically shows an installation example of the parallel-processing processor according to Embodiment 6;

FIG. 47 schematically shows an installation example of the parallel-processing processor according to Embodiment 6;

FIG. 48 schematically shows an installation example of the parallel-processing processor according to Embodiment 6;

FIG. 49 shows another installation example of the parallel-processing processor according to Embodiment 6;

FIG. 50 shows a configuration example of the parallel-processing processor which executes arithmetic processes, according to Embodiment 6;

FIG. 51 shows an outline of a matrix operation executed by the parallel-processing processor, according to Embodiment 6;

FIG. 52 is a conceptual diagram showing that a plurality of arithmetic processes are executed in parallel by the parallel-processing processor, according to Embodiment 6;

FIG. 53 schematically shows an outline of an arithmetic process regarding a convolution layer, according to Embodiment 6;

FIG. 54 is a flowchart showing analysis operations performed by the analysis unit and the measurement unit, according to Embodiment 6;

FIG. 55 is a flowchart showing details of the AI analysis in step S201 in FIG. 54, according to Embodiment 6;

FIG. 56 is a flowchart showing details of step S2011 in FIG. 55, according to Embodiment 6;

FIG. 57 is a block diagram showing another configuration of the measurement unit according to Embodiment 6;

EP 4 246 123 B1

FIG. 58 is a block diagram showing another configuration of the analysis unit according to Embodiment 6;

FIG. 59 is a block diagram showing another configuration of the measurement unit according to Embodiment 6;

FIG. 60 is a block diagram showing another configuration of the analysis unit according to Embodiment 6;

FIG. 61 is a block diagram showing another configuration of the specimen analyzer according to Embodiment 6;

FIG. 62 is a block diagram showing another configuration of the measurement unit according to Embodiment 6;

FIG. 63 is a block diagram showing another configuration of the analysis unit according to Embodiment 6;

FIG. 64 shows a configuration example of the parallel-processing processor which executes arithmetic processes, according to Embodiment 6;

FIG. 65 is a block diagram showing a configuration of a computer according to Embodiment 6;

FIG. 66 is a block diagram showing another configuration of the measurement unit according to Embodiment 6;

FIG. 67 is a block diagram showing another configuration of the analysis unit according to Embodiment 6;

FIG. 68 is a block diagram showing another configuration of the measurement unit according to Embodiment 6;

FIG. 69 is a block diagram showing another configuration of the analysis unit according to Embodiment 6;

FIG. 70 is a block diagram showing another configuration of the measurement unit according to Embodiment 6;

FIG. 71 is a block diagram showing another configuration of the analysis unit according to Embodiment 6;

FIG. 72 is a block diagram showing another configuration of the measurement unit according to Embodiment 6;

FIG. 73 is a block diagram showing another configuration of the analysis unit according to Embodiment 6;

FIG. 74 schematically shows a configuration of a waveform data analysis system according to Embodiment 7;

FIG. 75 is a block diagram showing a configuration of a deep learning apparatus according to Embodiment 7;

FIG. 76 is a function block diagram of the deep learning apparatus according to Embodiment 7;

FIG. 77 is a flowchart showing a process performed by the deep learning apparatus according to Embodiment 7; and

FIG. 78 is a schematic diagram showing an example of the structure of a neural network, a schematic diagram showing an arithmetic operation at each node, and a schematic diagram showing an arithmetic operation between nodes, according to Embodiment 7.

DETAILED DESCRIPTION

Hereinafter, outlines and embodiments of the disclosure will be described in detail with reference to attached drawings. In the following description and drawings, the same reference characters denote the same or similar components, and description of the same or similar components will be omitted for convenience.

[Embodiment 1]

The present embodiment discloses a specimen analyzer, a specimen analysis method, and a program that can execute both of analysis according to an artificial intelligence algorithm (AI algorithm) and analysis that does not use the AI algorithm, on data obtained through measurement of a specimen.

In the analysis by the AI algorithm, data is analyzed by a large number of matrix operation processes, for example. Hereinafter, analysis by the AI algorithm will be referred to as "AI analysis", for convenience. In the AI analysis, a convolution operation according to the AI algorithm is performed, for example.

In the analysis that does not use the AI algorithm, data is analyzed through calculation processing with respect to a representative value that corresponds to a feature of an analyte, for example. Hereinafter, an analysis method that analyzes data through calculation processing with respect to a representative value that corresponds to a feature of an analyte, without using the AI algorithm, will be referred to as "calculation processing analysis" or "non-AI analysis", for convenience. The representative value that is processed in the calculation processing analysis has a data amount smaller than that of data that is inputted to the AI algorithm in the AI analysis. In the calculation processing analysis, the data amount to be processed and the amount of arithmetic operation processing are smaller than those of the AI analysis, and thus, the load on the computer that performs analysis is smaller than that in the AI analysis. Accordingly, the TAT (Turn Around Time) of analysis of a measurement result can be shortened.

According to the specimen analyzer, the specimen analysis method, and the program of Embodiment 1, analysis of data obtained through measurement of a specimen is apportioned between and executed by the AI analysis and the calculation processing analysis, whereby the load on the computer that performs the analysis can be reduced.

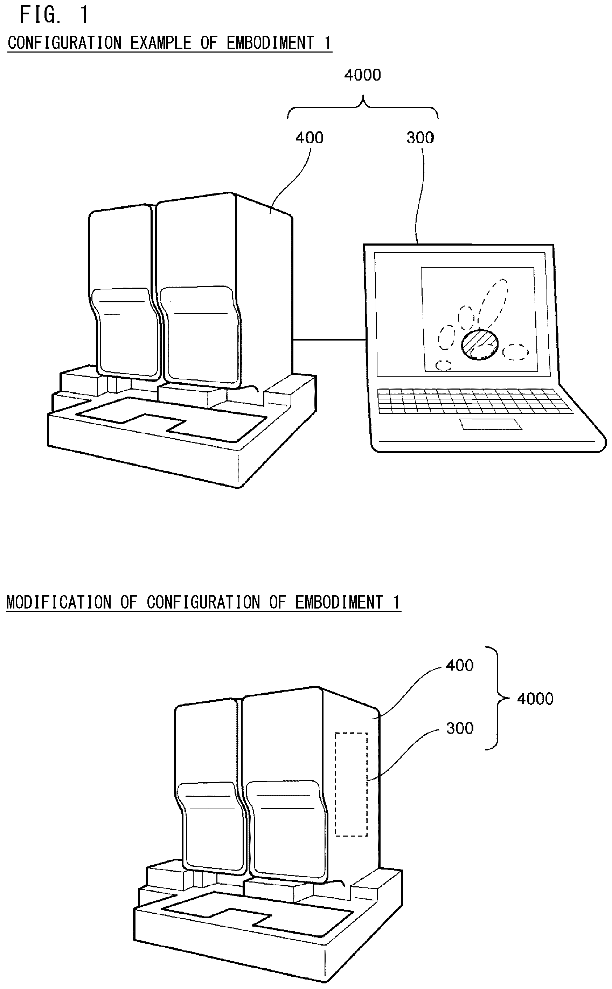

FIG. 1 schematically shows a configuration example of a specimen analyzer 4000 of Embodiment 1. In FIG. 1, the drawing in the upper part shows a configuration example of the specimen analyzer 4000 of the embodiment, and the drawing in the lower part shows a modification of the configuration of Embodiment 1.

As shown in the drawing in the upper part of FIG. 1, the specimen analyzer 4000 of Embodiment 1 includes a measurement unit 400 and an analysis unit 300, for example. As shown in the drawing in the lower part of FIG. 1, the specimen analyzer 4000 may be implemented by the measurement unit 400 and the analysis unit 300 that are integrally formed. The specimen analyzer 4000 is, for example, a blood cell analyzer, a urine analyzer, a blood coagulation measurement apparatus, an immunoassay apparatus, a biochemical measurement apparatus, a gene measurement apparatus, or the like. An analyte to serve as an analysis target of the specimen analyzer 4000 is, for example, a cell, a particle, a protein, a gene, or the like.

The measurement unit 400 measures a specimen and obtains data regarding the specimen. The analysis unit 300 analyzes the data obtained by the measurement unit 400. The analysis unit 300 may have a function of performing control for setting a measurement condition of a measurement sample and for execution of measurement in the measurement unit 400. The analysis unit 300 is implemented as an apparatus (e.g., computer) different from the measurement unit 400 and is connected to the measurement unit 400. The analysis unit 300 and the measurement unit 400 are connected to each other in a wired or wireless manner.

The measurement unit 400 includes an optical detection part for measuring a measurement sample prepared from a specimen.

The optical detection part is a detection part based on flow cytometry, for example, and is used in measurement of a blood specimen or a urine specimen. The optical detection part applies light to a measurement sample flowing in a flow cell, thereby obtaining optical signals. For example, the optical detection part applies light to a measurement sample containing an analyte (e.g., cell or particle) flowing in the flow cell, thereby causing forward scattered light, side scattered light, and fluorescence to be generated from the analyte. A photodetector provided to the optical detection part receives the lights having been generated, and outputs optical signals corresponding to the intensities of the received lights. The optical signals are analog signals having waveform shapes that correspond to temporal changes in forward scattered light, side scattered light, and fluorescence. An A/D converter provided to the optical detection part performs digital conversion on each optical signal to obtain digital data (hereinafter referred to as "waveform data") having a waveform shape that corresponds to each of analytes. For example, the waveform data in this case is used for classification of the types of white blood cells in a blood specimen, classification of the numbers of red blood cells and white blood cells in a blood specimen, and classification of particles in a urine specimen, and the like.

The optical detection part may be configured to apply light to a measurement sample contained in a container and to detect, by means of a photodetector, light transmitted through the measurement sample or scattered light scattered by the measurement sample. In this case, the optical detection part applies light to a measurement sample containing an analyte and left to stand in a state of being contained in the container. The photodetector provided to the optical detection part receives, for a predetermined period, transmitted light transmitted through the measurement sample or scattered light generated from the measurement sample, and outputs an optical signal that corresponds to the intensity of the received light. The optical signal in this case is an analog signal having a waveform shape that corresponds to change over time of the transmitted light or the scattered light associated with coagulation of the measurement sample. An A/D converter provided to the optical detection part performs digital conversion on the optical signal and obtains digital data (hereinafter, referred to as "coagulation waveform data") having a waveform shape that corresponds to change over time of the transmitted light or the scattered light. The coagulation waveform data in this case is used in, for example, analysis or the like of coagulability of the blood specimen.

Next, an example of analysis performed by the analysis unit 300 by using the data obtained by the measurement unit 400 will be described.

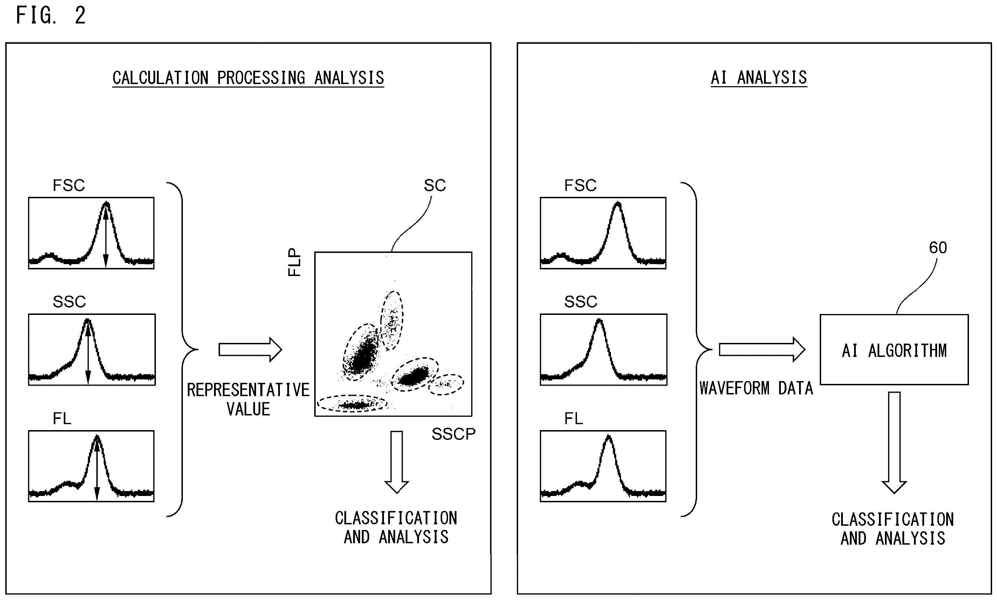

FIG. 2 shows an outline of analysis in a case where the optical detection part is a detection part based on flow cytometry.

In FIG. 2, the left drawing shows an outline of the calculation processing analysis, and the right drawing shows an outline of the AI analysis. FSC, SSC, and FL in FIG. 2 respectively show optical signals that correspond to the forward scattered light intensity, the side scattered light intensity, and the fluorescence obtained by the optical detection part of the measurement unit 400.

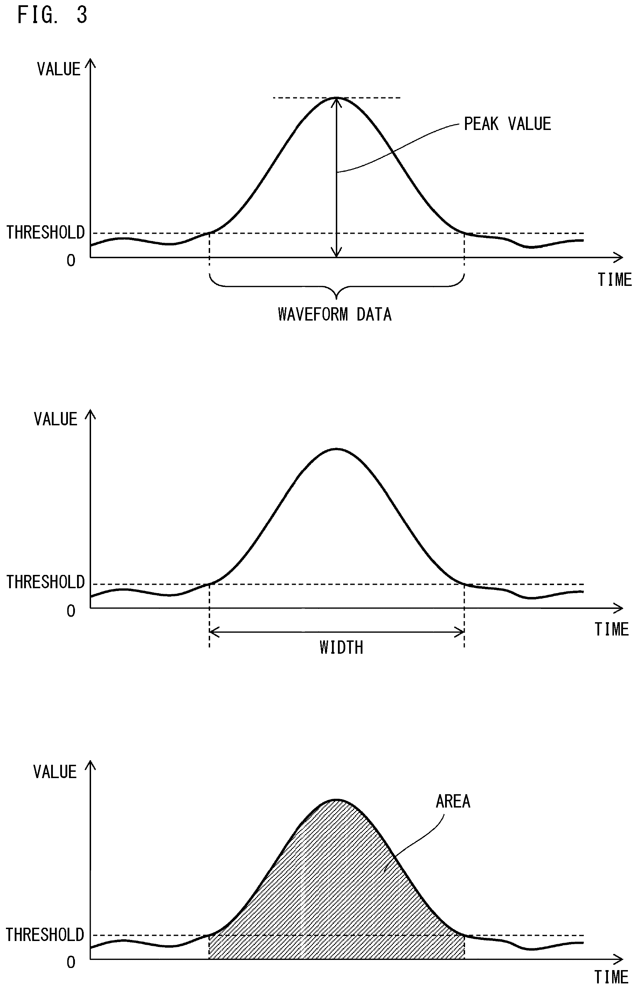

As shown in the graph in the upper part of FIG. 3, the measurement unit 400 specifies, in the digital data obtained through digital conversion of the optical signal, a region having a value greater than a predetermined threshold, as a region corresponding to an analyte in the specimen. The region having a value greater than the threshold of the digital data obtained through digital conversion of the optical signal is a region corresponding to each of analytes in the specimen. Each graph of FIG. 3 schematically shows a region (e.g., the region of the "waveform data" in the graph in the upper part of FIG. 3) that corresponds to one analyte in the specimen and that has been specified in the digital data. It should be noted that specifying a region having a value greater than the predetermined threshold may be performed on the optical signal.

The measurement unit 400 obtains, as waveform data, the region that corresponds to each of the analytes in the specimen, from the digital data obtained through digital conversion of the optical signal. The waveform data is obtained so as to correspond to a plurality of analytes in the specimen. Through calculation processing, the analysis unit 300 calculates a representative value, of the waveform data, that corresponds to a feature of the analyte. As shown in each graph in FIG. 3, the analysis unit 300 calculates, as a representative value, an amount such as the peak value, the width, or the area of the waveform data, for example. The peak value is the maximum value of the waveform data, the width is the width in the time axis direction of the waveform data, and the area is the area surrounded by the waveform data.

In the calculation processing analysis, a representative value corresponding to a feature of each analyte is determined in advance. For example, in a case where classification and counting of blood cells being an analyte are to be performed, the representative value determined in advance in the algorithm of the calculation processing analysis is the peak value. The analysis unit 300 obtains from the waveform data a representative value determined in advance, through a predetermined calculation, and processes the representative value obtained for analyzing the analyte. With respect to each of a plurality of pieces of the waveform data obtained by the measurement unit 400, the analysis unit 300 obtains a representative value determined in advance. That is, through the predetermined calculation by the analysis unit 300, representative values (e.g., peak value) of an identical type are obtained from the plurality of respective pieces of the waveform data. The representative value determined in advance may be obtained by the measurement unit 400 and the obtained representative value and the waveform data may be transmitted to the analysis unit 300.

Meanwhile, in the AI analysis, since the AI algorithm extracts a feature of the waveform data, the representative value is not determined in advance. Since the feature (i.e., a feature corresponding to an analyte) of the waveform data extracted by the AI algorithm may vary in accordance with the learning content of the AI algorithm, it is not necessary to determine in advance a representative value in the AI analysis. Since the AI algorithm can extract various features of the waveform data in accordance with the learning content, not only the representative value but also the waveform data itself is inputted to the AI algorithm. Since the waveform data itself is inputted to the AI algorithm, the computer load for arithmetic operations of data is increased in the AI analysis and the TAT (Turn Around Time) required in the arithmetic operations is also increased, when compared with the calculation processing analysis.

As shown in the left drawing in FIG. 2, in the calculation processing analysis, the analysis unit 300 obtains representative values from the waveform data obtained so as to correspond to each analyte, and generates, for example, a scattergram SC on the basis of the obtained representative values. In the scattergram SC shown as an example in FIG. 2, SSCP in the horizontal axis represents the peak value of the waveform data based on side scattered light, and FLP in the vertical axis represents the peak value of the waveform data based on fluorescence. On the scattergram SC, a plurality of analytes are plotted. On the basis of the scattergram SC, the analysis unit 300 executes classification and analysis of each analyte in the specimen.

As shown in the right drawing in FIG. 2, in the AI analysis, the analysis unit 300 inputs waveform data that corresponds to each analyte into an AI algorithm 60, and executes classification and analysis of the analyte in the specimen. The AI algorithm 60 is a learned AI algorithm, and is generated by inputting the waveform data as described above into an AI algorithm before being trained, to learn. The representative value obtained in the calculation processing analysis has a data amount smaller than that of the waveform data inputted to the AI algorithm in the AI analysis.

The types of the analytes classified through the calculation processing analysis and the AI analysis are, for example, the types of blood cells in a blood specimen, the types of particles in a urine specimen, and the like. For example, the analysis unit 300 executes the AI analysis with respect to measurement items for classifying the types of white blood cells in a blood specimen, and executes the calculation processing analysis with respect to the other measurement items.

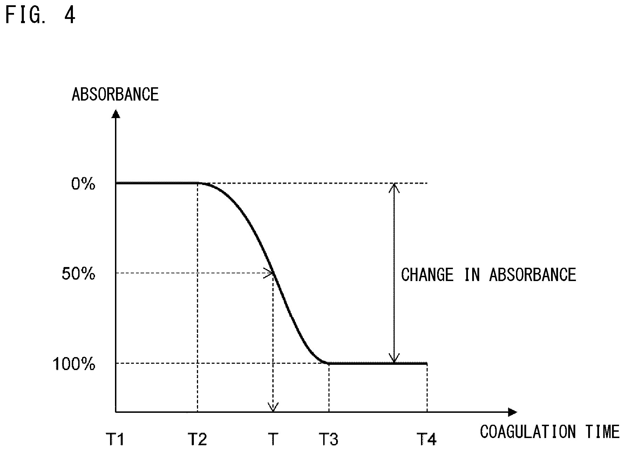

FIG. 4 shows an outline of an analysis in a case where the optical detection part is a detection part that detects transmitted light or scattered light from a measurement sample.

The measurement unit 400 obtains, as coagulation waveform data, digital data obtained through digital conversion of an optical signal. In a single measurement, one piece of coagulation waveform data is obtained from one measurement sample.

The graph in FIG. 4 is an example of coagulation waveform data based on transmitted light detected through application of light to a measurement sample. The horizontal axis represents elapsed time and the vertical axis represents absorbance. Absorbance is a value that indicates how much the light applied to a measurement sample is absorbed by the measurement sample. A state where the absorbance is 0% indicates a state where substantially all of the light applied to the measurement sample has reached the photodetector, and a state where the absorbance is 100% indicates a state where substantially none of the light applied to the measurement sample has reached the photodetector.

Instead of absorbance, transmitted light intensity may be used. In this case, if the percentage (transmitted light intensity) on the vertical axis is set so as to be increased upwardly, the coagulation waveform data has a shape in which the graph decreases in association with a lapse of time, as in FIG. 4.

The coagulation waveform data includes, at least, data that corresponds to the optical signal obtained from a timing T2 that indicates start of coagulation of the specimen to a timing T3 that indicates end of coagulation of the specimen. The coagulation waveform data may include data that corresponds to the optical signal obtained from a start timing T1 of the light measurement to an end timing T4 of the light measurement by the measurement unit 400.

In the calculation processing analysis, the analysis unit 300 calculates, through calculation processing, a representative value, of the coagulation waveform data, that corresponds to a feature of an analyte, and executes analysis on the basis of the calculated representative value. In the calculation processing analysis, the analysis unit 300 specifies, as a representative value, coagulation waveform data at a time when the intensity of the detected light satisfies a predetermined condition. For example, the analysis unit 300 obtains, as a representative value, a time (T-T2) required for the absorbance of the coagulation waveform data to decrease to a predetermined value (e.g., 50%), and provides the obtained representative value, as a result that indicates the time taken for the blood specimen to coagulate.

In the AI analysis, the analysis unit 300 analyzes the coagulation waveform data on the basis of the AI algorithm 60 (see FIG. 2). The analysis unit 300 obtains, for example, the presence or absence of an abnormality regarding the measurement, on the basis of a feature amount extracted by the AI algorithm 60 from the coagulation waveform data. The analysis unit 300 determines whether or not there is a suspected occurrence of nonspecific reaction, on the basis of the presence or absence of an abnormality regarding the measurement.

For example, the analysis unit 300 analyzes the presence or absence of an abnormality due to an interference substance in the blood specimen. As a specific example, the analysis unit 300 analyzes the presence or absence of an abnormality by using coagulation waveform data regarding PT (prothrombin time), which is an item for measuring coagulability regarding prothrombin being a coagulation factor.

It should be noted that, in the AI analysis, the analysis unit 300 may input coagulation waveform data into the AI algorithm 60 and obtain the time taken for the blood specimen to coagulate. Alternatively, in the AI analysis, the analysis unit 300 may input coagulation waveform data to the AI algorithm 60 and obtain a cause for prolongation in a case where the coagulation time has been prolonged.

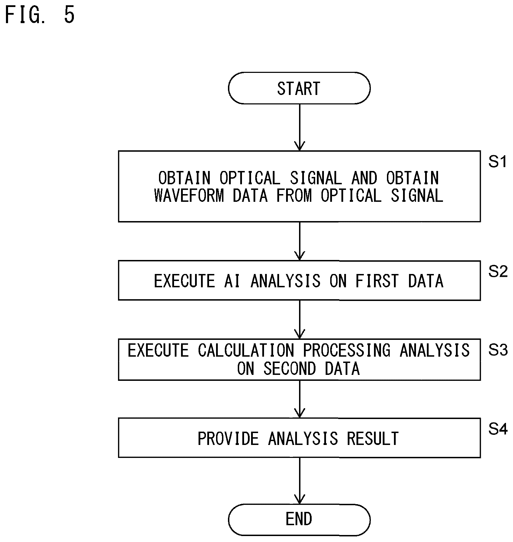

FIG. 5 is a flowchart showing an example of a specimen analysis method of Embodiment 1.

In step S1, the measurement unit 400 obtains an optical signal by means of the optical detection part, and obtains waveform data from the obtained optical signal.

In step S2, the analysis unit 300 executes the AI analysis on waveform data (first data) to serve as a target of the AI analysis, out of the waveform data obtained by the measurement unit 400. For example, the analysis unit 300 specifies, as the first data, waveform data that corresponds to a measurement item being a target of the AI analysis, and executes the AI analysis on the specified first data.

In step S3, the analysis unit 300 executes the calculation processing analysis on waveform data (second data) to serve as a target the calculation processing analysis, out of the waveform data obtained by the measurement unit 400. For example, the analysis unit 300 specifies, as the second data, waveform data that corresponds to a measurement item being a target of the calculation processing analysis, and executes the calculation processing analysis on the specified second data.

With respect to steps S2 and S3 above, an example case where measurement of classifying the types of white blood cells in a blood specimen is the target of the AI analysis will be described. For example, the measurement unit 400 prepares a blood specimen by using a reagent that corresponds to measurement of white blood cell classification, and measures the prepared measurement sample by means of an optical detection part based on flow cytometry. Since the measurement regarding white blood cell classification is a target of the AI analysis, the analysis unit 300 specifies, as the first data, waveform data based on the measurement sample for white blood cell classification, for example. The analysis unit 300 analyzes the first data by means of the AI algorithm 60, and classifies white blood cells. Meanwhile, for example, the analysis unit 300 specifies, as the second data, waveform data based on a measurement sample other than that for white blood cell classification. The analysis unit 300 specifies a representative value that corresponds to a feature of each analyte from the second data, executes the calculation processing analysis of processing the specified representative value, and classifies blood cells other than white blood cells.

In step S4, the analysis unit 300 provides analysis results obtained in steps S2 and S3. In step S4, for example, the analysis unit 300 performs display of the analysis results on a display part, transmission of the analysis results to another computer, and the like.

It should be noted that, in step S1, the measurement unit 400 may obtain the optical signal by means of the optical detection part from a single measurement sample, and may obtain waveform data from the obtained optical signal. In this case, the first data and the second data are each composed of a plurality of pieces of data, and a part thereof may be the same data between the first data and the second data.

Further, in step S1, from each of a plurality of measurement samples containing a specimen collected from an identical subject, an optical signal may be obtained by the optical detection part, and from each of the obtained optical signals, waveform data may be obtained. In this case, the analysis unit 300 executes, in step S2, the AI analysis on the waveform data (first data) obtained from one measurement sample, and executes, in step S3, the calculation processing analysis on the waveform data (second data) obtained from another measurement sample. The plurality of measurement samples containing a specimen collected from an identical subject may be prepared by using a reagent of the same type with each other, or may be prepared by using reagents of different types from each other.

Further, in step S1, from each of a plurality of measurement samples respectively containing specimens collected from subjects different from each other, an optical signal may be obtained by the optical detection part, and from each of the obtained optical signals, waveform data may be obtained. In this case, the analysis unit 300 executes, in step S2, the AI analysis on the waveform data (first data) obtained from one measurement sample, and executes, in step S3, the calculation processing analysis on the waveform data (second data) obtained from another measurement sample. The plurality of measurement samples respectively containing specimens collected from subjects different from each other may be prepared by using a reagent of the same type with each other, or may be prepared by using reagents of different types from each other.

In Embodiment 1 above, as the calculation processing analysis, in step S3, the analysis unit 300 specifies a representative value that corresponds to a feature of an analyte from the second data, and processes the specified representative value. However, the present disclosure is not limited thereto. For example, in step S1, the measurement unit 400 may obtain a representative value from the waveform data, and output the waveform data and the representative value to the analysis unit 300, and as the calculation processing analysis, in step S3, the analysis unit 300 may process the representative value obtained from the measurement unit 400.

[Embodiment 2]

In Embodiment 2, the AI analysis and the calculation processing analysis are selected on the basis of a rule set to the analysis unit 300.

The rule for selecting an analysis operation is set by a user via the analysis unit 300, for example. The user can set, to the analysis unit 300, a rule according to an operation policy of a laboratory, for example. Accordingly, in accordance with the operation policy of the laboratory, apportioning between the AI analysis and the calculation processing analysis can be changed as appropriate.

Since the rule for the analysis operation can be set, apportioning between the AI analysis and the calculation processing analysis can be flexibly changed while the load on the analysis unit 300 is reduced. For example, when the accuracy of the AI analysis has been improved as a result of causing the AI algorithm 60 to additionally learn, it is possible to set the rule such that data to serve as the target of the AI analysis is increased. Further, for example, when shortening of the TAT (Turn Around Time) of analysis of the measurement result is a priority, it is also possible to set the rule such that data to serve as the target of the calculation processing analysis is increased.

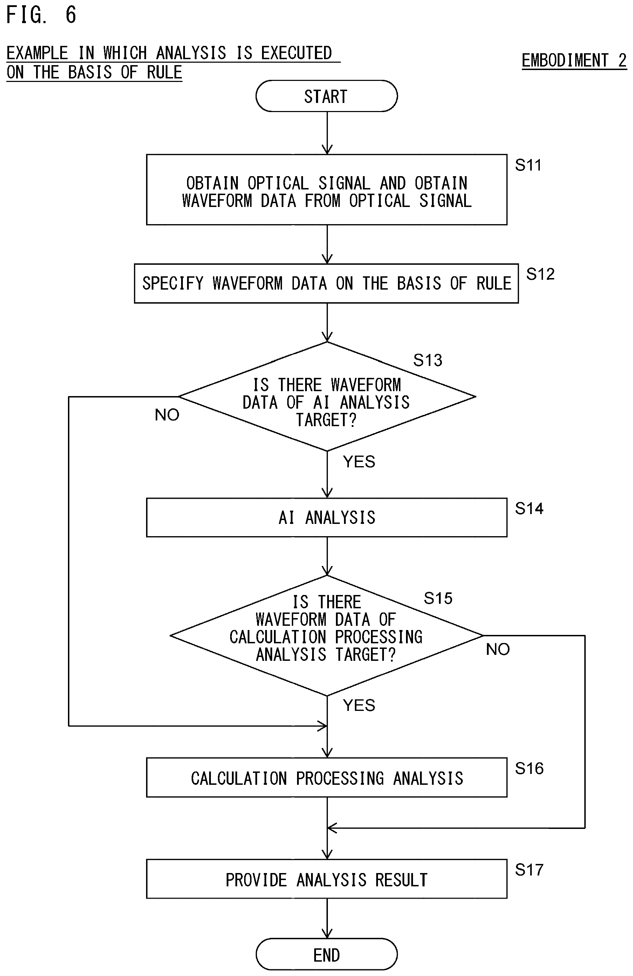

FIG. 6 is a flowchart showing an example in which an analysis operation is set on the basis of a rule set to the analysis unit 300.

In step S11, the measurement unit 400 obtains an optical signal by means of the optical detection part, and obtains waveform data from the obtained optical signal. In step S12, the analysis unit 300 refers to a rule for selecting an analysis operation, and on the basis of the rule referred to, the analysis unit 300 specifies, with respect to the waveform data obtained in step S11, waveform data to serve as a target of the AI analysis and waveform data to serve as a target of the calculation processing analysis.

In step S13, the analysis unit 300 determines whether or not waveform data to serve as a target of the AI analysis is included in the waveform data specified in step S12. When the waveform data to serve as a target of the AI analysis is included (S13: YES), the analysis unit 300 executes, in step S14, the AI analysis on the waveform data to serve as a target of the AI analysis specified in step S12.

Subsequently, in step S15, the analysis unit 300 determines whether or not there is waveform data to serve as a target of the calculation processing analysis other than the waveform data having been subjected to the AI analysis. When there is waveform data to serve as a target of the calculation processing analysis (S15: YES), the analysis unit 300 executes, in step S16, the calculation processing analysis on the waveform data to serve as a target of the calculation processing analysis specified in step S12.

In some cases, the measurement unit 400 obtains, in step S12, both of waveform data to serve as a target of the AI analysis and waveform data to serve as a target of the calculation processing analysis. For example, when, in accordance with a measurement order, the measurement unit 400 has executed measurement regarding white blood cell classification and measurement regarding reticulocytes, the measurement unit 400 obtains waveform data for white blood cell classification and waveform data for reticulocyte measurement. When the white blood cell classification is the target of the AI analysis, and the reticulocyte measurement is the target of the calculation processing analysis, the analysis unit 300 determines that waveform data for white blood cell classification being the target of the AI analysis is included (S13: YES), and executes the AI analysis on the waveform data. Further, the analysis unit 300 determines that waveform data for reticulocyte classification being the target of the calculation processing analysis is also included (S15: YES), and executes the calculation processing analysis on the waveform data.

On the other hand, when waveform data to serve as a target of the AI analysis is not included in the waveform data obtained by the measurement unit 400 (S13: NO), the analysis unit 300 executes, in step S16, the calculation processing analysis on the waveform data to serve as a target of the calculation processing analysis specified in step S12. When the AI analysis has been executed and waveform data to serve as a target of the calculation processing analysis is not included (S15: NO), the calculation processing analysis is not executed and the process is advanced to step S17.

In step S17, the analysis unit 300 provides the analysis result.

It should be noted that the analysis unit 300 may determine, in step S13, whether or not waveform data to serve as a target of the calculation processing analysis is included, and may determine, in step S15, whether or not waveform data to serve as a target of the AI analysis is included. In this case, when the analysis unit 300 has determined, in step S13, that waveform data to serve as a target of the calculation processing analysis is included, the analysis unit 300 executes the calculation processing analysis in step S14. Further, when the analysis unit 300 has determined, in step S15, that waveform data to serve as a target of the AI analysis is included, the analysis unit 300 executes the AI analysis in step S16.

[Embodiment 3]

In Embodiment 3, various examples of apportioning between the AI analysis and the calculation processing analysis will be described.

For example, apportioning between the AI analysis and the calculation processing analysis is determined by a software program with which the analysis unit 300 executes analysis of waveform data. The software program of the analysis unit 300 specifies waveform data to serve as a target of the AI analysis and waveform data to serve as a target of the calculation processing analysis, and executes analysis. The software program is designed in accordance with a requirement (e.g., improving the TAT, increasing the analysis accuracy, etc.) regarding the test, for example.

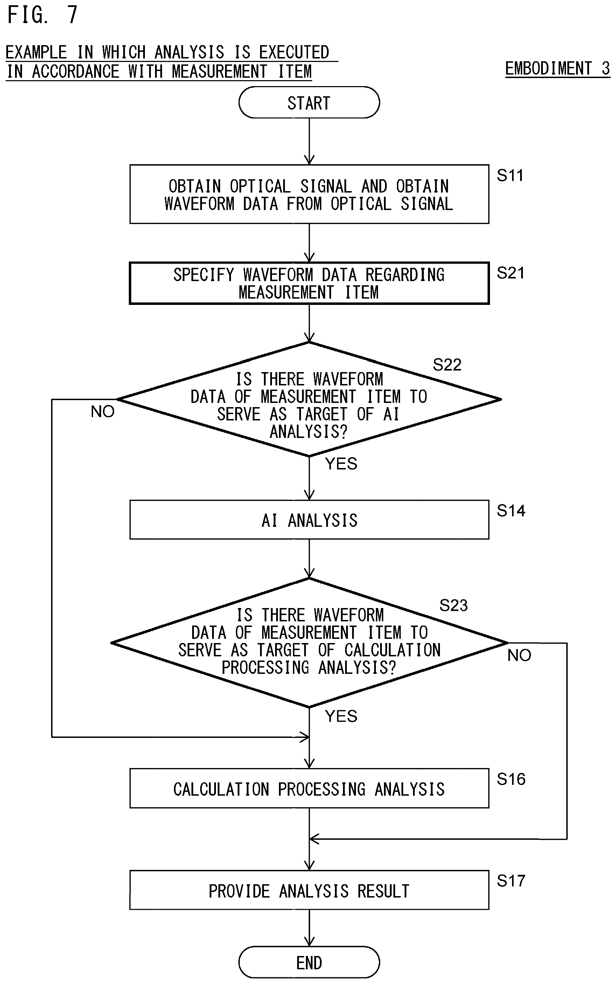

FIG. 7 is a flowchart showing an example in which analysis is executed in accordance with a measurement item.

In FIG. 7, when compared with FIG. 6, steps S21, S22, and S23 are added in place of steps S12, S13, and S15. Hereinafter, changes from FIG. 6 will be described.

In step S21, the analysis unit 300 refers to a rule that includes which of the AI analysis and the calculation processing analysis is to be performed on the basis of measurement items, and on the basis of the rule referred to, the analysis unit 300 specifies, with respect to the waveform data obtained in step S11, waveform data of a measurement item to serve as a target of the AI analysis and waveform data of a measurement item to serve as a target of the calculation processing analysis.

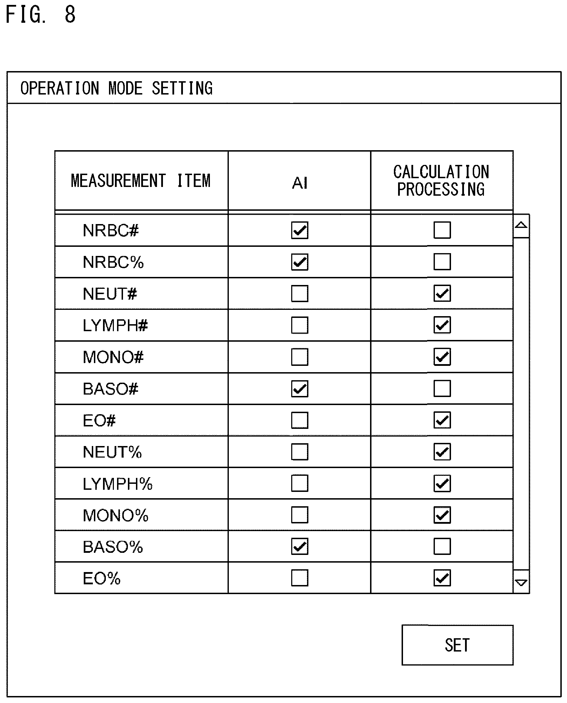

FIG. 8 is an exemplary drawing schematically showing a screen for setting the AI analysis or the calculation processing analysis for each measurement item. The measurement items shown as examples in FIG. 8 are those for a blood cell analyzer.

The screen in FIG. 8 is displayed on a display part of the analysis unit 300, for example. The screen in FIG. 8 includes, for each measurement item, a check box for setting the AI analysis and a check box for setting the calculation processing analysis. The check box for the AI analysis and the check box for the calculation processing analysis that correspond to one measurement item are configured such that only either one is selectable. The user operates a check box for each measurement item, to select which analysis out of the AI analysis and the calculation processing analysis is to be performed, and operates a setting button. Accordingly, the rule is stored in a storage of the analysis unit 300.

Although the user sets either one of the AI analysis and the calculation processing analysis with respect to each measurement item via the screen shown in FIG. 8, the screen may be configured such that both of the AI analysis and the calculation processing analysis can be set. Accordingly, the result of the AI analysis and the result of the calculation processing analysis can be compared with each other. Selection of the analysis with respect to each measurement item may be set in advance at the time of shipment of the apparatus, or only a manager may be allowed to change the setting.

With reference back to FIG. 7, in step S22, the analysis unit 300 determines whether or not waveform data of a measurement item to serve as a target of the AI analysis is included in the waveform data specified in step S21. When waveform data of a measurement item to serve as a target of the AI analysis is included (S22: YES), the analysis unit 300 executes, in step S14, the AI analysis regarding the measurement item on the waveform data of the measurement item to serve as a target of the AI analysis specified in step S21.

For example, in accordance with a measurement order of classifying white blood cells (e.g., five classifications of neutrophil, lymphocyte, monocyte, eosinophil, and basophil), the measurement unit 400 mixes a specimen with a reagent that corresponds to the classification, to prepare a white blood cell measurement sample. The measurement unit 400 obtains optical signals that correspond to the white blood cell measurement sample by means of the optical detection part. The measurement unit 400 obtains waveform data that corresponds to each obtained optical signal. When a measurement item (e.g., the count and proportion of each of neutrophils, lymphocytes, monocytes, eosinophils, and basophils) regarding the white blood cell classification is the target of the AI analysis, the analysis unit 300 executes the AI analysis on the waveform data obtained, by the measurement unit 400, through measurement of the white blood cell measurement sample.

Subsequently, in step S23, the analysis unit 300 determines whether or not there is waveform data of a measurement item to serve as a target of the calculation processing analysis in the waveform data specified in step S12. When there is waveform data to serve as a target of the calculation processing analysis (S23: YES), the analysis unit 300 executes, in step S16, the calculation processing analysis regarding the measurement item on the waveform data to serve as a target of the calculation processing analysis specified in step S21.

For example, in accordance with a measurement order of classifying reticulocytes, the measurement unit 400 mixes a specimen with a reagent that corresponds to the classification, to prepare a reticulocyte measurement sample. The measurement unit 400 obtains optical signals that correspond to the reticulocyte measurement sample by means of the optical detection part. The measurement unit 400 obtains waveform data that corresponds to each obtained optical signal. When a measurement item (e.g., the count and proportion of reticulocytes) regarding classification of reticulocytes is a target of the calculation processing analysis, the analysis unit 300 executes the calculation processing analysis on the waveform data obtained, by the measurement unit 400, through measurement of the reticulocyte measurement sample.

Not limited to a blood cell analyzer, the specimen analyzer 4000 may be a urine analyzer or a blood coagulation measurement apparatus. For example, when the specimen analyzer 4000 is a urine analyzer, the analysis unit 300 executes the AI analysis with respect to some of measurement items and performs the calculation processing analysis with respect to the remaining measurement items. When the specimen analyzer 4000 is a blood coagulation measurement apparatus, the analysis unit 300 executes the calculation processing analysis with respect to all of measurement items, executes, with respect to some measurement items, the AI analysis in addition to the calculation processing analysis, and determines whether or not there is a suspected occurrence of nonspecific reaction.

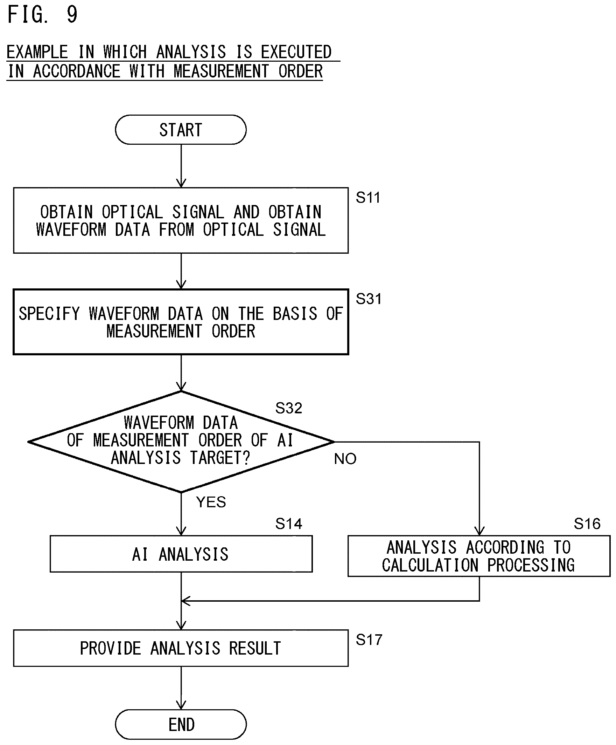

FIG. 9 is a flowchart showing an example in which analysis is executed in accordance with a measurement order.

In FIG. 9, when compared with FIG. 6, steps S31 and S32 are added in place of steps S12 and S13, and step S15 is deleted. Hereinafter, changes from FIG. 6 will be described.

In step S31, on the basis of a measurement order, the analysis unit 300 specifies which of waveform data to serve as a target of the AI analysis and waveform data to serve as a target of the calculation processing analysis the waveform data obtained in step S11 is. The analysis mode for a measurement order is either an AI analysis mode or a calculation processing analysis mode, and is stored in a storage of the analysis unit 300 in association with the measurement order.

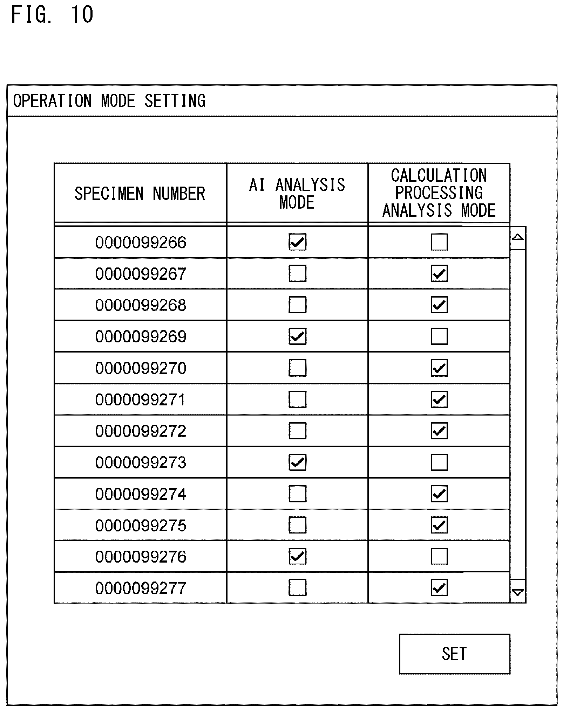

FIG. 10 is an exemplary drawing schematically showing a screen for setting an analysis mode for a measurement order.

The screen in FIG. 10 is displayed on a display part of the analysis unit 300, for example. In the screen in FIG. 10, each row corresponds to a measurement order identified by a specimen number. The screen in FIG. 10 includes, for each measurement order, a check box for setting the AI analysis mode and a check box for setting the calculation processing analysis mode. The user operates a check box for each measurement order, to select which analysis out of the AI analysis and the calculation processing analysis is to be performed by the analysis unit 300, and operates a setting button. Accordingly, an analysis mode is stored in association with the measurement order, in a storage of the analysis unit 300.

Not limited to the configuration in which the analysis mode that is associated with each measurement order is set by the user via the analysis unit 300, the analysis mode may be set in advance at the time of setting of a measurement order at a host computer or the like.

With reference back to FIG. 9, in step S32, the analysis unit 300 determines whether or not the waveform data specified in step S31 is waveform data of a measurement order being a target of the AI analysis. When the specified waveform data is waveform data of a measurement order being a target of the AI analysis (S32: YES), the analysis unit 300 performs, in step S14, the AI analysis on the waveform data of the measurement order. On the other hand, when the specified waveform data is waveform data of a measurement order being a target of the calculation processing analysis (S32: NO), the analysis unit 300 performs, in step S16, the calculation processing analysis on the waveform data of the measurement order.

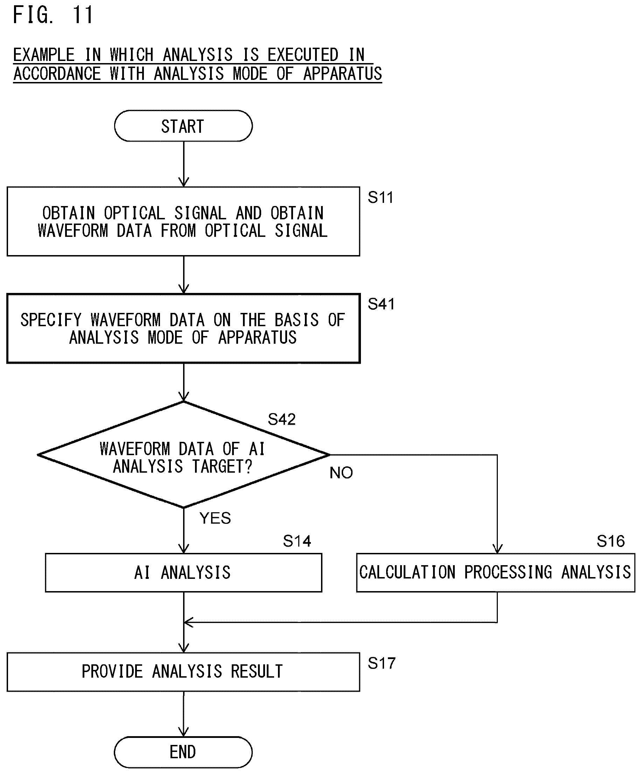

FIG. 11 is a flowchart showing an example in which analysis is executed in accordance with the analysis mode of the apparatus.

In FIG. 11, when compared with FIG. 6, steps S41 and S42 are added in place of steps S12 and S13, and step S15 is deleted. Hereinafter, changes from FIG. 6 will be described.

In step S41, the analysis unit 300 refers to a rule including the analysis mode of the analysis unit 300, and on the basis of the rule referred to, the analysis unit 300 specifies which of waveform data to serve as a target of the AI analysis and waveform data to serve as a target of the calculation processing analysis the waveform data obtained in step S11 is. When the AI analysis mode has been set in the above rule, all of the waveform data serves as the target of the AI analysis, and when the calculation processing analysis mode has been set in the above rule, all data serves as the target of the calculation processing analysis.

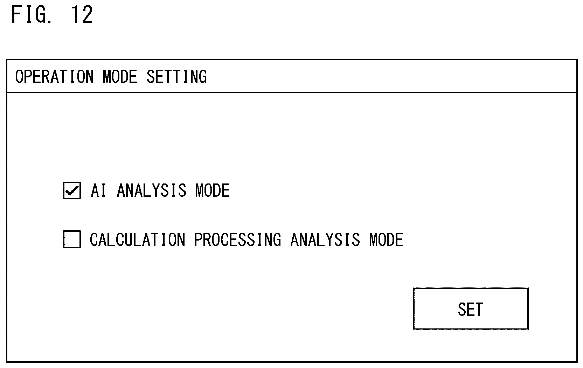

FIG. 12 is an exemplary drawing schematically showing a screen for setting an analysis mode of the analysis unit 300.

The screen in FIG. 12 is displayed on a display part of the analysis unit 300, for example. The screen in FIG. 12 includes a check box for setting the AI analysis mode and a check box for setting the calculation processing analysis mode, to the analysis unit 300. The user operates a check box, to select which analysis out of the AI analysis and the calculation processing analysis is to be performed by the analysis unit 300, and operates a setting button. Accordingly, a rule is stored in a storage of the analysis unit 300.

With reference back to FIG. 11, in step S42, the analysis unit 300 determines whether or not the waveform data specified in step S41 is waveform data to serve as a target of the AI analysis. When the specified waveform data is data of a target of the AI analysis (S42: YES), i.e., the analysis mode of the analysis unit 300 is the AI analysis mode, the analysis unit 300 performs, in step S14, the AI analysis on the waveform data. On the other hand, when the specified waveform data is waveform data of a target of the calculation processing analysis (S42: NO), i.e., the analysis mode of the analysis unit 300 is the calculation processing analysis mode, the analysis unit 300 performs, in step S16, the calculation processing analysis on the waveform data.

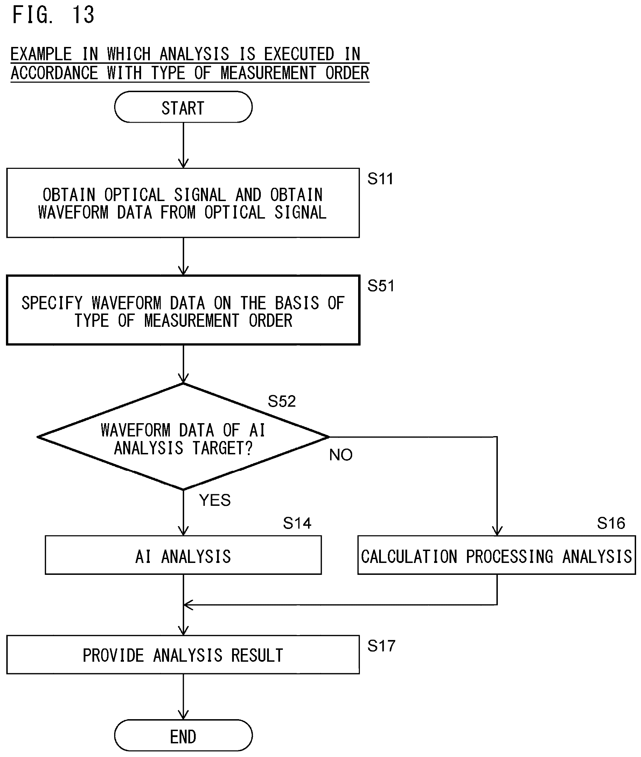

FIG. 13 is a flowchart showing an example in which analysis is executed in accordance with the type of a measurement order.

In FIG. 13, when compared with FIG. 6, steps S51 and S52 are added in place of steps S12 and S13, and step S15 is deleted. Hereinafter, changes from FIG. 6 will be described.

In step S51, the analysis unit 300 refers to a rule that includes an analysis mode that corresponds to the type of the measurement order, and on the basis of the type of the measurement order and the rule referred to, the analysis unit 300 specifies which of waveform data to serve as a target of the AI analysis and waveform data to serve as a target of the calculation processing analysis the waveform data obtained in step S11 is. The type of the measurement order includes "Normal" corresponding to normal measurement such as an initial test, "Rerun" corresponding to a re-test in which a measurement item identical to that of the initial test is set, and "Reflex" corresponding to a re-test in which the measurement item has been changed from that of the initial test. In the above rule, for each type of the measurement order, either one of the AI analysis mode and the calculation processing analysis mode is set.

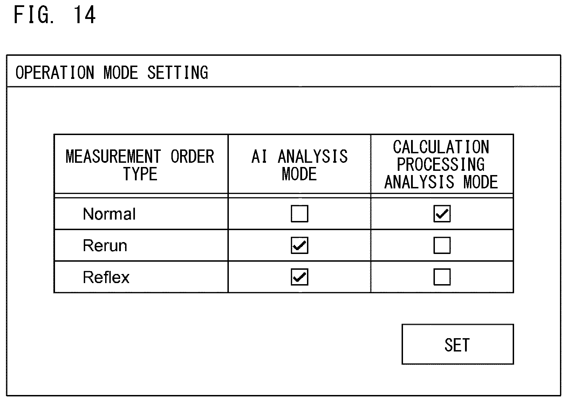

FIG. 14 is an exemplary drawing schematically showing a screen for setting an analysis mode for each type of the measurement order.

The screen in FIG. 14 is displayed on a display part of the analysis unit 300, for example. The screen in FIG. 14 includes, for each type (Normal, Rerun, Reflex) of the measurement order, a check box for setting the AI analysis mode and a check box for setting the calculation processing analysis mode. The user operates a check box, to select which analysis out of the AI analysis and the calculation processing analysis is to be performed for each type of the measurement order, and operates a setting button. Accordingly, a rule is stored in a storage of the analysis unit 300.

Not limited to the configuration in which the analysis mode that is associated with the type of each measurement order is set by the user via the analysis unit 300, the analysis mode may be set in advance in accordance with the type of the measurement order at a host computer or the like.

With reference back to FIG. 13, in step S52, the analysis unit 300 determines whether or not the waveform data specified in step S51 is waveform data to serve as a target of the AI analysis. When the specified waveform data is waveform data of a target of the AI analysis (S52: YES), i.e., the analysis mode according to the type of the measurement order is the AI analysis mode, the analysis unit 300 performs, in step S14, the AI analysis on the waveform data. On the other hand, when the specified waveform data is waveform data of a target of the calculation processing analysis (S52: NO), i.e., the analysis mode according to the type of the measurement order is the calculation processing analysis mode, the analysis unit 300 performs, in step S16, the calculation processing analysis on the waveform data.

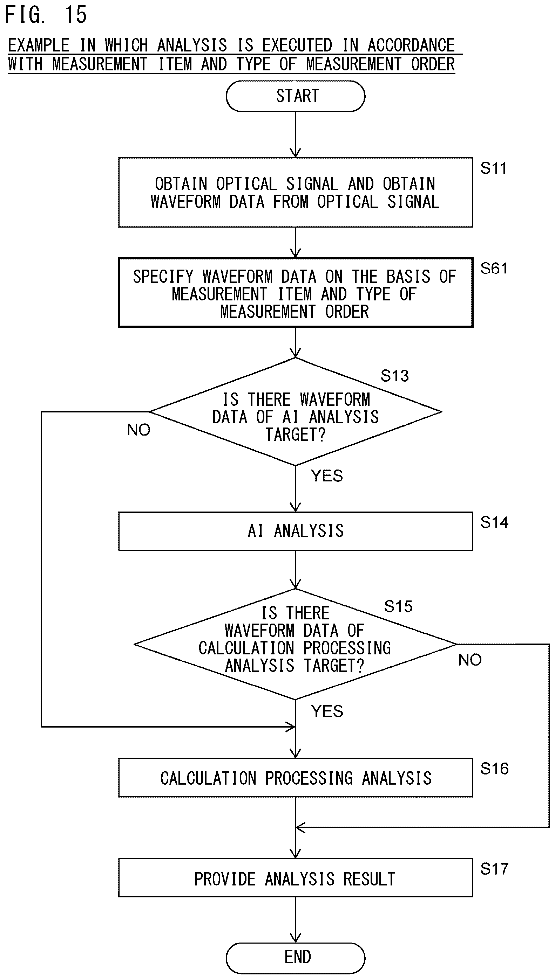

FIG. 15 is a flowchart showing an example in which analysis is executed in accordance with a measurement item and the type of a measurement order.

In FIG. 15, when compared with FIG. 6, step S61 is added in place of step S12. Hereinafter, changes from FIG. 6 will be described.

In step S61, the analysis unit 300 refers to a rule for selecting an analysis operation, and on the basis of the measurement item and the type of the measurement order, the analysis unit 300 specifies, with respect to the waveform data obtained in step S11, waveform data to serve as a target of the AI analysis and waveform data to serve as a target of the calculation processing analysis.

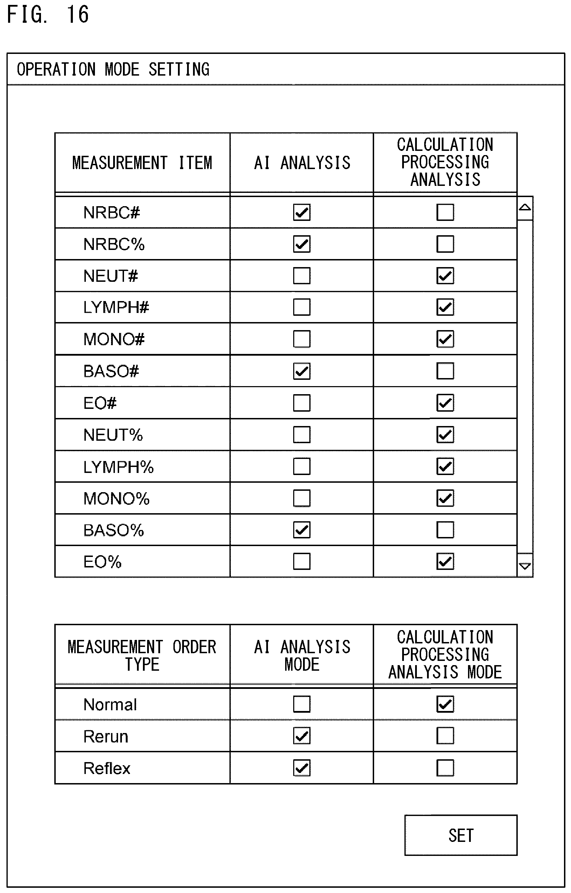

FIG. 16 is an exemplary drawing schematically showing a screen for setting the AI analysis or the calculation processing analysis for each measurement item and each type of the measurement order.

The screen in FIG. 16 is displayed on a display part of the analysis unit 300, for example. Similar to FIG. 8, the screen in FIG. 16 includes, for each measurement item, a check box for setting the AI analysis and a check box for setting the calculation processing analysis, and includes, for each type (Normal, Rerun, Reflex) of the measurement order, check boxes for setting either of the AI analysis and the calculation processing analysis, similar to FIG. 14. The user operates, for each measurement item, a check box in the list in the upper part, to select which analysis out of the AI analysis and the calculation processing analysis is to be performed, operates, for each type of the measurement order, a check box in the list in the lower part, to select which analysis out of the AI analysis and the calculation processing analysis is to be performed, and operates a setting button. Accordingly, a rule is stored in a storage of the analysis unit 300.

In a case where setting has been performed as shown in FIG. 16, when the type of the measurement order is "Normal", the analysis unit 300 specifies the waveform data obtained in the measurement unit 400, as a target of the calculation processing analysis. For example, when the type of the measurement order is "Normal", irrespective of the setting of the analysis for each measurement item, the calculation processing analysis is executed with respect to all of the measurement items based on the measurement order. When the type of the measurement order is "Rerun" or "Reflex", the analysis unit 300 sets the waveform data obtained in the measurement unit 400, as a target of the AI analysis or the calculation processing analysis, in accordance with the analysis setting set for each measurement item. For example, when the type of the measurement order is "Rerun" and "Reflex", the measurement items regarding nucleated red blood cells (NRBC) and basophils (BASO) serve as targets of the AI analysis, and the other measurement items serve as targets of the calculation processing analysis.

With reference back to FIG. 15, in step S13, the analysis unit 300 determines whether or not data to serve as a target of the AI analysis is included in the waveform data specified in step S61. When there is waveform data to serve as a target of the AI analysis (S13: YES), the analysis unit 300 executes, in step S14, the AI analysis on the waveform data to serve as a target of the AI analysis specified in step S61.

Subsequently, in step S15, the analysis unit 300 determines whether or not waveform data to serve as a target of the calculation processing analysis is included in the waveform data specified in step S61. When there is waveform data to serve as a target of the calculation processing analysis (S15: YES), the analysis unit 300 executes, in step S16, the calculation processing analysis on the waveform data to serve as a target of the calculation processing analysis specified in step S61.

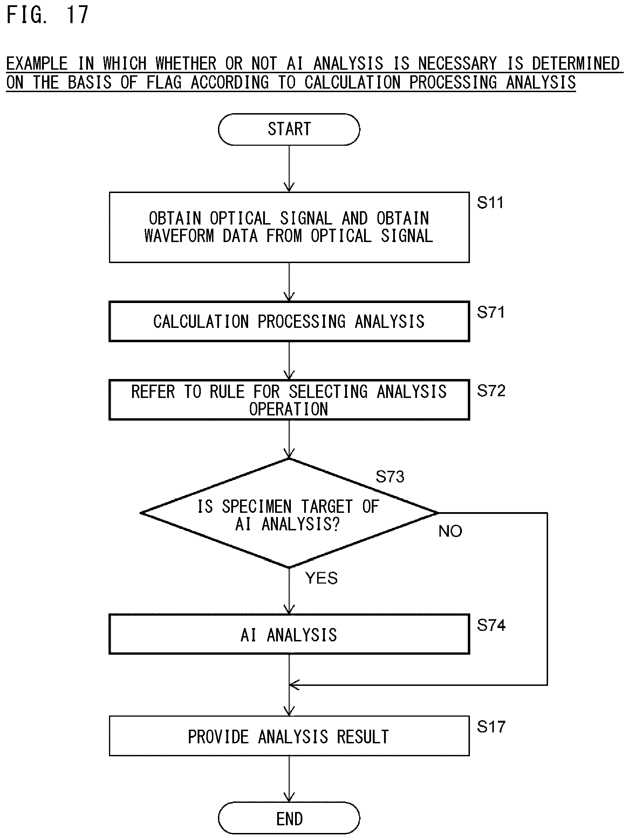

FIG. 17 is a flowchart showing an example in which whether or not the AI analysis is necessary is determined on the basis of a flag provided through the calculation processing analysis.

In FIG. 17, when compared with FIG. 6, steps S71 to S74 are added after step S11, and steps S12 to S16 are deleted. Hereinafter, changes from FIG. 6 will be described.

In step S71, the analysis unit 300 executes the calculation processing analysis on the waveform data obtained in step S11, and sets a flag that suggests an abnormality regarding an analyte in the specimen, on the basis of the result of the calculation processing analysis. The flag is, for example, a flag that indicates a predetermined abnormal cell has been detected, a flag that indicates the count value of predetermined blood cells is an abnormal value, or the like. In step S72, the analysis unit 300 refers to a rule that includes whether or not to perform the AI analysis on an analysis result having a flag.

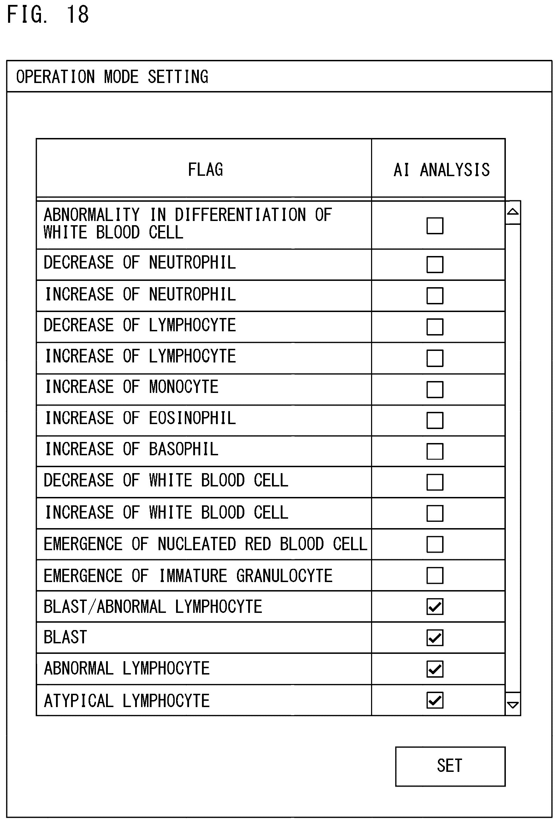

FIG. 18 is an exemplary drawing schematically showing a screen for setting the AI analysis for each flag of an analysis result.

The screen in FIG. 18 is displayed on a display part of the analysis unit 300, for example. The screen in FIG. 18 includes, for each flag provided through the calculation processing analysis to an analysis result, a check box for setting the AI analysis. When the specimen analyzer 4000 is a blood cell analyzer, the flag provided through the calculation processing analysis to an analysis result includes decrease of blood cells, increase of blood cells, emergence of an abnormal cell, and the like. The user operates a check box for each flag, to select whether to perform the AI analysis, and operates a setting button. Accordingly, a rule is stored in a storage of the analysis unit 300.

When the check box of a flag is on, the AI analysis is executed on the waveform data that corresponds to the analysis result. In the example shown in FIG. 18, when the check boxes for the analysis results of blast/abnormal lymphocyte, blast, abnormal lymphocyte, and atypical lymphocyte are on, and flags indicating that these blood cells are present have been set through the calculation processing analysis, the AI analysis is executed with respect to these blood cells.

With reference back to FIG. 17, in step S73, on the basis of the flag provided to the analysis result obtained in step S71 and the rule referred to in step S72, the analysis unit 300 determines whether or not the specimen is an AI analysis target. When the specimen is an AI analysis target (S73: YES), the analysis unit 300 executes, in step S74, the AI analysis on each piece of the waveform data. For example, when a flag indicating that a blast has been detected through the calculation processing analysis has been issued, the AI analysis is, according to the rule shown as an example in FIG. 18, executed on each piece of the waveform data obtained in step S11.

On the other hand, when the specimen is not an AI analysis target (S73: NO), the analysis unit 300 skips step S74.

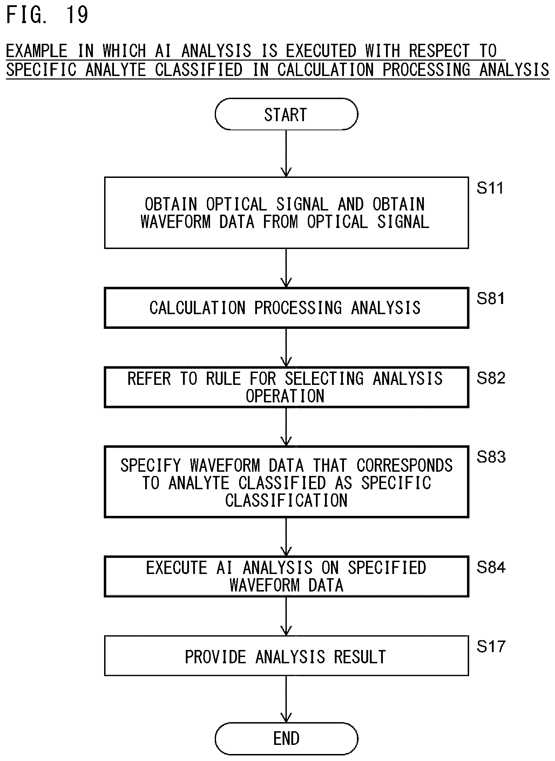

FIG. 19 is a flowchart showing an example in which the AI analysis is executed with respect to a specific analyte classified in the calculation processing analysis.

In FIG. 19, when compared with FIG. 6, steps S81 to S84 are added after step S11, and steps S12 to S16 are deleted. Hereinafter, changes from FIG. 6 will be described.

In step S81, the analysis unit 300 executes the calculation processing analysis on the waveform data obtained in step S11, to classify the analytes. In step S82, the analysis unit 300 refers to a rule that includes whether or not to perform the AI analysis for each type of analyte.

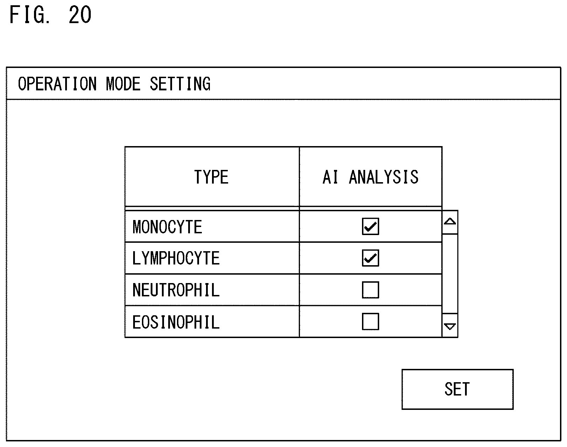

FIG. 20 is an exemplary drawing schematically showing a screen for setting whether or not to perform the AI analysis for each type of analyte.

The screen in FIG. 20 is displayed on a display part of the analysis unit 300, for example. The screen in FIG. 20 includes, for each type of analyte, a check box for setting the AI analysis. When the specimen analyzer 4000 is a blood cell analyzer, the types to be classified through the calculation processing analysis include eosinophil, neutrophil, lymphocyte, monocyte, and the like. The user operates a check box, to select whether or not to perform the AI analysis for each type of analyte, and operates a setting button. Accordingly, a rule is stored in a storage of the analysis unit 300.

When the check box for a type of an analyte is on, the AI analysis is executed on the waveform data classified according to the type. In the example shown in FIG. 20, the AI analysis is executed with respect to monocytes and lymphocytes.

With reference back to FIG. 19, in step S83, on the basis of the types of the analytes classified in step S81 and the rule referred to in step S82, the analysis unit 300 specifies waveform data that corresponds to the analyte (e.g., in the case of the rule shown in FIG. 20, monocyte and lymphocyte) classified as a specific classification. In step S84, the analysis unit 300 executes the AI analysis on the specified waveform data.

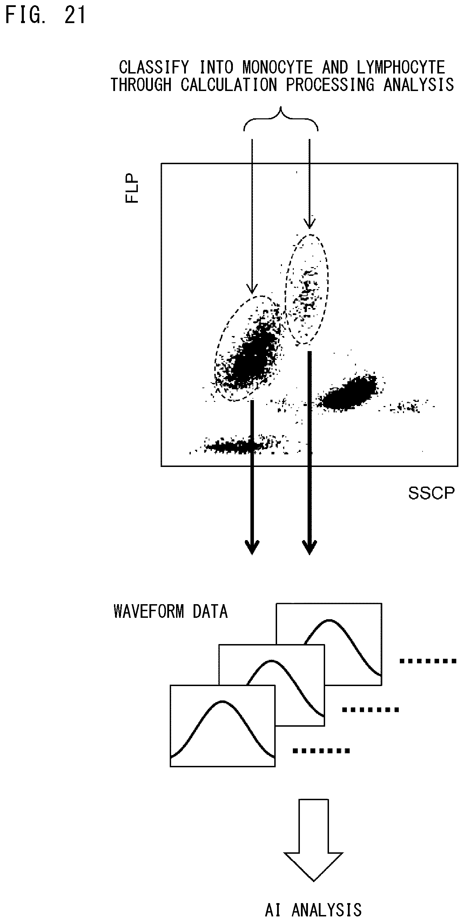

FIG. 21 is a diagram describing a classification method according to the calculation processing analysis and the AI analysis executed in the process shown in FIG. 19.

In the calculation processing analysis, a scattergram of which axes represent two types of representative values calculated from waveform data is used. For example, when the AI analysis has been set to be executed with respect to monocytes and lymphocytes as shown in FIG. 20, plots in regions surrounded by broken lines that correspond to monocytes and lymphocytes on the scattergram are specified through the calculation processing analysis in step S81. Then, in step S83, waveform data that corresponds to the plots in each surrounded region is specified, and the AI analysis is executed on the specified waveform data in step S84.

With reference back to FIG. 19, in step S17, the analysis unit 300 provides analysis results obtained through the calculation processing analysis and the AI analysis. At this time, the analysis unit 300 replaces, out of the analysis results obtained through the calculation processing analysis in step S81, the analysis result of the type having served as a target of the AI analysis, with an analysis result obtained through the AI analysis, and provides the analysis result. The analysis result obtained through the calculation processing analysis and the analysis result obtained through the AI analysis may be provided in combination.

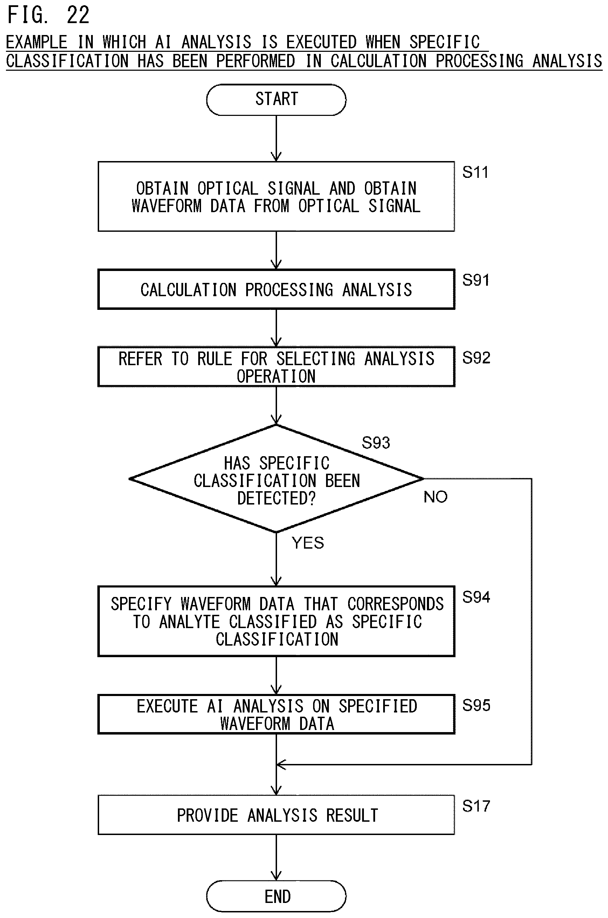

FIG. 22 is a flowchart showing an example in which the AI analysis is executed when a specific classification has been performed in the calculation processing analysis.

In FIG. 22, when compared with FIG. 6, steps S91 to S95 are added after step S11, and steps S12 to S16 are deleted. Hereinafter, changes from FIG. 6 will be described.

In step S91, the analysis unit 300 executes the calculation processing analysis on the waveform data obtained in step S11, and classifies analytes. In step S92, the analysis unit 300 refers to a rule that includes whether or not to perform the AI analysis on an analyte of a specific type, e.g., a cell that is not present in peripheral blood of a healthy individual.

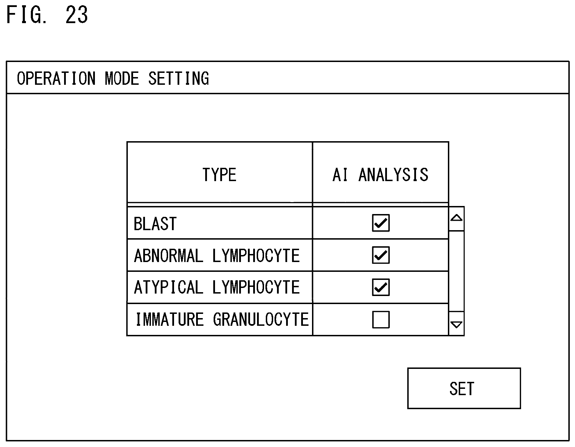

FIG. 23 is an exemplary drawing schematically showing a screen for setting whether or not to perform the AI analysis with respect to an analyte of a specific type.

The screen in FIG. 23 is displayed on a display part of the analysis unit 300, for example. The screen in FIG. 23 includes, for each specific type of analyte, a check box for setting the AI analysis. When the specimen analyzer 4000 is a blood cell analyzer, the types that are classified through the calculation processing analysis can include blast, abnormal lymphocyte, atypical lymphocyte, immature granulocyte, and the like. The user operates a check box to select whether or not to perform the AI analysis for each specific type of analyte, and operates a setting button. Accordingly, a rule is stored in a storage of the analysis unit 300.

When a check box for a specific type of analyte is on, the AI analysis is executed on the waveform data classified as the type. In the example shown in FIG. 23, the AI analysis is executed with respect to blasts, abnormal lymphocytes, and atypical lymphocytes.

With reference back to FIG. 22, in step S93, on the basis of the type of each analyte classified in step S91 and the rule referred to in step S92, the analysis unit 300 determines whether or not the analyte (e.g., in the case of the rule shown in FIG. 23, blast, abnormal lymphocyte, atypical lymphocyte) classified as a specific classification has been detected. When the analyte classified as the specific classification has been detected (S93: YES), the analysis unit 300 specifies, in step S94, waveform data that corresponds to the analyte classified as the specific classification. In step S95, the analysis unit 300 executes the AI analysis on the specified waveform data.

In step S17, the analysis unit 300 provides analysis results obtained through the calculation processing analysis and the AI analysis. At this time, the analysis unit 300 replaces, out of the analysis results obtained through the calculation processing analysis in step S91, the analysis result of the type having served as a target of the AI analysis, with an analysis result obtained through the AI analysis, and provides the analysis result. The analysis result obtained through the calculation processing analysis and the analysis result obtained through the AI analysis may be provided in combination.

[Embodiment 4]

In Embodiment 4, a detailed configuration example in which, in the specimen analyzer 4000 that analyzes a specimen according to flow cytometry, analysis is executed by being apportioned between the calculation processing analysis and the AI analysis is shown.

Examples of a specimen measured by the specimen analyzer 4000 of Embodiment 4 can include a biological sample collected from a subject. For example, the specimen can include peripheral blood such as venous blood and arterial blood, urine, and a body fluid other than blood and urine. The body fluid other than blood and urine can include bone marrow aspirate, ascites, pleural effusion, cerebrospinal fluid, and the like, for example. Hereinafter, the body fluid other than blood and urine may be simply referred to as a "body fluid". The blood sample may be any blood sample that is in a state where the number of cells can be counted and the cell types can be determined. Preferably, blood is peripheral blood. Examples of blood include peripheral blood collected using an anticoagulant agent such as ethylenediamine tetraacetate (sodium salt or potassium salt), heparin sodium, or the like. Peripheral blood may be collected from an artery or may be collected from a vein.

The cell types to be determined in the present embodiment are those according to the cell types based on morphological classification, and are different depending on the type of the specimen. When the specimen is blood and the blood is collected from a healthy individual, the cell types to be determined in the present embodiment include, for example, nucleated cell such as nucleated red blood cell and white blood cell, red blood cell, platelet, and the like. Nucleated cells include, for example, neutrophils, lymphocytes, monocytes, eosinophils, and basophils. Neutrophils include, for example, segmented neutrophils and band neutrophils. When blood is collected from an unhealthy individual, nucleated cells may include, for example, at least one type selected from the group consisting of immature granulocyte and abnormal cell. Such cells are also included in the cell types to be determined in the present embodiment. Immature granulocytes can include, for example, cells such as metamyelocytes, myelocytes, promyelocytes, and myeloblasts.

The nucleated cells may include, in addition to normal cells, abnormal cells that are not contained in peripheral blood of a healthy individual. Examples of abnormal cells are cells that appear when a person has a certain disease, and such abnormal cells are tumor cells, for example. In a case of the hematopoietic system, the certain disease can be a disease selected from the group consisting of, for example: myelodysplastic syndrome; leukemia such as acute myeloblastic leukemia, acute promyelocytic leukemia, acute myelomonocytic leukemia, acute monocytic leukemia, erythroleukemia, acute megakaryoblastic leukemia, acute myeloid leukemia, acute lymphocytic leukemia, lymphoblastic leukemia, chronic myelogenous leukemia, or chronic lymphocytic leukemia; malignant lymphoma such as Hodgkin's lymphoma or non-Hodgkin's lymphoma; and multiple myeloma.

Further, abnormal cells can include, for example, cells that are not usually observed in peripheral blood of a healthy individual, such as: lymphoblasts; plasma cells; atypical lymphocytes; reactive lymphocytes; erythroblasts, which are nucleated red blood cells, such as proerythroblasts, basophilic erythroblasts, polychromatic erythroblasts, orthochromatic erythroblasts, promegaloblasts, basophilic megaloblasts, polychromatic megaloblasts, and orthochromatic megaloblasts; megakaryocytes including micromegakaryocytes; and the like.

When the specimen is urine, the cell types to be determined in the present embodiment can include, for example, epithelial cell such as that of transitional epithelium and squamous epithelium, red blood cell, white blood cell, and the like. Examples of abnormal cells include, for example, bacteria, fungi such as filamentous fungi and yeast, tumor cells, and the like.

When the specimen is a body fluid that usually does not contain blood components, such as ascites, pleural effusion, or spinal fluid, the cell types can include, for example, red blood cell, white blood cell, and large cell. The large cell here means a cell that is separated from an inner membrane of a body cavity or a peritoneum of a viscus, and that is larger than white blood cells. For example, mesothelial cells, histiocytes, tumor cells, and the like correspond to the large cell.

When the specimen is bone marrow aspirate, the cell types to be determined in the present embodiment can include, as normal cells, mature blood cells and immature hematopoietic cells. Mature blood cells include, for example, nucleated cells such as nucleated red blood cells and white blood cells, red blood cells, platelets, and the like. Nucleated cells such as white blood cells include, for example, neutrophils, lymphocytes, plasma cells, monocytes, eosinophils, and basophils. Neutrophils include, for example, segmented neutrophils and band neutrophils. Immature hematopoietic cells include, for example, hematopoietic stem cells, immature granulocytic cells, immature lymphoid cells, immature monocytic cells, immature erythroid cells, megakaryocytic cells, mesenchymal cells, and the like. Immature granulocytes can include cells such as, for example, metamyelocytes, myelocytes, promyelocytes, myeloblasts, and the like. Immature lymphoid cells include, for example, lymphoblasts and the like. Immature monocytic cells include monoblasts and the like. Immature erythroid cells include, for example, nucleated red blood cells such as proerythroblasts, basophilic erythroblasts, polychromatic erythroblasts, orthochromatic erythroblasts, promegaloblasts, basophilic megaloblasts, polychromatic megaloblasts, and orthochromatic megaloblasts. Megakaryocytic cells include, for example, megakaryoblasts and the like.

Examples of abnormal cells that can be contained in bone marrow include, for example, hematopoietic tumor cells of a disease selected from the group consisting of: myelodysplastic syndrome; leukemia such as acute myeloblastic leukemia, acute promyelocytic leukemia, acute myelomonocytic leukemia, acute monocytic leukemia, erythroleukemia, acute megakaryoblastic leukemia, acute myeloid leukemia, acute lymphocytic leukemia, lymphoblastic leukemia, chronic myelogenous leukemia, or chronic lymphocytic leukemia; malignant lymphoma such as Hodgkin's lymphoma or non-Hodgkin's lymphoma; and multiple myeloma, which have been described above, and metastasized tumor cells of a malignant tumor developed in an organ other than bone marrow.

As the signal obtained from an analyte (e.g., cell or particle) in a specimen shown in the above example, a forward scattered light signal, a side scattered light signal, and a fluorescence signal, which are each an analog optical signal obtained through application of light to each cell flowing in a flow cell, are shown as examples. However, the signal is not limited in particular as long as the signal indicates a feature of each analyte and allows classification of analytes for each type.

Preferably, the optical signal is a light signal obtained as an optical response to application of light to a cell. The light signal can include at least one type selected from a signal based on light scattering, a signal based on light absorption, a signal based on transmitted light, and a signal based on fluorescence.

The signal based on light scattering can include a scattered light signal caused by light application and a light loss signal caused by light application. The scattered light signal represents a feature of an analyte in the specimen in accordance with the light reception angle of scattered light with respect to the advancing direction of application light. The forward scattered light signal is used in calculation of a representative value that indicates the size of the analyte. The side scattered light signal is used in calculation of a representative value that indicates, when the analyte in the specimen is a cell, complexity of the nucleus of the cell.

"Forward" of the forward scattered light means the advancing direction of light emitted from a light source. When the angle of application light is defined as 0°, "forward" can include a forward low angle at which the light reception angle is about 0° to 5°, and/or a forward high angle at which the light reception angle is about 5° to 20°. "Side" is not limited as long as the "side" does not overlap "forward". When the angle of application light is defined as 0°, "side" can include a light reception angle being about 25° to 155°, preferably about 45° to 135°, and more preferably about 90°. Fluorescence in the present embodiment is detected at a light reception angle similar to that of side scattered light.

The signal based on light scattering may include polarized light or depolarized light as a component of the signal. For example, when scattered light caused by application of light to an analyte in the specimen is received through a polarizing plate, only scattered light polarized at a specific angle can be received. Meanwhile, when light is applied to an analyte in the specimen through a polarizing plate, and the resultant scattered light is received through a polarizing plate that allows passage therethrough of only polarized light having an angle different from that of the polarizing plate for light application, only depolarized scattered light can be received.

A light loss signal indicates the loss amount of received light based on decrease, of the received light amount at a light receiving part, which is caused by application of light to an analyte and scattering of the light. Preferably, the light loss signal is obtained as a light loss (axial light loss) in the optical axis direction of the application light. The light loss signal can be expressed as a proportion of the received light amount at the time of flowing of a measurement sample in the flow cell, when the received light amount at the light receiving part in a state where the measurement sample is not flowing in the flow cell is defined as 100%. Similar to the forward scattered light signal, the axial light loss is used in calculation of a representative value that indicates the size of the analyte, but the signal that is obtained differs depending on whether the analyte has translucency or not.

The signal based on fluorescence may be fluorescence that is excited as a result of application of light to an analyte labeled with a fluorescent substance, or may be an intrinsic fluorescence that is generated from a non-stained analyte. When the analyte in the specimen is a cell, the fluorescent substance may be a fluorescent dye that binds to nucleic acid or membrane protein, or may be a labeled antibody obtained by modifying, with a fluorescent dye, an antibody that binds to a specific protein of the cell.

The optical signal may be obtained in a form of image data obtained by applying light to an analyte in the specimen and capturing an image of the analyte to which the light has been applied. The image data can be obtained by capturing, with an imaging element such as a TDI camera or a CCD camera, an image of each individual analyte flowing in a flow path in a flow cell. Alternatively, a specimen or a measurement sample containing cells is applied, sprayed, or spot-applied on a slide glass, and an image of the slide glass is captured by an imaging element, whereby image data of cells may be obtained.

The signal obtained from an analyte in the specimen is not limited to an optical signal, and may be an electrical signal obtained from a cell. As for the electrical signal, for example, DC current is applied to the flow cell, and change in impedance caused by an analyte flowing in the flow cell may be used as the electrical signal. The thus obtained electrical signal is used in calculation of a representative value that reflects the volume of the analyte. Alternatively, the electrical signal may be the change in impedance at the time of application of a radio frequency to an analyte flowing in the flow cell. The thus obtained electrical signal is used in calculation of a representative value that reflects conductivity of the analyte.

The signal obtained from an analyte in the specimen may be a combination of at least two types of signals out of the above-described signals. Through combination of a plurality of signals, the features of an analyte can be pleiotropically analyzed, and thus, cell classification with a higher accuracy is enabled. As for the combination, for example, at least two out of a plurality of optical signals, e.g., a forward scattered light signal, a side scattered light signal, and a fluorescence signal, may be combined. Alternatively, scattered light signals having different angles, e.g., a low angle scattered light signal and a high angle scattered light signal, may be combined. Still alternatively, an optical signal and an electrical signal may be combined, and the type and number of the signals to be combined are not limited in particular.

The AI algorithm 60 used in the AI analysis in the present embodiment is a deep learning algorithm, for example. The deep learning algorithm is one of artificial intelligence algorithms, and configured as a neural network that includes a middle layer composed of multiple layers. Data inputted to the neural network is processed by a large number of matrix operations. From digital data obtained through A/D conversion of each analog optical signal shown as an example in FIG. 2 described above, waveform data corresponding to each analyte is obtained, and the obtained waveform data is inputted to and analyzed by the AI algorithm 60. For example, by the AI algorithm 60, the type of the analyte that corresponds to the inputted waveform data is classified.

In the present embodiment, classifying the type of each analyte in the specimen is not limited to the classification performed by the AI algorithm 60. From individual analytes passing through a predetermined position in a flow path, a signal intensity is obtained, for each of the analytes, at a plurality of time points in a time period while the analyte is passing through the predetermined position, and on the basis of a result in which the obtained signal intensities at the plurality of time points regarding each individual analyte are recognized as a pattern, the type of each analyte may be determined. The pattern may be recognized as a numerical pattern of signal intensities at a plurality of time points, or may be recognized as a shape pattern obtained when signal intensities at a plurality of time points are plotted on a graph. When the pattern is recognized as a numerical pattern, if a numerical pattern of an analyte and a numerical pattern for which the type is already known are compared with each other, the type of the analyte can be determined. For the comparison between the numerical pattern of an analyte and a control numerical pattern, Spearman rank correlation, z-score, or the like can be used, for example. When the pattern of the graph shape of an analyte and the pattern of a graph shape for which the type is already known are compared with each other, the type of the analyte can be determined. For the comparison between the pattern of the graph shape of an analyte and the pattern of the graph shape for which the type is already known, geometric shape pattern matching may be used, or a feature descriptor represented by SIFT Descriptor may be used, for example.

(Configuration example)

A configuration example of the specimen analyzer 4000 when the measurement unit 400 includes an FCM detection part (detection part based on flow cytometry) for measuring a specimen (e.g., blood specimen, urine specimen, body fluid, bone marrow aspirate) will be described.

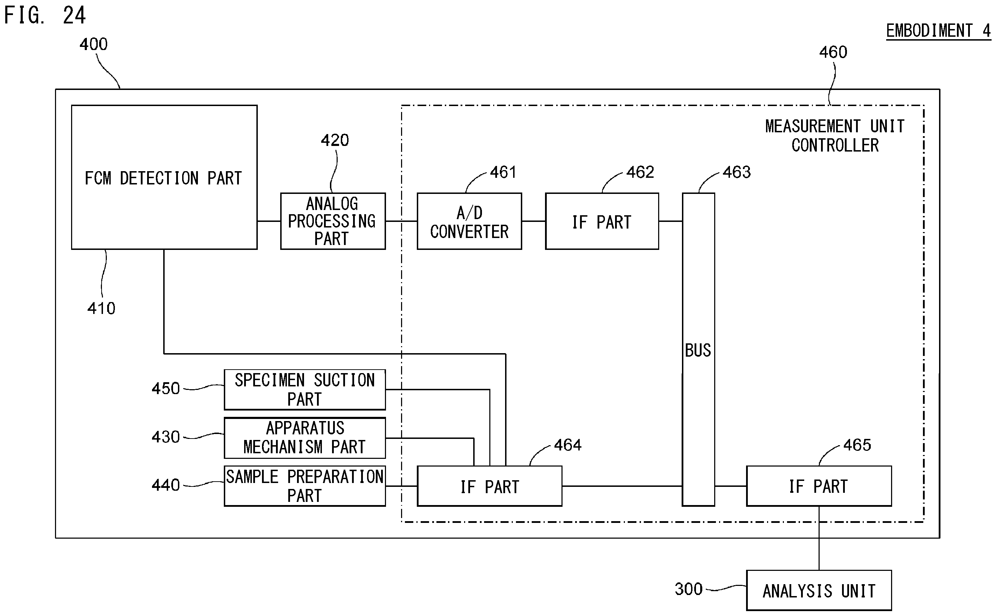

FIG. 24 is a block diagram showing a configuration of the measurement unit 400.

As shown in FIG. 24, the measurement unit 400 includes: an FCM detection part 410 for detecting an analyte in a specimen; an analog processing part 420 for processing an analog optical signal outputted from the FCM detection part 410; an apparatus mechanism part 430; a sample preparation part 440; a specimen suction part 450; and a measurement unit controller 460.

The specimen suction part 450 suctions a specimen from a specimen container, and discharges the suctioned specimen into a reaction container (e.g., reaction chamber, reaction cuvette), for example. The sample preparation part 440 suctions a reagent for preparing a measurement sample, and discharges the reagent into the reaction container that contains the specimen, for example. The specimen and the reagent are mixed in the reaction container, whereby a measurement sample is prepared. The apparatus mechanism part 430 includes mechanisms in the measurement unit 400.

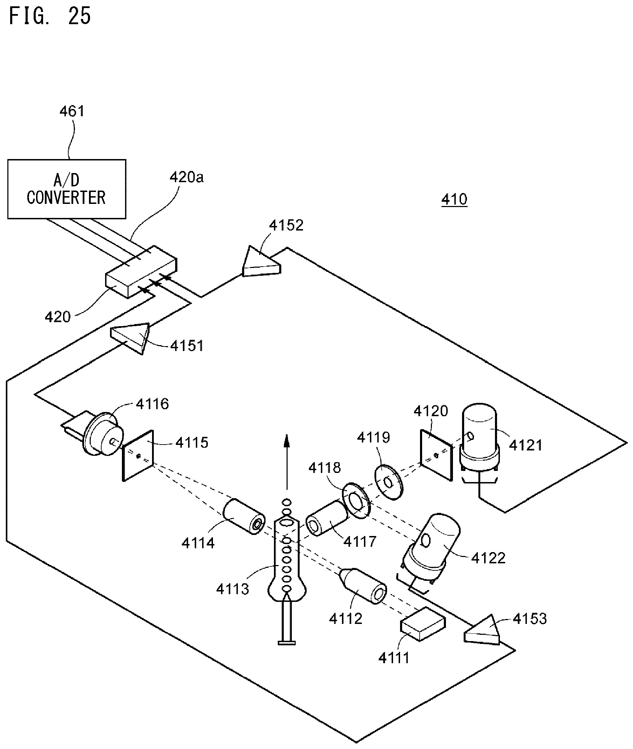

FIG. 25 schematically shows a configuration of an optical system of the FCM detection part 410.

Light emitted from a light source 4111 is applied via a light application lens system 4112 to each analyte in a measurement sample passing through a flow cell (sheath flow cell) 4113. Accordingly, scattered light and fluorescence are emitted from the analyte flowing in the flow cell 4113.

The wavelength of light emitted from the light source 4111 is not limited in particular, and a wavelength suitable for excitation of the fluorescent dye is selected. As the light source 4111, for example, a semiconductor laser light source, a gas laser light source such as an argon laser light source or a helium-neon laser, a mercury arc lamp, or the like is used. In particular, a semiconductor laser light source is suitable because the semiconductor laser light source is very inexpensive when compared with a gas laser light source.

Forward scattered light generated from an analyte in the flow cell 4113 is received by a light receiving element 4116 via a condenser lens 4114 and a pin hole part 4115. The light receiving element 4116 is a photodiode, for example. Side scattered light generated from the analyte in the flow cell 4113 is received by a light receiving element 4121 via a condenser lens 4117, a dichroic mirror 4118, a bandpass filter 4119, and a pin hole part 4120. The light receiving element 4121 is a photodiode, for example. Fluorescence generated from the analyte in the flow cell 4113 is received by a light receiving element 4122 via the condenser lens 4117 and the dichroic mirror 4118. The light receiving element 4122 is an avalanche photodiode, for example. As the light receiving elements 4116, 4121, 4122, a photomultiplier may be used.

The analog reception light signals (optical signals) outputted from the respective light receiving elements 4116, 4121, 4122 are inputted to the analog processing part 420 via amplifiers 4151, 4152, 4153, respectively.

The analog processing part 420 performs processes such as noise removal and smoothing on the optical signals inputted from the FCM detection part 410, and outputs the processed optical signals to an A/D converter 461.

With reference back to FIG. 24, the measurement unit controller 460 includes the A/D converter 461, an IF (interface) part 462, a bus 463, and IF parts 464, 465.

The A/D converter 461 converts each analog optical signal that is from measurement start to measurement end of the measurement sample and that has been outputted from the analog processing part 420, into digital data. When a plurality of types of optical signals (e.g., optical signals respectively corresponding to forward scattered light intensity, side scattered light intensity, and fluorescence intensity) are generated from a single measurement sample, the A/D converter 461 converts each optical signal from measurement start to measurement end into digital data. For example, as shown in FIG. 25, three types of optical signals (forward scattered light signal, side scattered light signal, and fluorescence signal) are inputted via a plurality of corresponding signal transmission paths 420a to the A/D converter 461. The A/D converter 461 converts each of the optical signals inputted from the plurality of signal transmission paths 420a, into a digital data. Each signal transmission path 420a is configured to transmit an analog optical signal as a differential signal, for example.

The A/D converter 461 compares the signal level of each optical signal and a predetermined threshold, and samples the optical signal that has a signal level higher than the threshold. The A/D converter 461 samples the analog optical signal at a predetermined sampling rate (e.g., sampling at 1024 points at a 10 nanosecond interval, sampling at 128 points at an 80 nanosecond interval, sampling at 64 points at a 160 nanosecond interval, or the like). By executing sampling processes on the three types of optical signals that correspond to each analyte, for example, the A/D converter 461 generates digital data (waveform data) of the forward scattered light signal, digital data (waveform data) of the side scattered light signal, and digital data (waveform data) of the fluorescence signal for each analyte. Each piece of digital data (waveform data) corresponds to one analyte in the specimen.

The A/D converter 461 provides an index to each piece of the generated waveform data. The generated pieces of waveform data are pieces of digital data that respectively correspond to N analytes contained in one specimen, for example. Accordingly, for each analyte, three types of waveform data are generated so as to correspond to the three types of optical signals (forward scattered light signal, side scattered light signal, and fluorescence signal).

The waveform data generated by the A/D converter 461 is transmitted to the analysis unit 300 via the IF parts 462, 465 and the bus 463. The apparatus mechanism part 430, the sample preparation part 440, and the specimen suction part 450 are controlled by the analysis unit 300 via the IF parts 464, 465 and the bus 463.

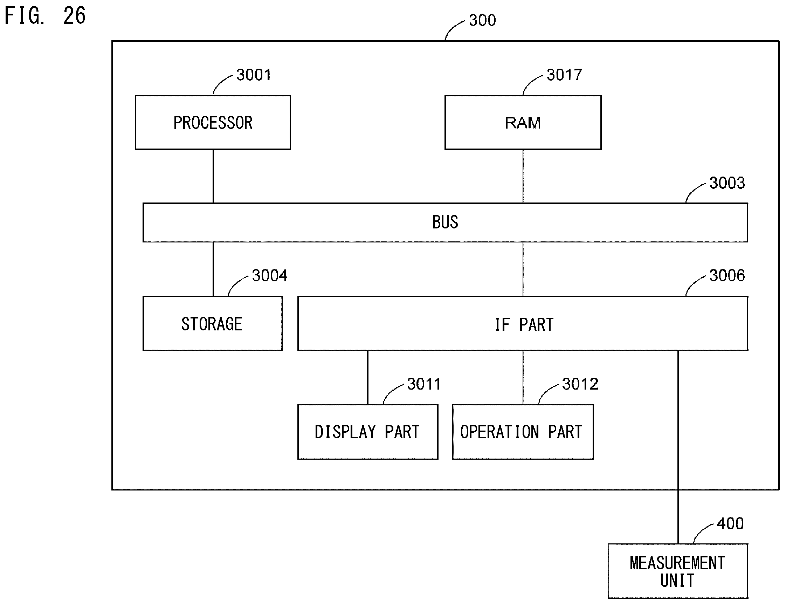

FIG. 26 is a block diagram showing a configuration of the analysis unit 300.

The analysis unit 300 includes a processor 3001, a RAM 3017, a bus 3003, a storage 3004, an IF part 3006, a display part 3011, and an operation part 3012. The analysis unit 300 is implemented by a personal computer, for example. The analysis unit 300 is connected to the measurement unit 400 via the IF part 3006.

The processor 3001 is implemented by a CPU, for example. The processor 3001 executes a program developed on the RAM 3017 from the storage 3004. The RAM 3017 is a so-called main memory. The processor 3001 executes a program for analysis, thereby analyzing waveform data obtained in the measurement unit 400. The processor 3001 executes a program for control, thereby controlling the analysis unit 300 and the measurement unit 400.

The storage 3004 is implemented by a hard disk drive (HDD) or a solid state drive (SSD), for example. The storage 3004 stores waveform data received from the measurement unit 400, a program for controlling the analysis unit 300 and the measurement unit 400, and a program for analyzing waveform data. The program for analyzing waveform data is configured to analyze waveform data on the basis of the calculation processing analysis and the AI analysis described above. The storage 3004 stores a rule for specifying waveform data to serve as a target of each of the AI analysis and the calculation processing analysis, and a rule for selecting an analysis operation.

The display part 3011 is implemented by a liquid crystal display, for example. The display part 3011 is connected to the processor 3001 via the bus 3003 and the IF part 3006. On the display part 3011, an analysis result obtained in the measurement unit 400 is displayed, for example.

The operation part 3012 is implemented by a pointing device and the like including a keyboard, a mouse, and a touch panel, for example. The user such as a doctor or a laboratory technician operates the operation part 3012, to input a measurement order to the specimen analyzer 4000, thereby being able to input a measurement instruction based on the measurement order. By operating the operation part 3012, the user can also input an instruction to display an analysis result. The analysis result includes, for example, a numerical value result, a graph, and a chart that are based on the analysis, flag information provided to the specimen, and the like.

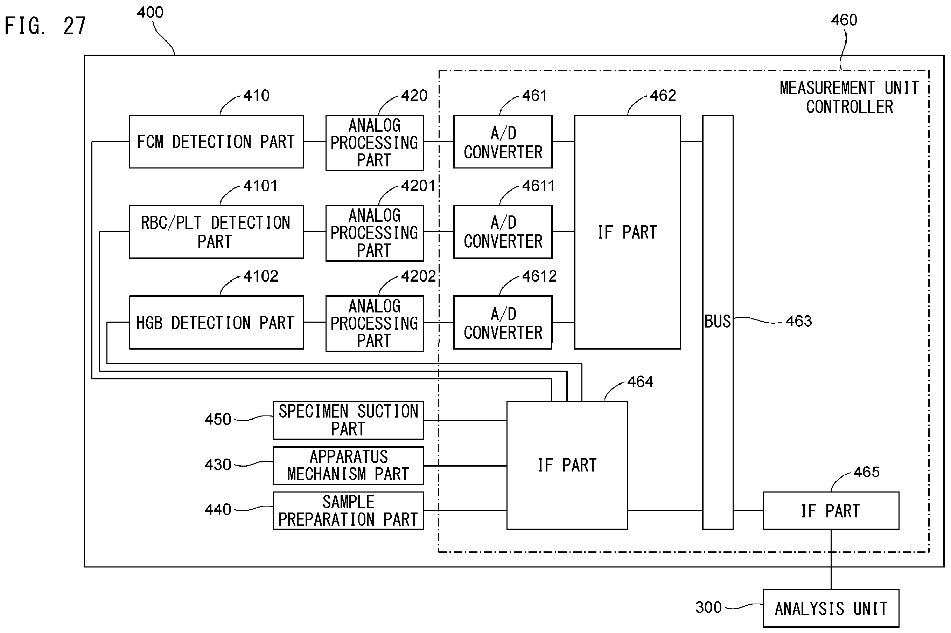

FIG. 27 is a block diagram showing a configuration of the measurement unit 400 in a case where the specimen analyzer 4000 executes counting and classification of blood cells in a blood specimen.

The measurement unit 400 in FIG. 27 further includes, in addition to the configuration in FIG. 24, an RBC/PLT detection part 4101, an HGB detection part 4102, analog processing parts 4201, 4202, and A/D converters 4611, 4612.

The RBC/PLT detection part 4101 is a detection part of an electrical resistance type, and performs measurement of blood cells according to a sheath flow DC detection method on the basis of an RBC/PLT measurement sample. The HGB detection part 4102 performs measurement of hemoglobin according to an SLS-hemoglobin method on the basis of a hemoglobin measurement sample. Data obtained through A/D conversion of an analog signal obtained from each of the RBC/PLT detection part 4101 and the HGB detection part 4102 serves as a target of the calculation processing analysis. From the data based on the RBC/PLT detection part 4101, red blood cells and platelets in the blood specimen are counted. From the data based on the HGB detection part 4102, the hemoglobin content in the blood specimen is obtained.

The data obtained through A/D conversion of the analog signal obtained from each of the RBC/PLT detection part 4101 and the HGB detection part 4102 may serve as a target of the AI analysis. Alternatively, with respect to the data based on the RBC/PLT detection part 4101 and the HGB detection part 4102 as well, the AI analysis and the calculation processing analysis may be selectively used. Accordingly, the load on the analysis unit 300 which processes data can be reduced.

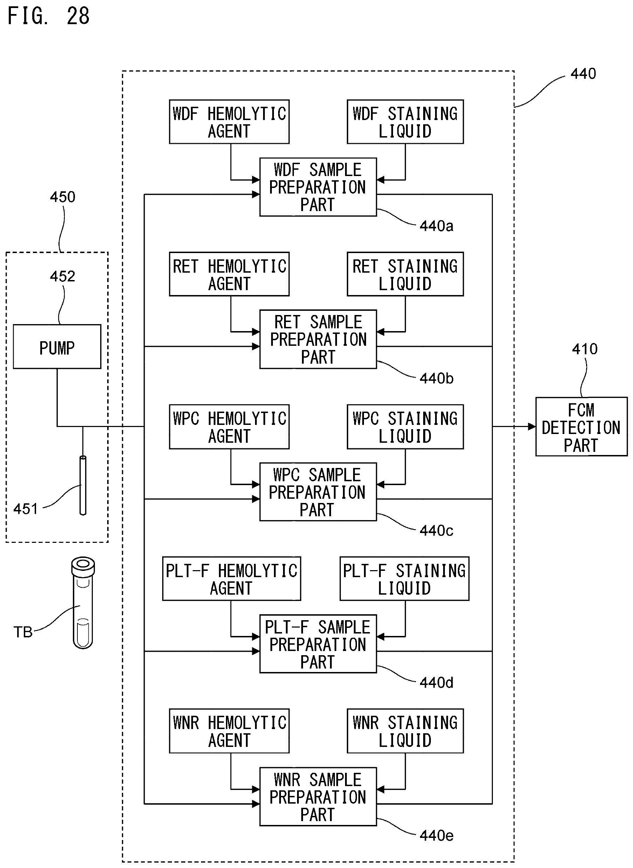

FIG. 28 is a block diagram showing configurations of the specimen suction part 450 and the sample preparation part 440 in the measurement unit 400 in FIG. 27.

The specimen suction part 450 includes: a nozzle 451 for suctioning a blood specimen (e.g., whole blood) from a collection tube TB; and a pump 452 for providing a negative pressure and a positive pressure to the nozzle. The nozzle 451 is moved upwardly and downwardly by the apparatus mechanism part 430 (see FIG. 27), to be inserted into the collection tube TB. When the pump 452 provides a negative pressure in a state where the nozzle 451 is inserted in the collection tube TB, the blood specimen is suctioned via the nozzle 451. The apparatus mechanism part 430 may include a hand member for inverting and stirring the collection tube TB before suctioning blood from the collection tube TB.

The sample preparation part 440 includes a WDF sample preparation part 440a, a RET sample preparation part 440b, a WPC sample preparation part 440c, a PLT-F sample preparation part 440d, and a WNR sample preparation part 440e. The sample preparation parts 440a to 440e each include a reaction chamber for mixing a specimen and a reagent (e.g., hemolytic agent and staining liquid). The sample preparation parts 440a to 440e are used in a WDF channel, a RET channel, a WPC channel, a PLT-F channel, and a WNR channel, respectively.

Here, the specimen analyzer 4000 includes a plurality of measurement channels so as to respectively correspond to a plurality of types of measurement samples that are prepared. The specimen analyzer 4000 includes the WDF channel, the RET channel, the WPC channel, the PLT-F channel, and the WNR channel, for example. The WDF channel is a channel for detecting neutrophils, lymphocytes, monocytes, and eosinophils. The RET channel is a channel for detecting reticulocytes. The WPC channel is a channel for detecting blasts and lymphocytic abnormal cells. The PLT-F channel is a channel for detecting platelets. The WNR channel is a channel for detecting white blood cells other than basophils, basophils, and nucleated red blood cells.

The sample preparation parts 440a to 440e each have connected thereto, via flow paths, a hemolytic agent container containing a hemolytic agent being a reagent corresponding to the measurement channel, and a staining liquid container containing a staining liquid being a reagent corresponding to the measurement channel. For example, the WDF sample preparation part 440a has connected thereto, via flow paths, a hemolytic agent container containing a WDF hemolytic agent (e.g., Lysercell WDF II; manufactured by Sysmex Corporation) being a WDF measurement reagent, and a staining liquid container containing a WDF staining liquid (e.g., Fluorocell WDF; manufactured by Sysmex Corporation) being a WDF measurement reagent. Here, a configuration example in which one sample preparation part is connected to a hemolytic agent container and a staining liquid container is shown. However, one sample preparation part need not necessarily be connected to both of a hemolytic agent container and a staining liquid container, and one reagent container may be used in common by a plurality of sample preparation parts. In addition, each sample preparation part and the corresponding reagent container need not be connected by a flow path. A configuration in which a reagent is suctioned by a nozzle from a reagent container, the nozzle is moved, and the suctioned reagent is discharged from the nozzle into a reaction chamber of the sample preparation part, may be adopted.

Through horizontal and up-down movement by the apparatus mechanism part 430, the nozzle 451 having suctioned a blood specimen is positioned above a reaction chamber of a sample preparation part that corresponds to a measurement order, among the sample preparation parts 440a to 440e. In this state, when the pump 452 provides a positive pressure, the blood specimen is discharged from the nozzle 451 to the corresponding reaction chamber. The sample preparation part 440 supplies a hemolytic agent and a staining liquid that correspond to the reaction chamber having discharged therein the blood specimen, and mixes the blood specimen, the hemolytic agent, and the staining liquid in the reaction chamber, thereby preparing a measurement sample.

A WDF measurement sample is prepared in the WDF sample preparation part 440a, a RET measurement sample is prepared in the RET sample preparation part 440b, a WPC measurement sample is prepared in the WPC sample preparation part 440c, a PLT-F measurement sample is prepared in the PLT-F sample preparation part 440d, and a WNR measurement sample is prepared in the WNR sample preparation part 440e. Each prepared measurement sample is supplied from the reaction chamber to the FCM detection part 410 via a flow path, and measurement of cells by flow cytometry is performed.

The measurement channels (WDF, RET, WPC, PLT-F, WNR) described above correspond to measurement items included in a measurement order. For example, the WDF channel corresponds to a measurement item regarding classification of white blood cells, the RET channel corresponds to a measurement item regarding reticulocytes, the PLT-F channel corresponds to a measurement item regarding platelets, and the WNR channel corresponds to a measurement item regarding the number of white blood cells and nucleated red blood cells. The measurement samples prepared in the measurement channels described above are measured by the FCM detection part 410.

The measurement result by the RBC/PLT detection part 4101 corresponds to a measurement item regarding the number of red blood cells. The measurement result by the HGB detection part 4102 corresponds to a measurement item regarding the hemoglobin content.

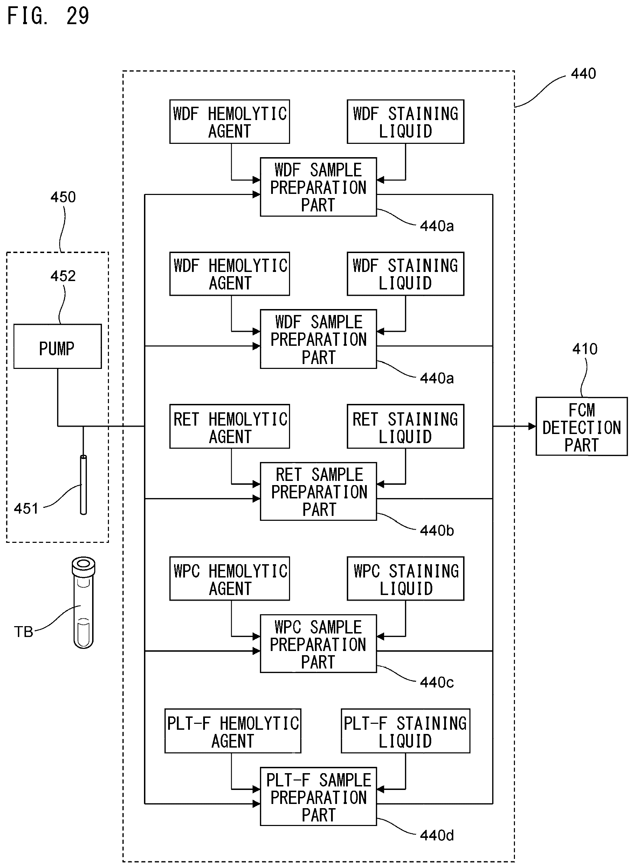

FIG. 29 is a block diagram showing another configuration of the sample preparation part 440 shown in FIG. 28.

In the example shown in FIG. 29, the configuration of the measurement channels of the sample preparation part 440 has been changed in accordance with apportioning between the AI analysis and the calculation processing analysis. Specifically, in the sample preparation part 440 in FIG. 29, when compared with the sample preparation part 440 in FIG. 28, a WDF sample preparation part 440a for a WDF channel and a reagent (WDF hemolytic agent and WDF staining liquid) connected to the WDF sample preparation part 440a have been added, in place of the WNR sample preparation part 440e for the WNR channel and the reagent (WNR hemolytic agent and WNR staining liquid) connected to the WNR sample preparation part 440e. That is, the sample preparation part 440 in FIG. 29 includes two sets of the WDF sample preparation part 440a and the reagent that correspond to the WDF channel. The sample preparation part 440 may include three or more sets of the WDF sample preparation part 440a and the reagent that correspond to the WDF channel.

When the sample preparation part 440 is configured as shown in FIG. 29, classification of basophils and nucleated red blood cells to be performed in the WNR channel is performed in a WDF channel. The analysis unit 300 executes the AI analysis on the waveform data obtained from a measurement sample prepared in the WDF channel, thereby classifying neutrophils, lymphocytes, monocytes, eosinophils, basophils, and nucleated red blood cells. In this case, for example, out of waveform data obtained through measurement according to the WDF channel, waveform data that corresponds to neutrophils, lymphocytes, monocytes, eosinophils, basophils, and nucleated red blood cells is caused to be learned, as teacher data, by the AI algorithm 60 in advance. Accordingly, the AI algorithm 60 that can classify neutrophils, lymphocytes, monocytes, eosinophils, basophils, and nucleated red blood cells from the waveform data of the WDF channels can be generated.

According to the configuration in FIG. 29, for example, in the respective reaction chambers of a plurality of the WDF sample preparation parts 440a, measurement samples of different specimens can be prepared in parallel. Accordingly, measurements regarding the WDF channels on different specimens can be executed in parallel.

In the configuration in FIG. 29, the reaction chamber and the reagent corresponding to the original measurement channel (the WNR channel) are replaced with a reaction chamber and a reagent corresponding to the replacement measurement channel (the WDF channel). In this case, the analysis regarding the original measurement channel needs to be performed in the analysis regarding the replacement measurement channel.

In the configuration in FIG. 29, analysis regarding the original measurement channel (the WNR channel) is executed as the AI analysis, which is performed on the waveform data according to the replacement measurement channel (the WDF channel). Therefore, the original measurement channel (the WNR channel) can be replaced with the replacement measurement channel (the WDF channel). Thus, without increasing the total number of measurement channels provided to the specimen analyzer 4000, the number of measurement channels that are desired to be additionally provided can be increased. When the number of WDF channels is increased, different specimens can be measured in a parallel manner in a plurality of the WDF channels, whereby the throughput of measurement according to the WDF channels is improved. Since apportioning between the AI analysis and the calculation processing analysis is performed, a remarkable effect that the arithmetic operation load on the AI analysis is reduced and that the throughput of specimen processing is also improved can be obtained.

With respect to the process described with reference to FIG. 7, an example in which analysis is performed by either the AI analysis or the calculation processing analysis in accordance with a measurement item has been shown. However, which of the AI analysis and the calculation processing analysis is performed may be determined in accordance with a measurement channel.

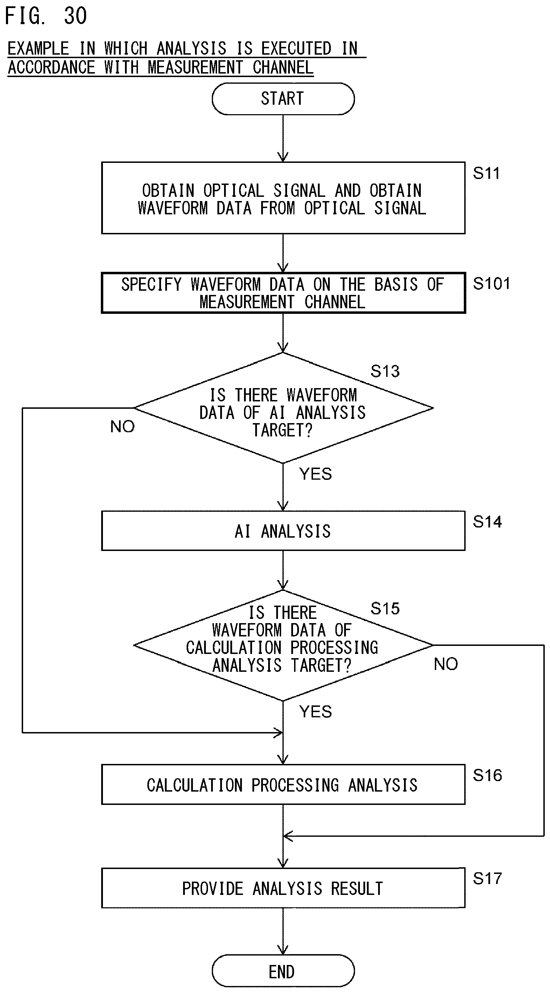

FIG. 30 is a flowchart showing an example in which analysis is executed in accordance with a measurement channel.

In FIG. 30, when compared with FIG. 6, step S101 is added in place of step S12. Hereinafter, changes from FIG. 6 will be described.

In step S101, the analysis unit 300 refers to a rule that includes which of the AI analysis and the calculation processing analysis is performed on the basis of a measurement channel, and on the basis of the rule referred to, the analysis unit 300 specifies waveform data to serve as a target of the AI analysis and waveform data to serve as a target of the calculation processing analysis, with respect to the waveform data obtained in step S11.

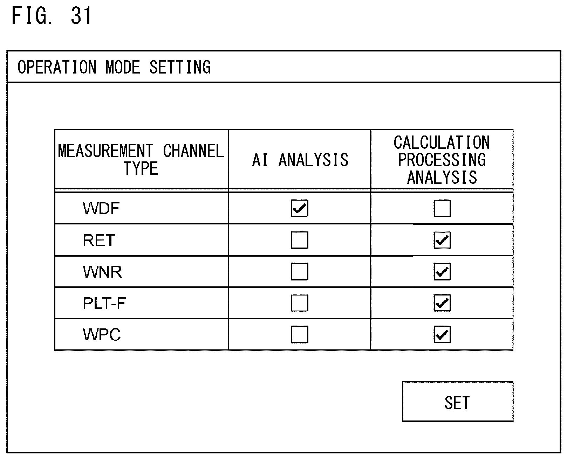

FIG. 31 is an exemplary drawing schematically showing a screen for setting the AI analysis or the calculation processing analysis for each measurement channel. The measurement channels shown as examples in FIG. 31 are those for a blood cell analyzer.

The screen in FIG. 31 is displayed on a display part of the analysis unit 300, for example. The screen in FIG. 31 includes, for each measurement channel, a check box for setting the AI analysis and a check box for setting the calculation processing analysis. The user operates a check box, to select which analysis out of the AI analysis and the calculation processing analysis is to be performed for each measurement channel, and operates a setting button. Accordingly, a rule is stored in a storage of the analysis unit 300.

With reference back to FIG. 30, when waveform data to serve as a target of the AI analysis is included in the waveform data specified in step S101 (S13: YES), the analysis unit 300 executes, in step S14, the AI analysis on the waveform data to serve as a target of the AI analysis specified in step S101. When waveform data to serve as a target of the calculation processing analysis is included in the waveform data specified in step S101 (S15: YES), the analysis unit 300 executes, in step S16, the calculation processing analysis on the waveform data to serve as a target of the calculation processing analysis specified in step S101.

In the case of FIG. 31, since the WDF channel is set as a target of the AI analysis, analysis of the measurement item (e.g., the types of white blood cells) associated with the WDF channel is executed as the AI analysis, which is performed on waveform data obtained from a measurement sample prepared in the WDF channel. Meanwhile, the other channels are set to be targets of the calculation processing analysis, and thus, analyses of measurement items associated with the other channels are executed as the calculation processing analysis, which is performed on waveform data obtained from measurement samples prepared in the other channels.

When the WDF channel is set to be a target of the AI analysis, the AI analysis may be executed with respect to all of the measurement items that correspond to the WDF channel, or the AI analysis may be executed with respect to some measurement items that correspond to the WDF channel and the calculation processing analysis may be executed with respect to the other measurement items.

<Example of analysis method for analyte in specimen>

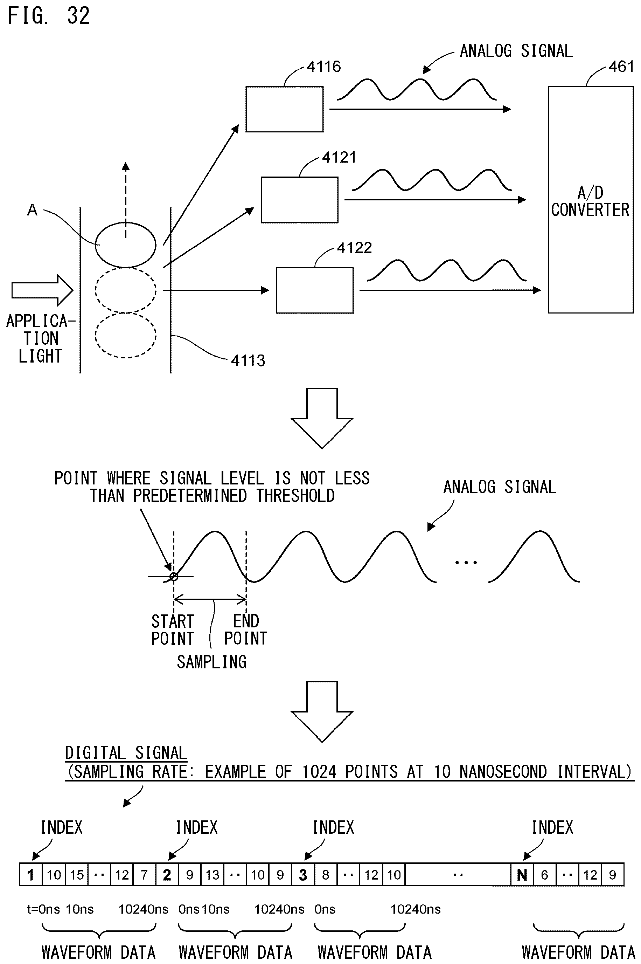

Next, using an example shown in FIG. 32 to FIG. 35, a generation method for training data 75 and an analysis method for waveform data will be described.

<Waveform data>

FIG. 32 is a schematic diagram for describing waveform data to be used in the present analysis method.

As shown in the drawing in the upper part of FIG. 32, when a measurement sample prepared from a specimen containing an analyte A is caused to flow in the flow cell 4113, and light is applied to the analyte A flowing in the flow cell 4113, forward scattered light is generated in a forward direction with respect to the advancing direction of light. Similarly, side scattered light and fluorescence are generated to a side direction with respect to the advancing direction of light. The forward scattered light, the side scattered light, and the fluorescence are respectively received by the light receiving elements 4116, 4121, 4122, and signals according to the received light intensities are outputted. Accordingly, analog optical signals indicating changes in signals associated with the lapse of time are outputted from the light receiving elements 4116, 4121, 4122, respectively. An optical signal corresponding to forward scattered light will be referred to as a "forward scattered light signal", an optical signal corresponding to side scattered light will be referred to as a "side scattered light signal", and an optical signal corresponding to fluorescence will be referred to as a "fluorescence signal". Each optical signal is inputted to the A/D converter 461 and is converted into digital data.

The drawing in the middle part of FIG. 32 schematically shows conversion to digital data performed by the A/D converter 461. Here, an analog optical signal is directly inputted to the A/D converter 461. The optical signal may be converted, without changing the level thereof, into digital data. However, processing such as noise removal, baseline correction, and normalization may be performed as appropriate.

As shown in the drawing in the middle part of FIG. 32, the A/D converter 461 performs sampling of the forward scattered light signal, the side scattered light signal, and the fluorescence signal, from a start point defined as the point when the level of the forward scattered light signal, among the analog optical signals inputted from the light receiving elements 4116, 4121, 4122, has become greater than a predetermined threshold, to an end point defined as the time point when the level of the forward scattered light signal has become lower than the predetermined threshold. From the waveform from the start point to the end point, digital waveform data corresponding to a single analyte is obtained. The A/D converter 461 samples each optical signal at a predetermined sampling rate (e.g., sampling at 1024 points at a 10 nanosecond interval, sampling at 128 points at an 80 nanosecond interval, sampling at 64 points at a 160 nanosecond interval, or the like).

Here, for convenience, a start point and an end point are set to an analog optical signal and waveform data is obtained. However, as described above, after all of the optical signal has been converted into digital data, a start point and an end point may be set to the digital data, and waveform data may be obtained.

The drawing in the lower part of FIG. 32 schematically shows waveform data obtained through sampling. Through the sampling, matrix data (one-dimensional array data) of which elements are values that digitally indicate analog signal levels at a plurality of time points, is obtained as the waveform data that corresponds to a single analyte. The A/D converter 461 generates, for each analyte, waveform data of forward scattered light, waveform data of side scattered light, and waveform data of fluorescence. The A/D converter 461 repeats the generation of waveform data until the obtained number of analytes reaches a predetermined number, or until a predetermined time elapses after a specimen has been caused to flow in the flow cell 4113. Accordingly, digital data composed of waveform data of N analytes contained in a single specimen is obtained. The set (e.g., a set of 1024 digital values obtained every 10 nanoseconds from t=0 ns to t=10240 ns) of sampling data with respect to each analyte corresponds to waveform data.

Each piece of waveform data generated by the A/D converter 461 may be provided with an index for identifying the corresponding analyte. As the indexes, for example, integers of 1 to N are provided in the sequential order of the generated pieces of waveform data, and the waveform data of forward scattered light, the waveform data of side scattered light, and the waveform data of fluorescence that have been obtained from the same analyte are each provided with an identical index. Since an identical index is provided to the pieces of waveform data that correspond to the same analyte, the AI algorithm 60 can analyze, as one set, the waveform data of forward scattered light, the waveform data of side scattered light, and the waveform data of fluorescence that correspond to each individual analyte, and can classify the type of the analyte.

<Generation of training data>

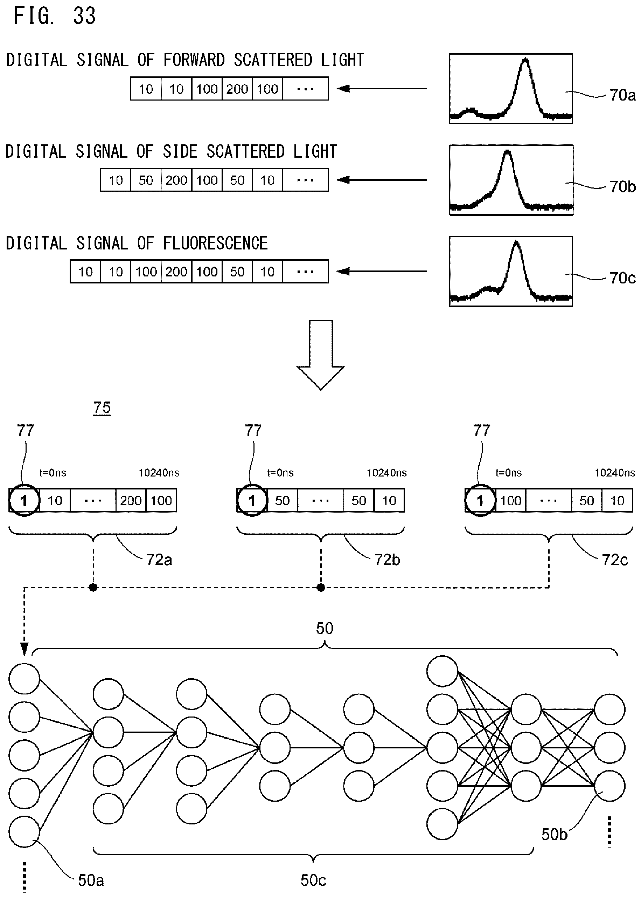

FIG. 33 is a schematic diagram showing an example of a generation method for training data to be used for training an AI algorithm 50 for determining the type of an analyte in a specimen.

As a result of measurement of an analyte according to flow cytometry, an optical signal 70a corresponding to forward scattered light, an optical signal 70b corresponding to side scattered light, and an optical signal 70c corresponding to fluorescence are obtained from the analyte. Pieces of waveform data 72a, 72b, 72c corresponding to the analyte are obtained on the basis of the optical signals 70a, 70b, 70c, respectively. As for the training data 75, for example, it is possible to use waveform data 72a, 72b, 72c of an analyte that has been determined, as a result of performing the calculation processing analysis on analytes in a specimen measured according to flow cytometry, to have a high possibility of being a specific type.

Hereinafter, an example in which the specimen analyzer 4000 serving as a blood cell counter that analyzes a blood specimen is used, will be described.

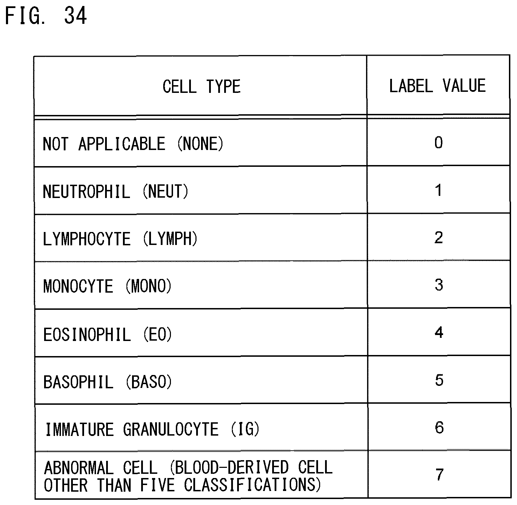

An operator measures a blood specimen by the FCM detection part 410, and accumulates waveform data of forward scattered light, side scattered light, and fluorescence of each individual analyte contained in the specimen. Subsequently, for example, on the basis of the peak value of the waveform data based on side scattered light and the peak value of the waveform data based on fluorescence, the operator classifies each analyte (cell) in the specimen into a group of neutrophil, lymphocyte, monocyte, eosinophil, basophil, immature granulocyte, or abnormal cell. The operator provides a label value 77 that corresponds to the classified cell type, to the waveform data of the cell, thereby obtaining the training data 75. Since the training data 75 is generated for each cell type, the label value 77 is different in accordance with the cell type, as shown in FIG. 34.

At this time, the operator obtains the mode, the average value, or the median of the peak values of the pieces of waveform data based on side scattered light and fluorescence of cells included in the neutrophil group, specifies a representative cell on the basis of the value, and provides waveform data of the specified cell with a label value "1" which corresponds to neutrophil.

The generation method of the training data 75 is not limited thereto. For example, the operator collects only specific cells by a cell sorter, measures each cell according to flow cytometry, and provides the obtained waveform data with a label value for the cell, whereby the operator may obtain the training data 75.

The pieces of the waveform data 72a, 72b, 72c are each combined with a label value 77 that indicates the type of the cell being the source of the data. The training data 75 includes the three pieces of waveform data (waveform data based on the optical signals 70a, 70b, 70c) that correspond to each cell in a state of being associated with each other. Then, the training data 75 is inputted to the AI algorithm 50.

<Outline of deep learning>

An outline of training of a neural network will be described using FIG. 33 as an example.

The AI algorithm 50 is configured as a neural network that includes a middle layer composed of multiple layers. The neural network in this case is a convolutional neural network having a convolution layer, for example. The number of nodes of an input layer 50a in the neural network corresponds to the number of elements of the array included in the waveform data 72a, 72b, 72c of the training data 75 to be inputted. The number of elements of the array is equal to the sum of the number of elements of the waveform data 72a, 72b, 72c of forward scattered light, side scattered light, and fluorescence that correspond to one analyte.

In the example in FIG. 33, each of the waveform data 72a, 72b, 72c includes 1024 elements, and thus, the number of nodes of the input layer 50a is $1024 \times 3 = 3072$. The waveform data 72a, 72b, 72c is inputted to the input layer 50a of the neural network. The label value 77 of each piece of the waveform data of the training data 75 is inputted to an output layer 50b of the neural network, whereby the neural network is trained. A middle layer 50c is positioned between the input layer 50a and the output layer 50b.

<Analysis method for waveform data>

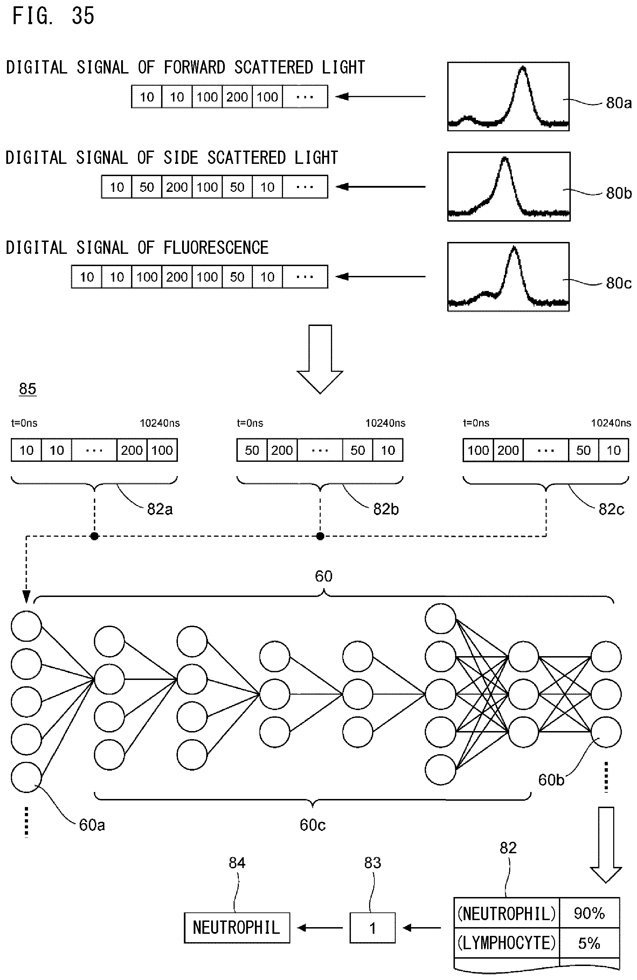

FIG. 35 schematically shows a method for analyzing waveform data of an analyte in a specimen by the AI algorithm 60.

In the analysis method of waveform data shown as an example in FIG. 35, as a result of measurement of an analyte according to flow cytometry, an optical signal 80a corresponding to forward scattered light, an optical signal 80b corresponding to side scattered light, and an optical signal 80c corresponding to fluorescence are obtained from the analyte. Pieces of waveform data 82a, 82b, 82c corresponding to the analyte are obtained on the basis of the optical signals 80a, 80b, 80c, respectively. Then, analysis data 85 composed of the waveform data 82a, 82b, 82c is generated.

Preferably, the analysis data 85 and the training data 75 at least have the same obtaining condition. The obtaining condition includes conditions for measuring an analyte in a specimen according to flow cytometry, e.g., a preparation condition for a measurement sample, the flow speed at which the measurement sample is caused to flow in a flow cell, the intensity of light applied to the flow cell, the amplification factor at light receiving parts that receive scattered light and fluorescence, and the like. The obtaining condition further includes a sampling rate at the time of performing A/D conversion on an analog optical signal.

The analysis data 85 includes the three pieces of waveform data (waveform data based on the optical signals 80a, 80b, 80c) that correspond to each analyte, in a state of being associated with each other. Then, the analysis data 85 is inputted to the trained AI algorithm 60. The AI algorithm 60 is configured as a neural network that includes a middle layer composed of multiple layers.

When the analysis data 85 has been inputted to an input layer 60a of the neural network forming the AI algorithm 60, classification information 82 regarding the type of the analyte that corresponds to the analysis data 85 is outputted from an output layer 60b. A middle layer 60c is positioned between the input layer 60a and the output layer 60b. The classification information 82 includes a probability at which the analyte corresponds to each of a plurality of types. Further, the type having the highest probability is determined to be the type to which the analyte belongs, and a label value 83 being an identifier representing the type and an analysis result 84 being a character string representing the type are outputted.

In the example in FIG. 35, the probability that the type of the analyte corresponding to the analysis data 85 is neutrophil is the highest, and thus, "1" is outputted as the label value 83, and character data "neutrophil" is outputted as the analysis result 84. The label value 83 and the analysis result 84 may be outputted by the AI algorithm 60, but another computer program may output the most preferable label value 83 and analysis result 84 on the basis of the probabilities calculated by the AI algorithm 60.

The analysis method for waveform data in the example shown in FIG. 19 to FIG. 21 above will be described on the basis of FIG. 32 and FIG. 35 described above.

In the case of the example shown in FIG. 19 to FIG. 21, first, the analysis unit 300 analyzes obtained waveform data through calculation processing. Then, the analysis unit 300 executes the AI analysis on waveform data that corresponds to each cell (in the example in FIG. 19 to FIG. 21, monocyte and lymphocyte) of a predetermined type classified through the calculation processing analysis.

When having been classified as a predetermined cell through the calculation processing analysis, the cell is identified by an index for the waveform data in FIG. 32, for example. Accordingly, the pieces of the waveform data classified as monocyte and lymphocyte through the calculation processing analysis are specified by the indexes in the AI analysis. The analysis unit 300 executes the AI analysis on the waveform data specified on the basis of the index, according to the example in FIG. 35. For example, the analysis unit 300 inputs the waveform data specified by the index, into the AI algorithm 60 having learned so as to be able to classify monocytes and lymphocytes in more detail.

The analysis method for waveform data described with reference to FIG. 29 above will be described on the basis of FIG. 32 and FIG. 35 described above.

In the analysis method described with reference to FIG. 29, through the AI analysis of waveform data obtained in, for example, measurement according to the WDF channel, the analysis unit 300 executes classification and counting of eosinophils, neutrophils, lymphocytes, and monocytes in addition to classification and counting of nucleated red blood cells (NRBC) and classification and counting of basophils (BASO). In the case of this example, the AI algorithm 60 has been caused to learn, with waveform data, so as to be able to classify nucleated red blood cells, basophils, eosinophils, neutrophils, lymphocytes, and monocytes. When such an AI algorithm 60 is used, the WNR channel can be replaced with the WDF channel.

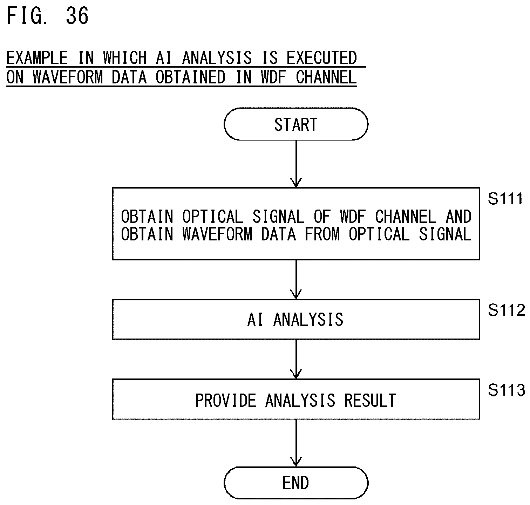

FIG. 36 is a flowchart showing an example in which the AI analysis is executed on waveform data obtained in the WDF channel.

In step S111, the measurement unit 400 obtains optical signals from a measurement sample prepared in the WDF channel, and obtains waveform data from each obtained optical signal. In step S112, the analysis unit 300 executes the AI analysis on the waveform data obtained in step S111. In step S113, the analysis unit 300 provides an analysis result of the waveform data of the WDF channel, and analysis results of waveform data of other channels in combination. How the analyses on the waveform data of the other channels are apportioned between and executed by the AI analysis and the calculation processing analysis is determined on the basis of one of the rules shown as examples in the embodiments described above, for example.

In another analysis method based on the configuration shown in FIG. 29, the analysis unit 300 performs classification and counting of nucleated red blood cells and classification and counting of basophils, through the AI analysis performed on the waveform data obtained in the WDF channel, for example. The analysis unit 300 executes the calculation processing analysis on waveform data that corresponds to cells that have been classified as neither nucleated red blood cells nor basophils, and executes classification and counting of eosinophils, neutrophils, lymphocytes, and monocytes. In the case of this example, the AI algorithm 60 has been caused to learn so as to be able to classify analytes into nucleated red blood cells, basophils, and analytes other than these, from waveform data, for example.

The analysis unit 300 executes the calculation processing analysis on waveform data that corresponds to cells that have been classified as neither nucleated red blood cells nor basophils. For example, the peak value of waveform data that corresponds to each cell that has been classified as neither a nucleated red blood cell nor a basophil is extracted, the cell type is classified on the basis of a two-dimensional graph (scattergram) generated from peak values that correspond to side scattered light and peak values that correspond to fluorescence. For example, on the basis of the two-dimensional graph, which of an eosinophil, a neutrophil, a lymphocyte, a monocyte, and other than these the cell is, is classified. A cell that has been classified as other than an eosinophil, a neutrophil, a lymphocyte, and a monocyte through the analysis based on the two-dimensional graph is classified as debris, for example.

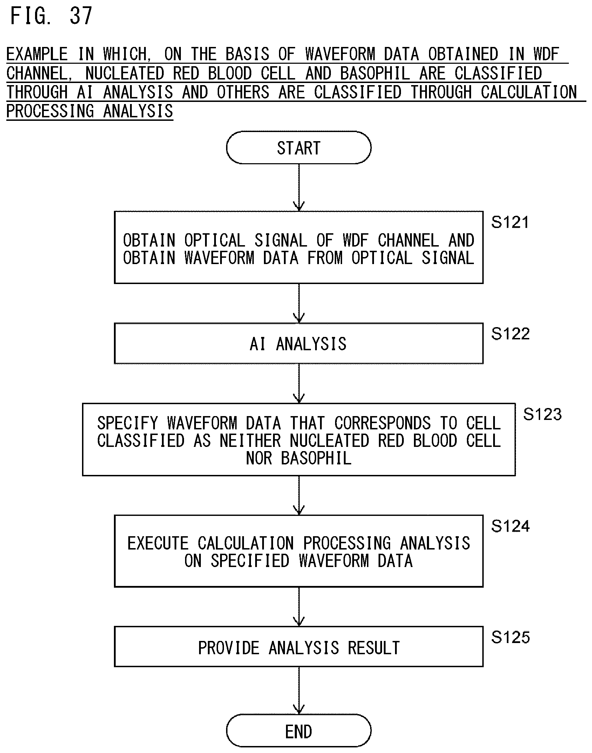

FIG. 37 is a flowchart showing an example in which, on the basis of waveform data obtained in the WDF channel, nucleated red blood cells and basophils are classified through the AI analysis, and the others are classified through the calculation processing analysis.

In step S121, the measurement unit 400 obtains optical signals from a measurement sample prepared in the WDF channel, and obtains waveform data from each obtained optical signal. In step S122, the analysis unit 300 executes the AI analysis on the waveform data obtained in step S121. Accordingly, nucleated red blood cells and basophils are classified. In step S123, the analysis unit 300 specifies waveform data that corresponds to cells that are classified as neither nucleated red blood cells nor basophils.

In step S124, the analysis unit 300 executes the calculation processing analysis on the waveform data specified in step S123. Accordingly, lymphocytes, monocytes, eosinophils, and neutrophils are classified. In step S125, the analysis unit 300 provides the analysis result of the waveform data of the WDF channel and analysis results of the waveform data of other channels in combination.

In another analysis method based on the configuration shown in FIG. 29, the analysis unit 300 executes classification and counting of lymphocytes, classification and counting of monocytes, classification and counting of eosinophils, and classification and counting of neutrophils or basophils through the calculation processing analysis of waveform data obtained in the WDF channel, for example. In the classification and counting of neutrophils or basophils, cells that are classified as either one of neutrophils and basophils are counted, for example. Subsequently, the analysis unit 300 executes the AI analysis on waveform data that corresponds to cells that have been classified as none of lymphocytes, monocytes, eosinophils, and neutrophils or basophils, and cells that have been classified as either one of neutrophils and basophils. Accordingly, the analytes are classified as nucleated red blood cells, basophils, and cells other than these.

For example, from the counting result of cells classified as either neutrophils or basophils through the calculation processing analysis, a counting result of cells classified as basophils through the AI analysis is subtracted, whereby a counting result of neutrophils and a counting result of basophils are calculated. Cells that have been classified as neither nucleated red blood cells nor basophils through the AI analysis are classified as debris, for example.

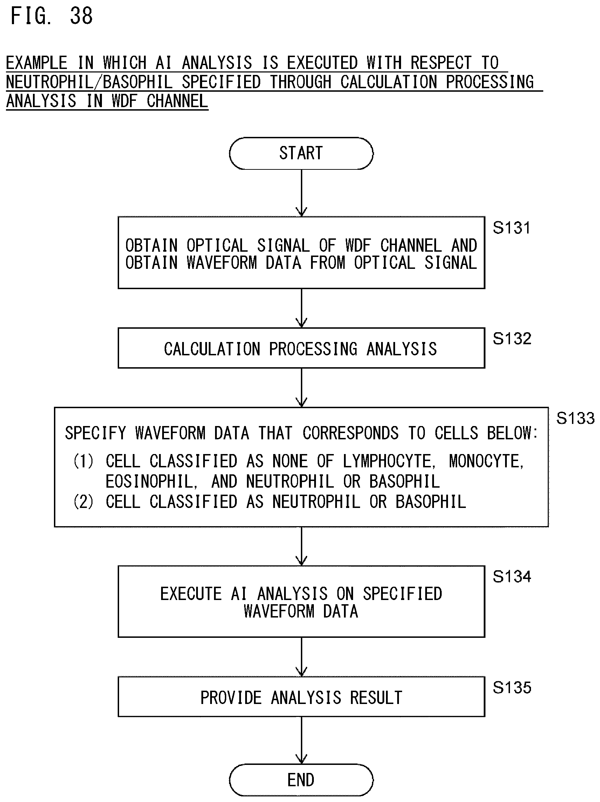

FIG. 38 is a flowchart showing an example in which the AI analysis is executed with respect to neutrophils/basophils specified through the calculation processing analysis in the WDF channel.

In step S131, the measurement unit 400 obtains optical signals from a measurement sample prepared in the WDF channel, and obtains waveform data from each obtained optical signal. In step S132, the analysis unit 300 executes the calculation processing analysis on the waveform data obtained in step S131. Accordingly, groups of lymphocytes, monocytes, eosinophils, and neutrophils and basophils are classified. In step S133, the analysis unit 300 specifies waveform data that corresponds to (1) cells that have been classified as none of lymphocytes, monocytes, eosinophils, and neutrophils or basophils, and (2) cells that have been classified as neutrophils or basophils.

In step S134, the analysis unit 300 executes the AI analysis on the waveform data specified in step S133. Accordingly, neutrophils and basophils are classified. In step S135, the analysis unit 300 provides an analysis result of waveform data of the WDF channel and analysis results of waveform data of other channels in combination.

[Embodiment 5]

In Embodiment 5, a detailed configuration example in which, in the specimen analyzer 4000 that analyzes coagulability of a blood specimen, analysis is executed by being apportioned between the calculation processing analysis and the AI analysis is shown.

An example of a specimen to be measured by the specimen analyzer 4000 of Embodiment 5 is a biological sample collected from a subject. The specimen can include whole blood, plasma, and the like, for example. The specimen analyzer 4000 of Embodiment 5 analyzes the presence or absence of an abnormality due to an interference substance in a specimen, on the basis of a coagulation method, a synthetic substrate method, an immunonephelometry, an agglutination method, a chemiluminescent enzyme immunoassay (CLEIA), or the like. Similar to the configuration example of Embodiment 1 shown in FIG. 1, for example, the specimen analyzer 4000 of Embodiment 5 includes the measurement unit 400 and the analysis unit 300.

(Configuration example)

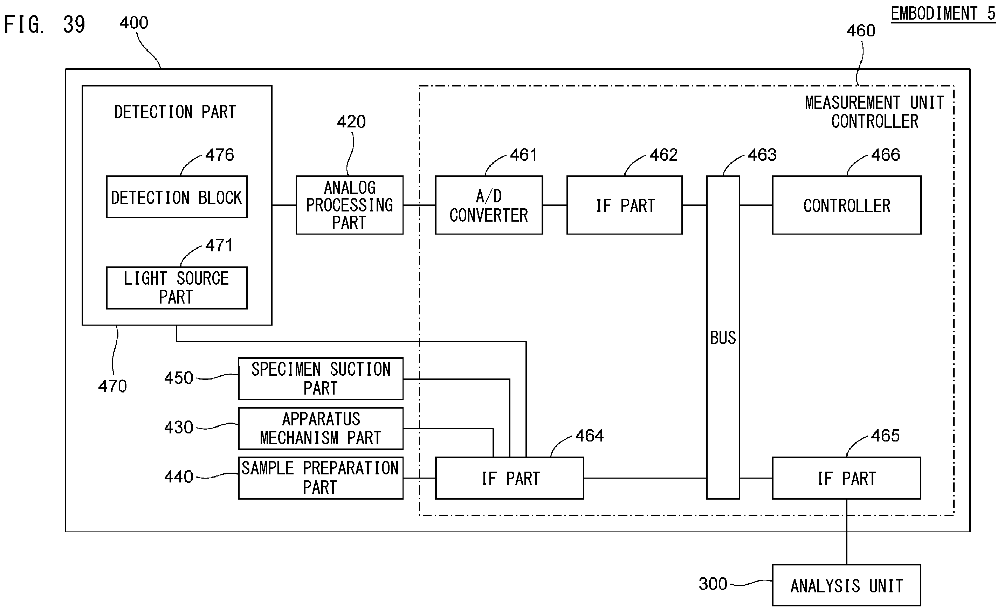

FIG. 39 is a block diagram schematically showing a configuration of the measurement unit 400 according to Embodiment 5.

When compared with the measurement unit 400 shown in FIG. 24, the measurement unit 400 in FIG. 39 includes a detection part 470 in place of the FCM detection part 410, and further includes a controller 466.

The detection part 470 includes a light source part 471 and a detection block 476. The light source part 471 includes a halogen lamp, for example. The light source part 471 is configured to be able to emit, for example, light having a wavelength of 660 nm for blood coagulation time measurement, light having a wavelength of 405 nm for synthetic substrate measurement, and light having a wavelength of 800 nm for immunonephelometric measurement. The sample preparation part 440 mixes a specimen with a blood coagulation reagent to prepare a measurement sample. The detection part 470 applies light from the light source part 471 to the measurement sample composed of the blood coagulation reagent and the specimen, and detects light transmitted through the specimen. The detection part 470 may apply light from the light source part 471 to the measurement sample and detect light scattered by the specimen.

The controller 466 is implemented by an FPGA, for example. The controller 466 is connected to the analysis unit 300 via the bus 463 and the IF part 465. The controller 466 controls each component of the measurement unit 400 on the basis of an instruction from the analysis unit 300.

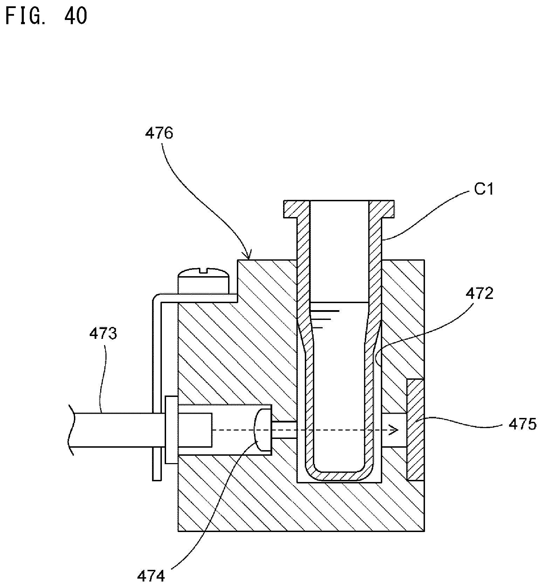

FIG. 40 is a side view schematically showing measurement performed by the detection block 476.

The detection block 476 includes a holding part 472, an optical fiber 473, a condenser lens 474, and a light receiving part 475.

A reaction container C1 containing a measurement sample prepared from a specimen and a reagent corresponding to a measurement item is held in the holding part 472. Accordingly, the measurement sample is left to stand. Light emitted from the light source part 471 (see FIG. 39) is guided by the optical fiber 473 to the condenser lens 474. The condenser lens 474 condenses the light from the optical fiber 473 to the reaction container C1. Transmitted light, which is the light having been condensed at the reaction container C1 and having passed through the measurement sample in the reaction container C1, is received by the light receiving part 475. The light receiving part 475 is a photodiode, for example. The light receiving part 475 outputs an optical signal based on the intensity of the received transmitted light.

With reference back to FIG. 39, the analog processing part 420 processes the analog optical signal outputted from the light receiving part 475 (see FIG. 40), and outputs the resultant analog optical signal to the A/D converter 461. The A/D converter 461 converts the analog optical signal into digital data. As described above, digital data obtained through digital conversion of the optical signal is coagulation waveform data as shown in FIG. 4. The controller 466 transmits the obtained coagulation waveform data to the analysis unit 300.

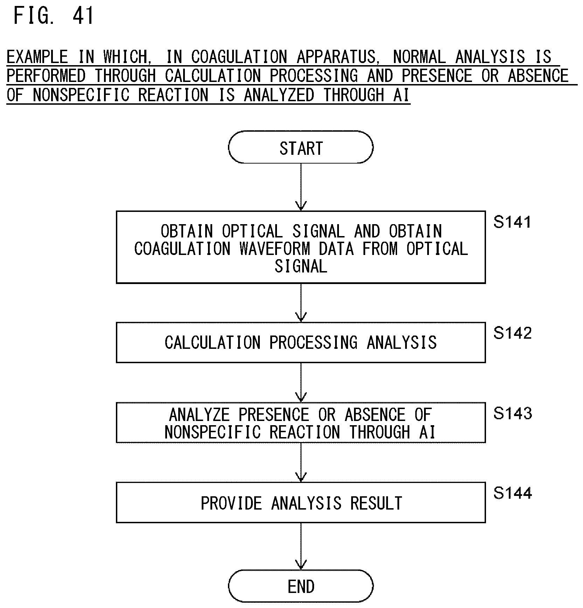

FIG. 41 is a flowchart showing an analysis example according to Embodiment 5. In Embodiment 5, the processor 3001 (see FIG. 26) of the analysis unit 300 executes the calculation processing analysis and the AI analysis on the coagulation waveform data.

In step S141, the measurement unit 400 obtains an optical signal in the detection part 470 and obtains coagulation waveform data from the obtained optical signal. In step S142, the analysis unit 300 executes the calculation processing analysis on the coagulation waveform data obtained in step S141. For example, as described with reference to FIG. 4, the analysis unit 300 obtains a time (T-T2) required for the absorbance of the coagulation waveform data to decrease to 50%, as a result that indicates the time taken for the blood specimen to coagulate.

In step S143, the analysis unit 300 executes the AI analysis on the coagulation waveform data obtained in step S141. Accordingly, the analysis unit 300 obtains information on the presence or absence of an abnormality regarding the measurement, on the basis of the feature amount extracted by the AI algorithm 60 from the coagulation waveform data. On the basis of the presence or absence of an abnormality regarding the measurement, the analysis unit 300 determines whether or not there is a suspected occurrence of nonspecific reaction.

In step S144, the analysis unit 300 provides the result that indicates the time for the blood specimen to coagulate obtained in step S142, and the result that indicates the presence or absence of an abnormality regarding the measurement obtained in step S143.

In FIG. 41, the AI analysis is always executed in step S143. However, the analysis unit 300 may execute the process of step S143 on the basis of a previously-set rule that indicates whether or not the AI analysis is necessary.

Although the specimen analyzer 4000 of Embodiment 5 is a blood coagulation measurement apparatus that optically measures change in the turbidity, of a measurement sample, that is associated with coagulation of a blood specimen, the present disclosure is not limited thereto. For example, the specimen analyzer 4000 of Embodiment 5 may be a blood coagulation measurement apparatus that measures change, in oscillation of a steel ball in a measurement sample, that is associated with change, in the viscosity of the measurement sample, that is associated with coagulation of the blood specimen, on the basis of the received frequency of a high frequency transmitted from a high frequency transmission coil. Although the specimen analyzer 4000 of Embodiment 5 is a blood coagulation measurement apparatus, the specimen analyzer 4000 of Embodiment 5 may be an immunoassay apparatus, a biochemical measurement apparatus, or a gene measurement apparatus.

[Embodiment 6]

In Embodiment 6, a configuration example of the specimen analyzer 4000 that includes a host processor and a parallel-processing processor is shown. In Embodiment 6, in a parallel-processing processor 3002, parallel processing is executed on waveform data, and on the basis of the result of the parallel processing, information regarding the type of each of analytes is generated.

According to Embodiment 6, even when data having a huge volume of several hundred megabytes to several gigabytes per specimen is analyzed, processing regarding waveform data can be executed in parallel by the parallel-processing processor provided separately from the host processor. Therefore, for example, even when data having a huge volume is processed by the AI algorithm 60, processing of the data is concluded in the specimen analyzer 4000. Therefore, for example, it is not necessary to transmit data via the Internet or an intranet to an analysis server that stores the AI algorithm 60. Therefore, according to Embodiment 6, it is not necessary to transmit a large volume of data from the specimen analyzer 4000 to the analysis server, and to obtain an analysis result returning from the analysis server. Thus, the throughput of the specimen analyzer 4000 can be maintained at a high level while the classification accuracy of analytes in the specimen is improved.

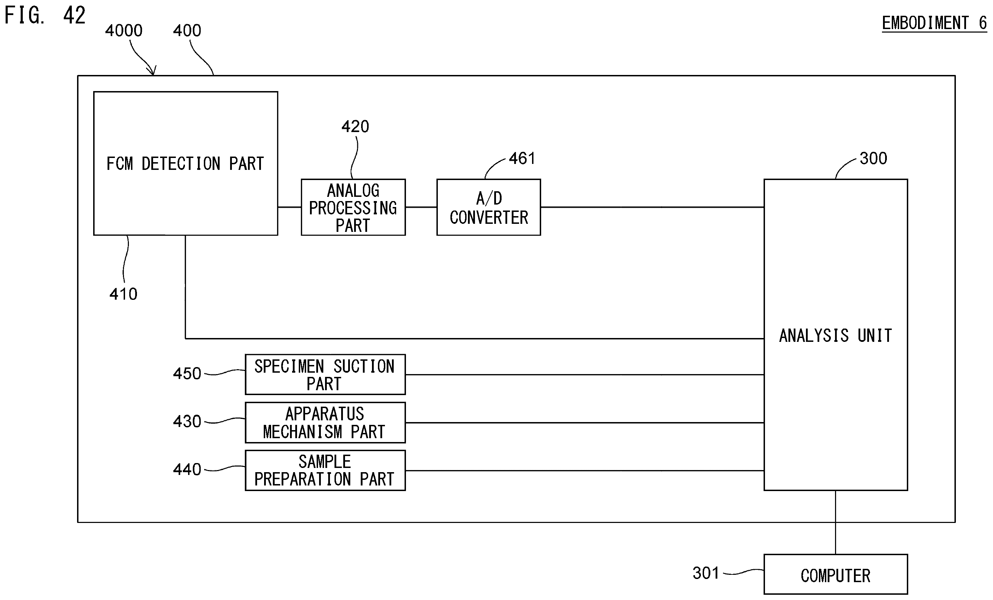

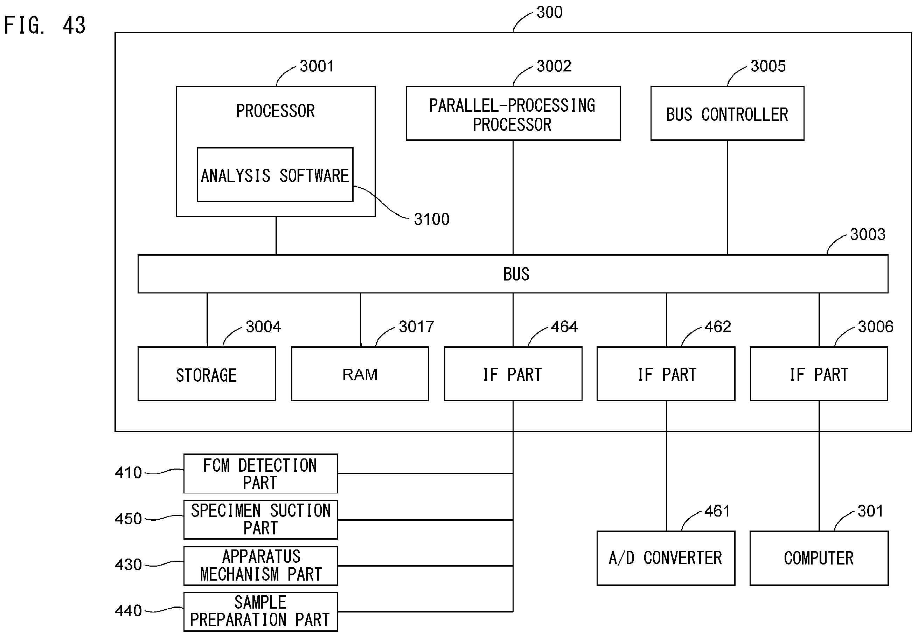

With reference to FIG. 42 and FIG. 43, a configuration of the specimen analyzer 4000 of Embodiment 6 will be described. In the configuration example shown in FIG. 42 and FIG. 43, the measurement unit 400 includes the FCM detection part 410 for measuring a specimen (e.g., blood specimen, urine specimen, body fluid, bone marrow aspirate).

FIG. 42 is a block diagram showing a configuration of the specimen analyzer 4000 according to Embodiment 6.

The specimen analyzer 4000 of Embodiment 6 includes the measurement unit 400, and the analysis unit 300 provided in the measurement unit 400. In the measurement unit 400 of Embodiment 6, when compared with the measurement unit 400 of Embodiment 4 shown in FIG. 24, the IF parts 462, 464, 465 and the bus 463 are omitted. The analysis unit 300 of Embodiment 6 is connected to the A/D converter 461, the apparatus mechanism part 430, the sample preparation part 440, and the specimen suction part 450 in the measurement unit 400, and a computer 301 disposed outside the measurement unit 400.

FIG. 43 is a block diagram showing a configuration of the analysis unit 300 according to Embodiment 6.

When compared with the analysis unit 300 of Embodiment 4 shown in FIG. 26, the analysis unit 300 of Embodiment 6 includes the parallel-processing processor 3002, a bus controller 3005, and the IF parts 462, 464.

The parallel-processing processor 3002 is configured to be able to process, instead of a master processor, arithmetic processes by the AI algorithm 60. By using the parallel-processing processor 3002 suitable for the processes of a matrix operation executed by the AI algorithm 60, it is possible to improve the TAT necessary for the AI analysis. However, although the TAT is improved by the parallel-processing processor 3002, if the data amount of the analysis target is increased, the computer load necessary for the AI analysis is increased. With regard to this, as described above, since data analysis is apportioned between the calculation processing analysis and the AI analysis, the computer load can be reduced, and improvement of the test efficiency can be realized.

Using the parallel-processing processor 3002, the processor 3001 executes an analysis process on waveform data by the AI algorithm 60. That is, the processor 3001 executes analysis software 3100, thereby executing the AI analysis of waveform data based on the AI algorithm 60. The analysis software 3100 is used in order to analyze waveform data that corresponds to each analyte in a specimen, on the basis of the AI algorithm 60.

The analysis software 3100 may be stored in the storage 3004. In this case, the processor 3001 executes the analysis software 3100 stored in the storage 3004, thereby executing the AI analysis of waveform data based on the AI algorithm 60.

In the present embodiment, for example, the AI analysis is executed by the processor 3001 and the parallel-processing processor 3002, and the calculation processing analysis is executed by the processor 3001 without using the parallel-processing processor 3002.

The processor 3001 is a CPU (Central Processing Unit), for example. For example, Core i9, Core i7, or Core i5 manufactured by Intel Corporation, or Ryzen 9, Ryzen 7, Ryzen 5, or Ryzen 3 manufactured by AMD, or the like may be used as the processor 3001.

The processor 3001 controls the parallel-processing processor 3002. The parallel-processing processor 3002 executes parallel processing regarding, for example, a matrix operation in accordance with control by the processor 3001. That is, the processor 3001 is a master processor of the parallel-processing processor 3002, and the parallel-processing processor 3002 is a slave processor of the processor 3001. The processor 3001 is also referred to as a host processor or a main processor. The processor 3001 executes the matrix operation according to the AI algorithm 60, through parallel processing performed by the parallel-processing processor 3002.

The parallel-processing processor 3002 executes in parallel, a plurality of arithmetic processes being at least a part of processes regarding analysis of waveform data. The parallel-processing processor 3002 is a GPU (Graphics Processing Unit), an FPGA (Field Programmable Gate Array), or an ASIC (Application Specific Integrated Circuit), for example. When the parallel-processing processor 3002 is an FPGA, the parallel-processing processor 3002 may have programed therein in advance an arithmetic process regarding the trained AI algorithm 60, for example. When the parallel-processing processor 3002 is an ASIC, the parallel-processing processor 3002 may have incorporated therein in advance a circuit for executing the arithmetic process regarding the trained AI algorithm 60, or may have built therein a programmable module in addition to such an incorporated circuit, for example.

As the parallel-processing processor 3002, GeForce, Quadro, TITAN, Jetson, or the like manufactured by NVIDIA Corporation may be used, for example. In the case of the Jetson series, Jetson Nano, Jetson Tx2, Jetson Xavier, or Jetson AGX Xavier is used, for example.

The processor 3001 executes calculation processing regarding control of the measurement unit 400, for example. The processor 3001 executes calculation processing regarding control signals transmitted/received between the apparatus mechanism part 430, the sample preparation part 440, and the specimen suction part 450, for example. The processor 3001 executes calculation processing regarding transmission/reception of information to/from the computer 301, for example.

The computer 301 has a function of displaying an analysis result transmitted from the analysis unit 300 on the basis of the processing performed by the processor 3001, for example. The computer 301 transmits a measurement order to the analysis unit 300, for example. The measurement order is transmitted from a host computer to the computer 301, for example. It is also possible for the user to input a measurement order via an input device of the computer 301.

The processor 3001 executes processes regarding reading-out of program data from the storage 3004, developing a program onto the RAM 3017, and transmission/reception of data to/from the RAM 3017, for example. The above-described processes executed by the processor 3001 are required to be executed in a predetermined sequential order, for example. For example, when processes needed for control of the apparatus mechanism part 430, the sample preparation part 440, and the specimen suction part 450 are assumed to be A, B, and C, respectively, the processes are required to be executed in a sequential order of B, A, and C, in some cases. The processor 3001 often executes such continuous processes that depend on a sequential order, and thus, even when the number of arithmetic units (each may be referred to as a "processor core", a "core", or the like) is increased, the processing speed is not always increased.

Meanwhile, the parallel-processing processor 3002 executes a large number of regular calculation processes such as arithmetic operations on matrix data including a large number of elements, for example. In the present embodiment, the parallel-processing processor 3002 executes parallel processing in which at least a part of processes of analyzing waveform data in accordance with the AI algorithm 60 are parallelized. The AI algorithm 60 includes a large number of matrix operations, for example. For example, the AI algorithm 60 may include at least 100 matrix operations, or may include at least 1000 matrix operations.

The parallel-processing processor 3002 has a plurality of arithmetic units, and the respective arithmetic units can simultaneously execute matrix operations. That is, the parallel-processing processor 3002 can execute, in parallel, matrix operations by a plurality of respective arithmetic units, as parallel processing. For example, a matrix operation included in the AI algorithm 60 can be divided into a plurality of arithmetic processes that are not dependent on a sequential order with each other. The thus divided arithmetic processes can be executed in parallel by a plurality of arithmetic units, respectively. These arithmetic units may be each referred to as a "processor core", a "core", or the like.

As a result of execution of such parallel processing, speed up of arithmetic processing in the entirety of the specimen analyzer 4000 can be realized. A process such as a matrix operation included in the AI algorithm 60 may be referred to as "Single Instruction Multiple Data (SIMD) processing", for example. The parallel-processing processor 3002 is suitable for such an SIMD operation, for example. Such a parallel-processing processor 3002 may be referred to as a vector processor.

As described above, the processor 3001 is suitable for executing diverse and complicated processes. Meanwhile, the parallel-processing processor 3002 is suitable for executing in parallel a large number of regular processes. Through parallel execution of a large number of regular processes, the TAT required for calculation processing is shortened.

The parallel processing to be executed by the parallel-processing processor 3002 is not limited to matrix operations. For example, when the parallel-processing processor 3002 executes a learning process with respect to the AI algorithm 50, differential operations or the like regarding the learning process can be the target of the parallel processing.

As for the number of arithmetic units of the processor 3001, a dual core (the number of cores: 2), a quad core (the number of cores: 4), or an octa core (the number of cores: 8) is adopted, for example. Meanwhile, the parallel-processing processor 3002 has, for example, at least ten arithmetic units (the number of cores: 10), and can execute in parallel ten matrix operations. The parallel-processing processor 3002 that has several ten arithmetic units also exists. The parallel-processing processor 3002 that has, for example, at least 100 arithmetic units (the number of cores: 100) and that can execute in parallel 100 matrix operations also exists. The parallel-processing processor 3002 that has several hundred arithmetic units also exists. The parallel-processing processor 3002 that has, for example, at least 1000 arithmetic units (the number of cores: 1000) and that can execute in parallel 1000 matrix operations also exists. The parallel-processing processor 3002 that has several thousand arithmetic units also exists.

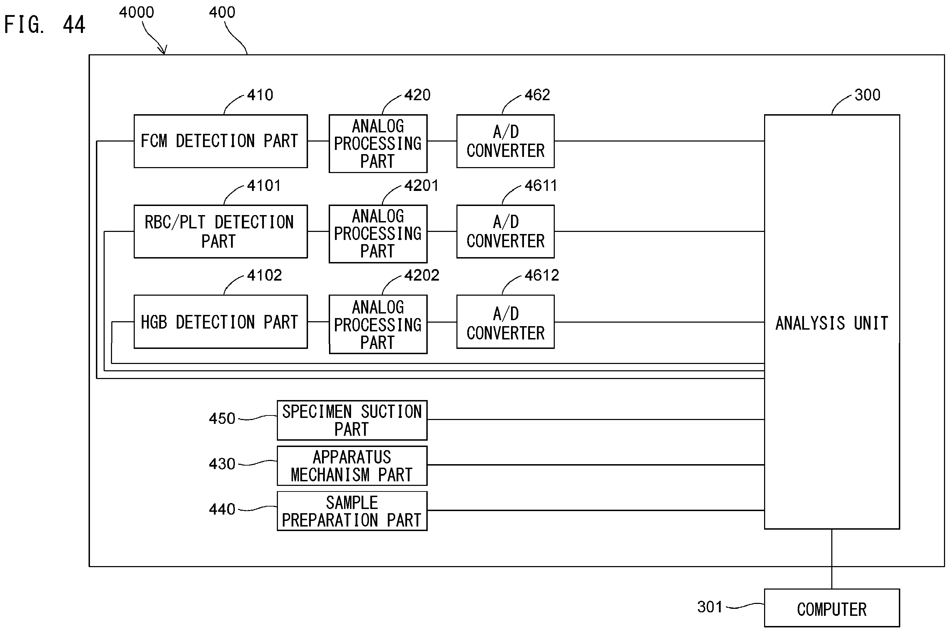

FIG. 44 is a block diagram showing another configuration of the specimen analyzer 4000 according to Embodiment 6. The specimen analyzer 4000 in FIG. 44 executes counting and classification of blood cells in a blood specimen.

When compared with the specimen analyzer 4000 in FIG. 42, the specimen analyzer 4000 in FIG. 44 includes the RBC/PLT detection part 4101, the HGB detection part 4102, the analog processing parts 4201, 4202, and the A/D converters 4611, 4612 similar to those in FIG. 27. The sample preparation part 440 in FIG. 44 is configured so as to be similar to the sample preparation part 440 shown in FIG. 28 or FIG. 29.

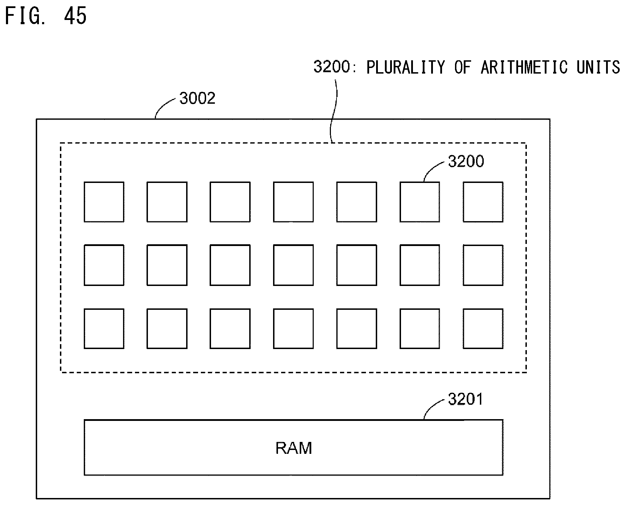

FIG. 45 shows a configuration example of the parallel-processing processor 3002.

The parallel-processing processor 3002 includes a plurality of arithmetic units 3200 and a RAM 3201. The respective arithmetic units 3200 execute arithmetic processes on matrix data in parallel. The RAM 3201 stores data regarding arithmetic processes executed by the arithmetic units 3200. The RAM 3201 is a memory that has a capacity of at least 1 gigabyte. The RAM 3201 may be a memory that has a capacity of 2 gigabytes, 4 gigabytes, 6 gigabytes, 8 gigabytes, 10 gigabytes, or more. Each arithmetic unit 3200 obtains data from the RAM 3201 and executes an arithmetic process. The arithmetic unit 3200 may be referred to as a "processor core", a "core", or the like.

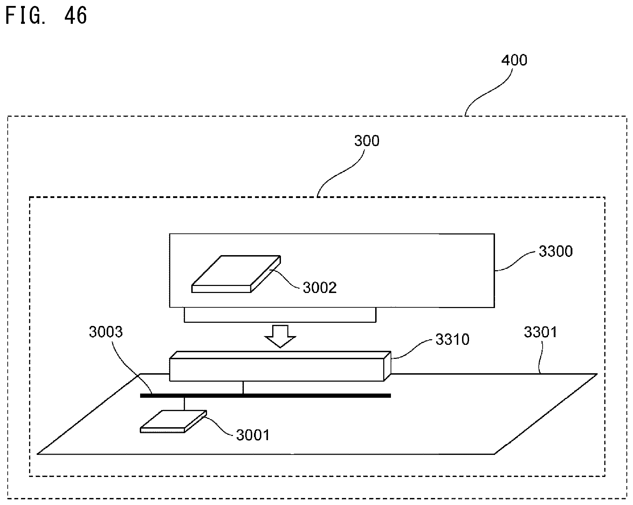

FIG. 46 to FIG. 48 each schematically show an installation example of the parallel-processing processor 3002.

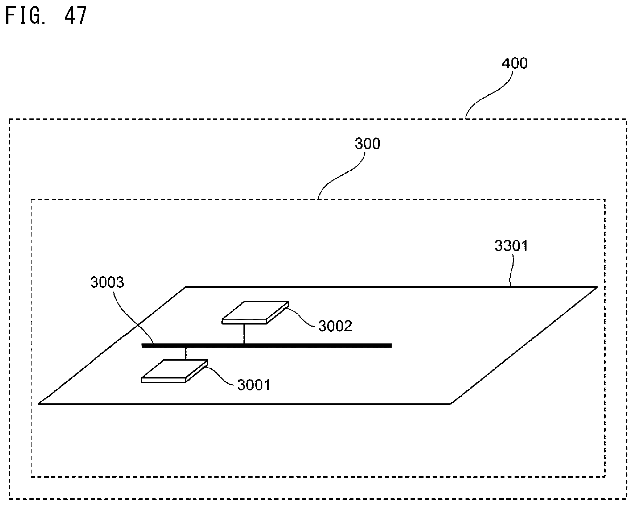

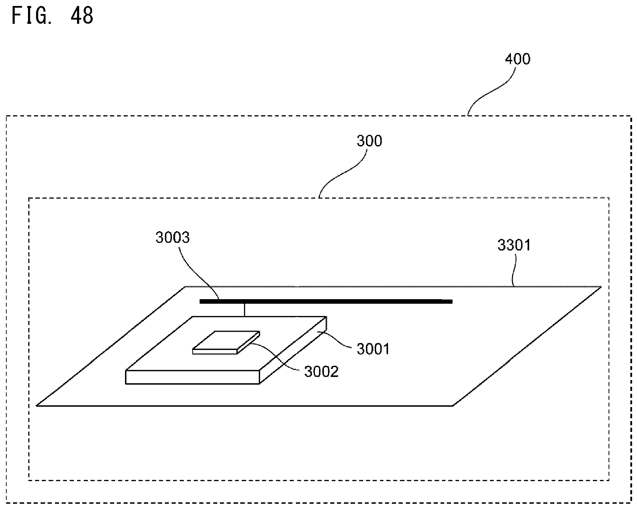

In the example shown in FIG. 46, the processor 3001 is installed on a substrate 3301. The parallel-processing processor 3002 is installed on a graphic board 3300, and the graphic board 3300 is connected to the substrate 3301 via a connector 3310. The processor 3001 is connected to the parallel-processing processor 3002 via the bus 3003. In the example shown in FIG. 47, the parallel-processing processor 3002 is directly installed on the substrate 3301, and connected to the processor 3001 via the bus 3003. In the example shown in FIG. 48, the processor 3001 and the parallel-processing processor 3002 are integrally provided. In this case, the parallel-processing processor 3002 is built in the processor 3001 installed on the substrate 3301.

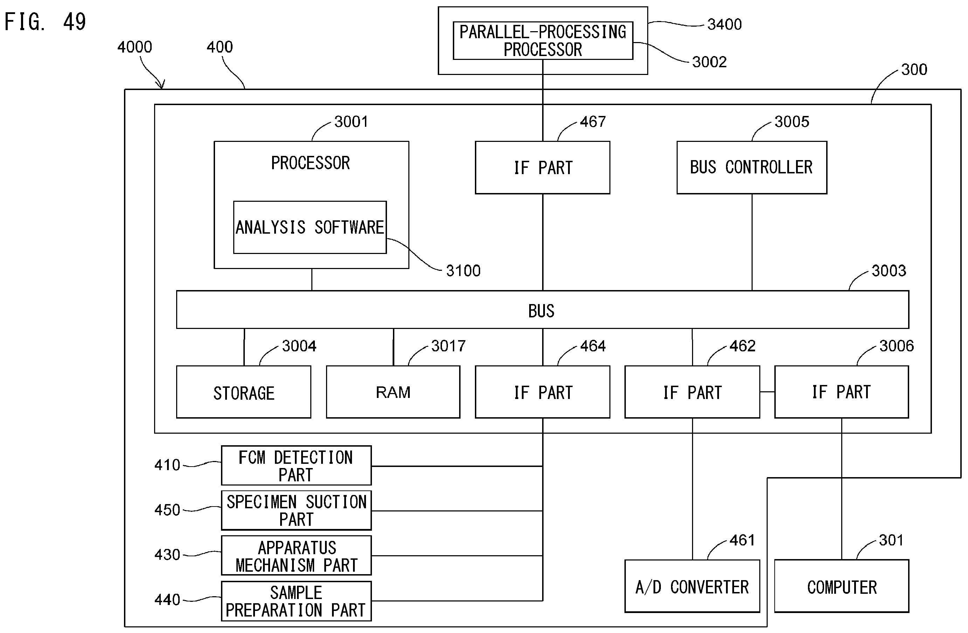

FIG. 49 shows another installation example of the parallel-processing processor 3002.

In the example shown in FIG. 49, the parallel-processing processor 3002 is installed to the measurement unit 400 by means of an external apparatus 3400 connected to the measurement unit 400. The parallel-processing processor 3002 is mounted to the external apparatus 3400 being a USB device, for example. The external apparatus 3400 is connected to the bus 3003 via an IF part 467, whereby the parallel-processing processor 3002 is installed to the specimen analyzer 4000. The USB device may be a small device such as a USB dongle, for example. The IF part 467 is a USB interface having a transfer rate of several hundred Mbps, for example, and more preferably, is a USB interface having a transfer rate of several Gbps to several ten Gbps, or higher. As the external apparatus 3400 having the parallel-processing processor 3002 mounted thereon, Neural Compute Stick 2 manufactured by Intel Corporation may be used, for example.

A plurality of USB devices each having the parallel-processing processor 3002 mounted thereon may be connected to the IF part 467, whereby a plurality of the parallel-processing processors 3002 may be installed to the specimen analyzer 4000. The parallel-processing processor 3002 mounted on one USB device has a smaller number of arithmetic units 3200 than a GPU or the like in some cases. Therefore, if a plurality of USB devices are connected to the measurement unit 400, scale-up of the number cores can be realized.

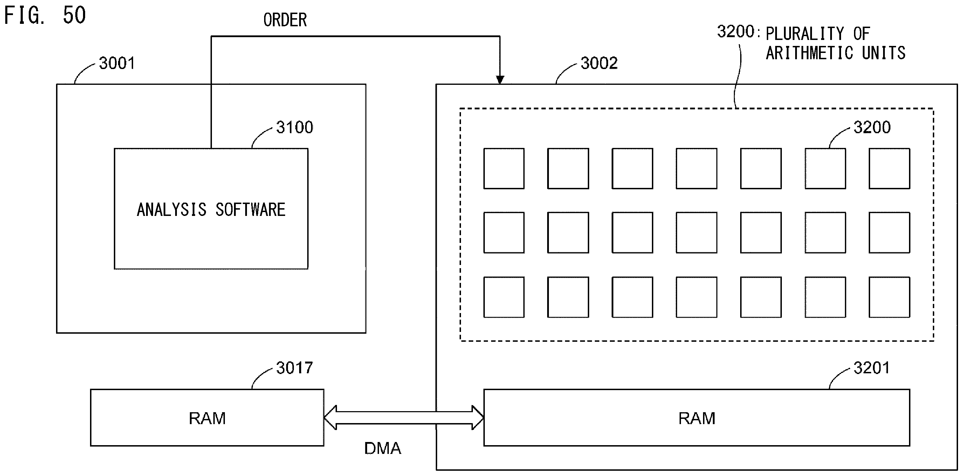

Next, with reference to FIG. 50 to FIG. 52, an outline of arithmetic processes executed by the parallel-processing processor 3002 on the basis of control of the analysis software 3100 which operates on the processor 3001 will be described.

FIG. 50 shows a configuration example of the parallel-processing processor 3002 which executes arithmetic processes.

The parallel-processing processor 3002 includes a plurality of the arithmetic units 3200 and the RAM 3201. The processor 3001, which executes the analysis software 3100, issues an order to the parallel-processing processor 3002, and causes the parallel-processing processor 3002 to execute at least a part of arithmetic processes necessary for analysis of waveform data according to the AI algorithm 60. The processor 3001 orders the parallel-processing processor 3002 to execute arithmetic processes regarding waveform data analysis based on the AI algorithm 60.

All or at least a part of waveform data is stored in the RAM 3017. The data stored in the RAM 3017 is transferred to the RAM 3201 of the parallel-processing processor 3002 by a DMA (Direct Memory Access) method, for example. The plurality of arithmetic units 3200 of the parallel-processing processor 3002 respectively execute, in parallel, arithmetic processes with respect to the data stored in the RAM 3201. Each of the plurality of arithmetic units 3200 obtains necessary data from the RAM 3201, to execute an arithmetic process. Data corresponding to the arithmetic result is stored into the RAM 3201 of the parallel-processing processor 3002. The data corresponding to the arithmetic result is transferred from the RAM 3201 to the RAM 3017 by a DMA method, for example.

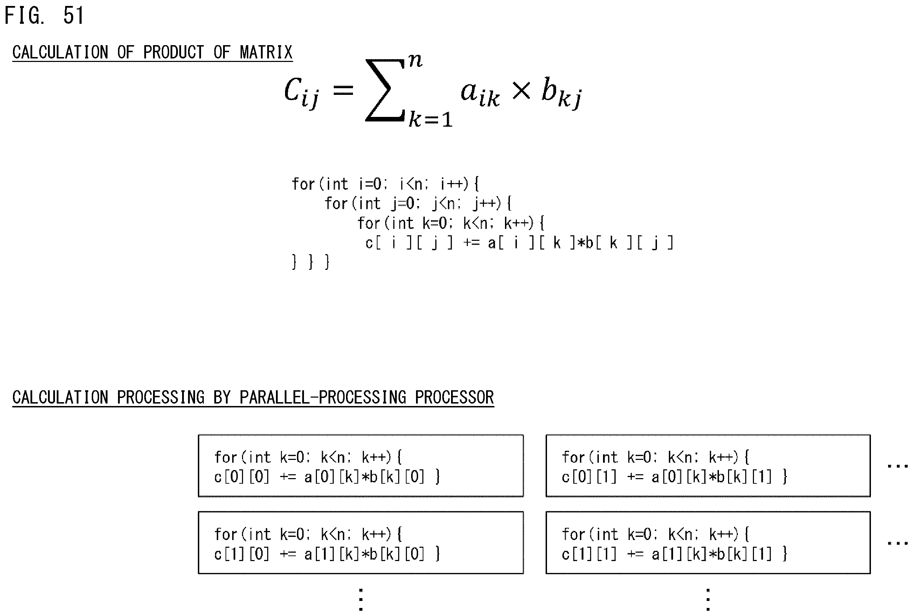

FIG. 51 shows an outline of a matrix operation executed by the parallel-processing processor 3002.

Prior to analyzing waveform data in accordance with the AI algorithm 60, calculation of the product of a matrix (matrix operation) is executed. The parallel-processing processor 3002 executes in parallel a plurality of arithmetic processes regarding the matrix operation, for example.

The drawing in the upper part of FIG. 51 shows a calculation formula of the product of a matrix. In this calculation formula, a matrix c is obtained by a product of a matrix a of n rows × n columns and a matrix b of n rows × n columns. As shown as an example in the drawing in the upper part of FIG. 51, the calculation formula is described in a hierarchical loop syntax. The drawing in the lower part of FIG. 51 shows an example of arithmetic processes executed in parallel in the parallel-processing processor 3002. The calculation formulas shown as an example in the drawing in the lower part of FIG. 51 can be divided into n × n arithmetic processes, n × n being the number of combinations of a loop variable i for the first hierarchical level and a loop variable j for the second hierarchical level, for example. Arithmetic processes thus divided are arithmetic processes that are not dependent on each other, and thus can be executed in parallel.

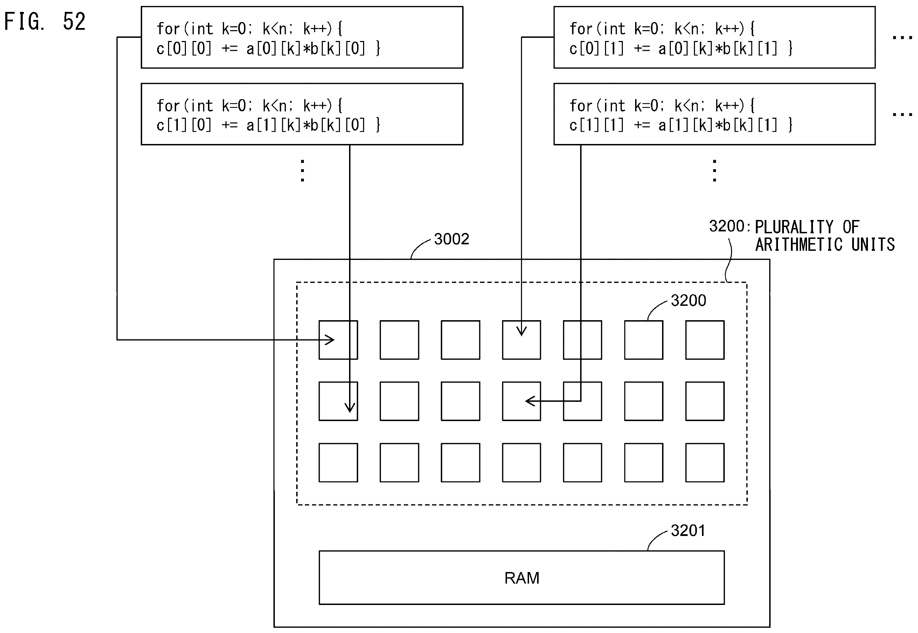

FIG. 52 is a conceptual diagram showing that a plurality of arithmetic processes shown as an example in the drawing in the lower part of FIG. 51 are executed in parallel in the parallel-processing processor 3002.

As shown in FIG. 52, each of the plurality of arithmetic processes is assigned to one of the plurality of arithmetic units 3200 of the parallel-processing processor 3002. The respective arithmetic units 3200 execute in parallel the assigned arithmetic processes. That is, the respective arithmetic units 3200 simultaneously execute the divided arithmetic processes.

Through the arithmetic operations shown as an example in FIG. 51 and FIG. 52 performed by the parallel-processing processor 3002, information regarding the probability at which a cell corresponding to the waveform data belongs to each of a plurality of cell types is obtained, for example. On the basis of the results of the arithmetic operations, the processor 3001, which executes the analysis software 3100, performs analysis regarding the cell type of the cell that corresponds to the waveform data.

The arithmetic operations of the probability at which an analyte in the specimen belongs to each of a plurality of classification types may be performed by a processor different from the parallel-processing processor 3002. For example, the arithmetic results by the parallel-processing processor 3002 may be transferred from the RAM 3201 to the RAM 3017, and on the basis of the arithmetic results read out from the RAM 3017, the processor 3001 may perform arithmetic operations of the information regarding the probability at which the analyte corresponding to each piece of waveform data belongs to each of a plurality of classification types. Alternatively, the arithmetic results by the parallel-processing processor 3002 may be transferred from the RAM 3201 to the analysis unit 300, and a processor installed in the analysis unit 300 may perform arithmetic operations of the information regarding the probability at which the analyte corresponding to each piece of waveform data belongs to each of a plurality of classification types.

The processes shown in FIG. 51 and FIG. 52 are applied to an arithmetic process (also referred to as a filtering process) regarding a convolution layer in the AI algorithm 60, for example.

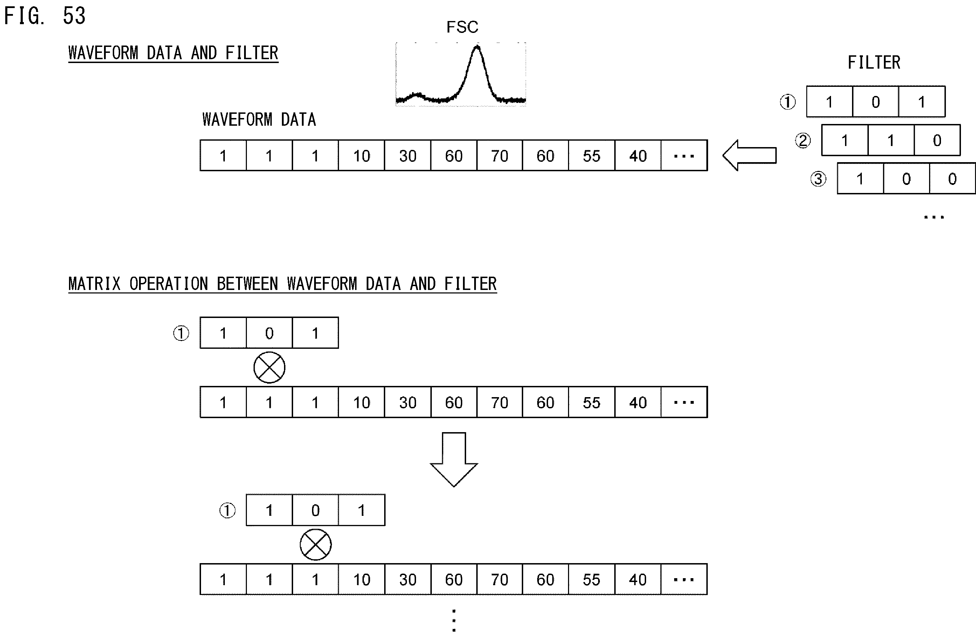

FIG. 53 schematically shows an outline of an arithmetic process regarding a convolution layer.

The drawing in the upper part of FIG. 53 shows waveform data obtained on the basis of forward scattered light, as waveform data that is inputted to the AI algorithm 60. As shown in FIG. 32, the waveform data of the present embodiment is one-dimensional matrix data. To put it more simply, the waveform data is array data in which elements are arranged in a line. Here, for convenience of description, the number of elements of the waveform data is assumed to be n (n is an integer of 1 or greater). The drawing in the upper part of FIG. 53 shows a plurality of filters. Each filter is generated through a learning process of the AI algorithm 50. Each of the plurality of filters is one-dimensional matrix data indicating features of the waveform data. Although each filter shown in the drawing in the upper part of FIG. 53 is matrix data of 1 row × 3 columns, the number of columns is not limited to three. A matrix operation is performed on each filter and the waveform data that is inputted to the AI algorithm 60, whereby features corresponding to the cell type regarding the waveform data are calculated.

The drawing in the lower part of FIG. 53 shows an outline of a matrix operation between waveform data and a filter. The matrix operation is executed while each filter is shifted with respect to the elements of the waveform data, one by one. Calculation of the matrix operation is executed according to Formula 1 below.

[Math 1]

$$\sum_{P=0}^{L-1}\sum_{q=0}^{M-1} h_{pq}x_{i+p,j+q} \quad \cdots \text{ (Formula 1)}$$

In Formula 1, the suffixes of x are variables that indicate the row number and the column number of the waveform data. The suffixes of h are variables that indicate the row number and the column number of the filter. In the example shown in FIG. 53, waveform data is one-dimensional matrix data, and the filter is matrix data of 1 row $\times$ 3 columns, and thus, L=1, M=3, p=0, q=0, 1, 2, i=0, and j=0, 1,..., n-1.

The parallel-processing processor 3002 executes in parallel the matrix operation represented by Formula 1, by means of the plurality of respective arithmetic units 3200. On the basis of the arithmetic processes executed by the parallel-processing processor 3002, classification information regarding the type of each analyte in the specimen is generated. The generated classification information is used in generation and display of a test result of the specimen based on the classification information.

As shown in FIG. 42 and FIG. 43, the computer 301 is connected to the processor 3001 via the IF part 3006 and the bus 3003, and can receive analysis results obtained by the processor 3001 and the parallel-processing processor 3002. The IF part 3006 is a USB interface, for example. The computer 301 receives, via the IF part 3006, the analysis results obtained by the analysis unit 300, and displays the analysis results on a display device of the computer 301.

The computer 301 may include an operation part implemented by a pointing device including a keyboard, a mouse, or a touch panel. The user such as a doctor or a laboratory technician operates the operation part to input a measurement order to the specimen analyzer 4000, thereby being able to input a measurement instruction in accordance with the measurement order. The user can input an instruction for displaying a test result, to the computer 301 via the operation part. By operating the operation part, the user can view various types of information regarding the test result, such as a numerical value result, a graph, a chart, and flag information provided to the specimen that are based on the analysis.

<Operation of specimen analyzer>

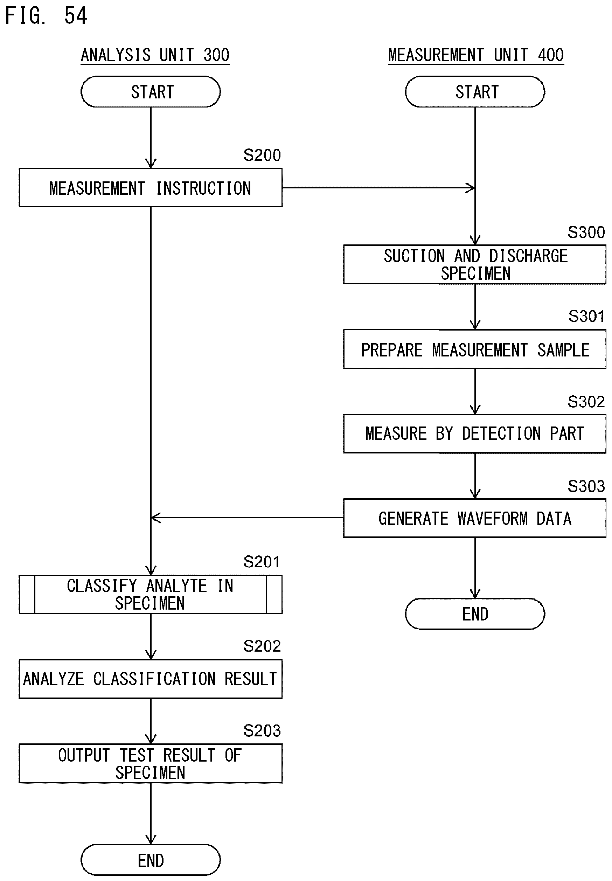

With reference to FIG. 54 to FIG. 56, a specimen analysis operation performed by the specimen analyzer 4000 will be described.

FIG. 54 is a flowchart showing analysis operations performed by the analysis unit 300 and the measurement unit 400.

In step S200, when the processor 3001 of the analysis unit 300 has received a measurement order, the processor 3001 instructs the measurement unit 400 to execute measurement. For example, through the instruction issued to the measurement unit 400, the analysis unit 300 controls operation of each detection part (the FCM detection part 410, the RBC/PLT detection part 4101, the HGB detection part 4102), the specimen suction part 450, and the sample preparation part 440 of the measurement unit 400. The measurement unit 400 starts measurement of a specimen in accordance with the instruction from the analysis unit 300.

In step S300, in accordance with the measurement instruction from the analysis unit 300, the specimen suction part 450 suctions a specimen from a collection tube and discharges the suctioned specimen into a reaction chamber. The measurement instruction from the analysis unit 300 includes information of a measurement channel with respect to which measurement is requested by the measurement order. On the basis of the information of the measurement channel included in the measurement instruction, the specimen suction part 450 discharges the specimen into the reaction chamber of the corresponding measurement channel.

In step S301, in accordance with the measurement instruction from the analysis unit 300, the sample preparation part 440 prepares a measurement sample. Specifically, on the basis of the information of the measurement channel included in the measurement instruction, the sample preparation part 440 supplies a reagent (hemolytic agent and staining liquid) to the reaction chamber having the specimen discharged therein, and mixes the specimen and the reagent. Accordingly, a measurement sample (e.g., WDF measurement sample, RET measurement sample, WPC measurement sample, PLT-F measurement sample, WNR measurement sample) is prepared.

The sample preparation part 440 supplies a reagent to a reaction chamber having the specimen discharged therein, and mixes the specimen and the reagent, to prepare an RBC/PLT measurement sample. The sample preparation part 440 supplies a reagent to a reaction chamber having the specimen discharged therein, and mixes the specimen and the reagent, to prepare a hemoglobin measurement sample.

In step S302, in accordance with the measurement instruction from the analysis unit 300, the FCM detection part 410 measures the prepared measurement sample. Specifically, in accordance with the measurement instruction from the analysis unit 300, the apparatus mechanism part 430 sends the measurement sample in the reaction chamber of the sample preparation part 440 to the FCM detection part 410. The measurement sample sent from the reaction chamber is caused to flow in the flow cell 4113, and is irradiated with laser light by the light source 4111 (see FIG. 25). When an analyte contained in the measurement sample passes through the flow cell 4113, light is applied to the analyte. Then, forward scattered light, side scattered light, and fluorescence generated from the analyte are detected by the light receiving elements 4116, 4121, 4122, respectively, and analog optical signals according to the received light intensities are outputted. Each optical signal is processed by the analog processing part 420, and then is outputted to the A/D converter 461.

The RBC/PLT detection part 4101 performs measurement of blood cells by a sheath flow DC detection method on the basis of the RBC/PLT measurement sample. The HGB detection part 4102 performs measurement of hemoglobin by an SLS-hemoglobin method on the basis of the hemoglobin measurement sample. An analog signal detected by the RBC/PLT detection part 4101 is processed by the analog processing part 4201, and then outputted to the A/D converter 4611. An analog signal detected by the HGB detection part 4102 is processed by the analog processing part 4202, and then outputted to the A/D converter 4612 (see FIG. 27).

In step S303, as described above, the A/D converter 461 generates digital data by sampling at a predetermined rate the analog optical signal, and generates waveform data that corresponds to each of analytes on the basis of the digital data. The waveform data generated by the A/D converter 461 is transferred directly to a RAM by, for example, DMA transfer, not via the processor 3001 of the analysis unit 300. Accordingly, waveform data based on a forward scattered light signal, waveform data corresponding to side scattered light, and waveform data corresponding to fluorescence, which have been obtained from each analyte, are taken into the RAM 3017.

The A/D converter 4611 generates digital data by sampling at a predetermined rate the analog signal from the RBC/PLT detection part 4101. The A/D converter 4612 generates digital data by sampling at a predetermined rate the analog signal from the HGB detection part 4102. These pieces of digital data may also be taken into the RAM 3017.

In step S201, the processor 3001 of the analysis unit 300 executes the AI analysis on the waveform data by using the AI algorithm 60, and executes the calculation processing analysis with respect to a representative value, of the waveform data, that corresponds to a feature of the analyte. Apportioning between the AI analysis and the calculation processing analysis has been described above. Accordingly, the analyte in the specimen is classified. Although the process of the AI analysis in step S201 will be described later, the processor 3001 obtains, as a result of the process using the parallel-processing processor 3002, classification information 82 of each individual analyte in the specimen, for example, and obtains a label value 83 and an analysis result 84 (see FIG. 35).

In step S202, the processor 3001 analyzes the label value 83 and the analysis result 84 by using a program stored in the storage 3004, and generates a test result of the specimen. In step S202, for example, on the basis of the label value 83 and the analysis result 84 of each individual analyte, the number of analytes is counted for each type of analyte.

For example, in a case of an example in which a test of blood cells in a blood specimen is performed, if, in one specimen, there are N pieces of classification information provided with a label value "1" which indicates neutrophil, a counting result that the number of neutrophils = N is obtained as a test result of the specimen. The processor 3001 obtains the counting result regarding the measurement item corresponding to the measurement channel on the basis of the analysis results 84, and stores the counting result, together with identification information of the specimen, into the storage 3004.

Here, the measurement item corresponding to the measurement channel is an item of which the counting result is requested by the measurement order. For example, a measurement item corresponding to the WDF channel includes a measurement item of the number of five classifications of white blood cells, i.e., monocytes, neutrophils, lymphocytes, eosinophils, and basophils. A measurement item corresponding to the RET channel includes a measurement item of the number of reticulocytes. A measurement item corresponding to PLT-F includes a measurement item of the number of platelets. A measurement item corresponding to WPC includes a measurement item of the number of hematopoietic progenitor cells. A measurement item corresponding to WNR includes a measurement item of the number of white blood cells and nucleated red blood cells.

The counting result is not limited to that of an item (also referred to as "reportable item") for which measurement as listed above is requested, and can include a counting result of another cell of which measurement can be performed in the same measurement channel. For example, in the case of the WDF channel, as shown in FIG. 34, immature granulocytes (IG) and abnormal cells are also included in the counting result in addition to the five classifications of white blood cells.

Further, the processor 3001 analyzes the obtained counting result to generate a test result of the specimen, and stores the result into the storage 3004. The analysis of the counting result includes performing determination as to, for example, whether the counting result is in a normal value range, whether any abnormal cell has been detected, whether separation from the previous test result is in an allowable range, and the like.

In step S203, the computer 301 displays the test result generated by the analysis unit 300, on a display part.

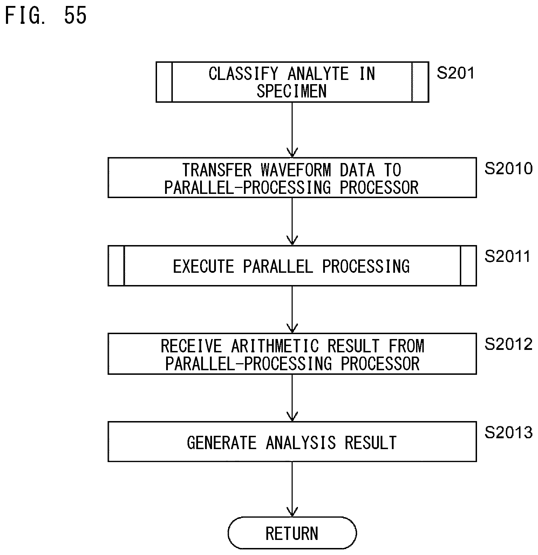

FIG. 55 is a flowchart showing details of the AI analysis performed in step S201 in FIG. 54.

Step S201 is executed by the processor 3001 in accordance with operation of the analysis software 3100.

In step S2010, the processor 3001 causes the waveform data taken into the RAM 3017 in step S303, to be transferred to the parallel-processing processor 3002. As shown in FIG. 50, the waveform data is DMA-transferred from the RAM 3017 to the RAM 3201. At this time, for example, the processor 3001 controls the bus controller 3005 to DMA-transfer the waveform data from the RAM 3017 to the RAM 3201.

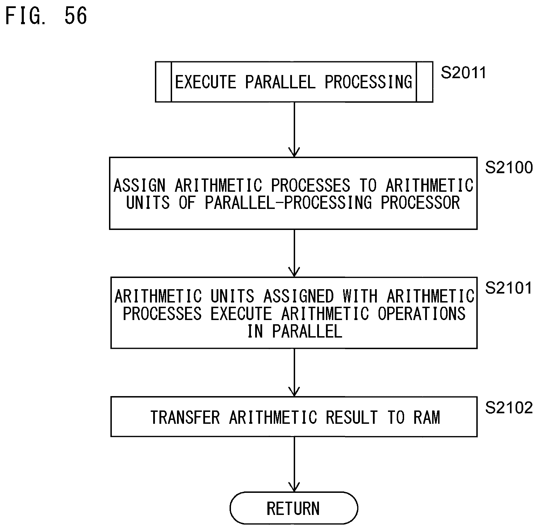

In step S2011, the processor 3001 instructs the parallel-processing processor 3002 to execute parallel processing on the waveform data. The processor 3001 instructs the execution of parallel processing by calling a kernel function of the parallel-processing processor 3002, for example. The process executed by the parallel-processing processor 3002 will be described later with reference to FIG. 56. The processor 3001 instructs the parallel-processing processor 3002 to execute a matrix operation regarding the AI algorithm 60, for example. The waveform data corresponding to each of the analytes in the specimen is inputted to the AI algorithm 60. The waveform data inputted to the AI algorithm 60 is subjected to arithmetic operations performed by the parallel-processing processor 3002.

In step S2012, the processor 3001 receives results of arithmetic operations executed by the parallel-processing processor 3002. The arithmetic results are DMA-transferred from the RAM 3201 to the RAM 3017 as shown in FIG. 50. In step S2013, on the basis of the arithmetic results by the parallel-processing processor 3002, the processor 3001 generates an analysis result of the type of each analyte.

FIG. 56 is a flowchart showing details of step S2011 in FIG. 55.

Step S2011 is executed by the parallel-processing processor 3002 on the basis of an instruction from the processor 3001.

In step S2100, the processor 3001, which executes the analysis software 3100, causes the parallel-processing processor 3002 to execute assignment of arithmetic processes to the arithmetic units 3200. For example, the processor 3001 causes the parallel-processing processor 3002 to execute assignment of arithmetic processes to the arithmetic units 3200, by calling a kernel function of the parallel-processing processor 3002. As shown in FIG. 52, for example, a matrix operation regarding the AI algorithm 60 is divided into a plurality of arithmetic processes, and the respective divided arithmetic processes are assigned to the arithmetic units 3200. Waveform data corresponding to each of the analytes in the specimen is inputted to the AI algorithm 60. A matrix operation corresponding to the waveform data is divided into a plurality of arithmetic processes, to be assigned to the arithmetic units 3200.

In step S2101, the arithmetic processes are processed in parallel by a plurality of arithmetic units 3200. The arithmetic processes are executed on the plurality of pieces of waveform data. In step S2102, arithmetic results generated through the parallel processing by the plurality of arithmetic units 3200 are transferred from the RAM 3201 to the RAM 3017. The arithmetic results are DMA-transferred from the RAM 3201 to the RAM 3017 as shown in FIG. 50.

In step S201 in FIG. 54, the processor 3001 of the analysis unit 300 may use the AI algorithm 60 on digital data based on the analog signal from the RBC/PLT detection part 4101, thereby obtaining an analysis result of a measurement item (e.g., the number of red blood cells, hematocrit value, or the like) that corresponds to the RBC/PLT channel. Further, the processor 3001 may use the AI algorithm 60 on digital data based on the analog signal from the HGB detection part 4102, thereby obtaining an analysis result of a measurement item (e.g., hemoglobin content, or the like) with respect to the HGB channel.

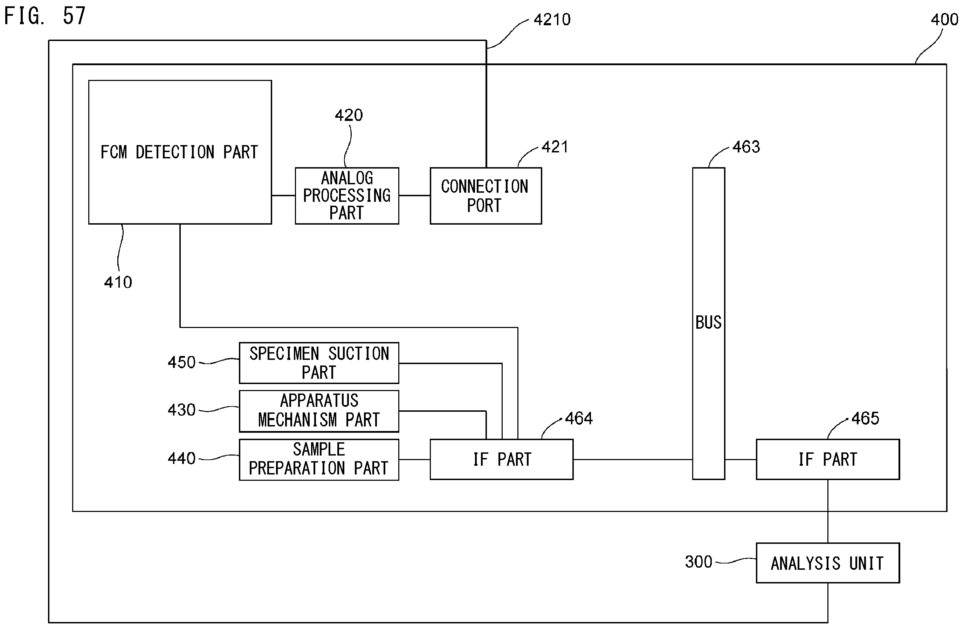

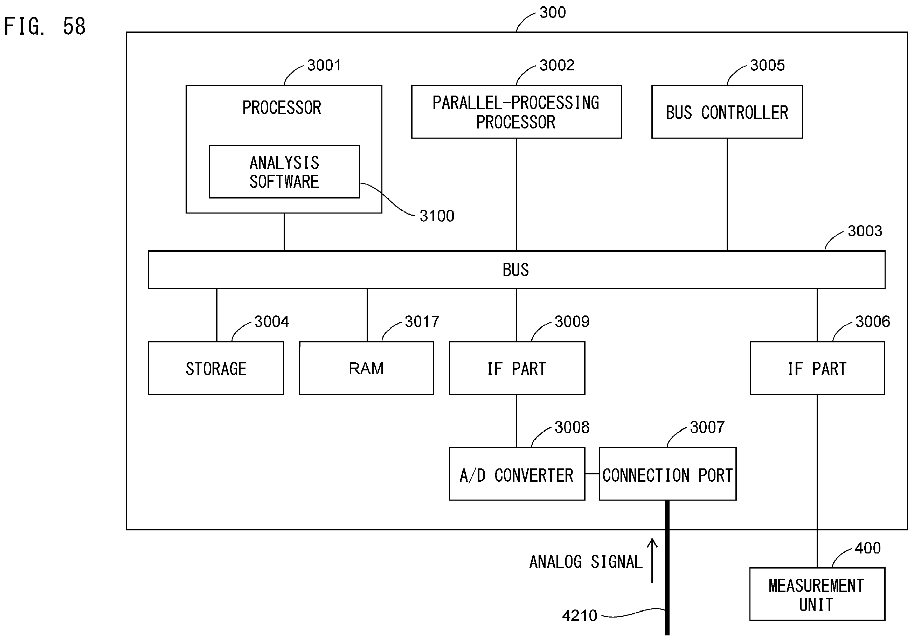

Next, with reference to FIG. 57 and FIG. 58, another configuration example of the specimen analyzer 4000 composed of the measurement unit 400 and the analysis unit 300 will be described.

FIG. 57 is a block diagram showing another configuration of the measurement unit 400.

In the example shown in FIG. 57, the analog optical signal processed by the analog processing part 420 is transmitted to the analysis unit 300 via a connection port 421. A connection cable 4210 is connected to the connection port 421. The other configurations shown in FIG. 57 have configurations and functions similar to those of the measurement unit 400 in the embodiments described above.

FIG. 58 is a block diagram showing another configuration of the analysis unit 300.

In the example shown in FIG. 58, the analysis unit 300 is connected to the measurement unit 400 via the IF part 3006. The bus 3003 is a transmission line having a data transfer rate of not less than several hundred MB/s, for example. The bus 3003 may be a transmission line having a data transfer rate of not less than 1 GB/s. The bus 3003 performs data transfer on the basis of the PCI-Express or PCI-X standard, for example. Configurations of the processor 3001, the parallel-processing processor 3002, the storage 3004, and the RAM 3017, and processes executed by these are similar to the configurations and the processes described above.

The analysis unit 300 includes a connection port 3007, an A/D converter 3008, and an IF part 3009.

The connection port 3007 is connected to the connection port 421 (see FIG. 57) of the measurement unit 400 via the connection cable 4210. The connection cable 4210 includes transmission paths of which the number corresponds to the types of analog signals transmitted from the measurement unit 400 to the analysis unit 300, for example. For example, the connection cable 4210 is implemented by twisted-pair cables, and has pairs of wires, the number of the pairs corresponding to the types of analog signals transmitted to the analysis unit 300. For noise reduction during signal transmission, the connection cable 4210 preferably has a length of 1 meter or less, for example.

The A/D converter 3008 is connected to the connection port 3007. As described above, the A/D converter 3008 samples each analog optical signal outputted from the measurement unit 400, to generate waveform data that corresponds to each analyte in the specimen. The generated waveform data is stored into the storage 3004 or the RAM 3017 via the IF part 3009 and the bus 3003. The transmission path from the connection port 3007 to the A/D converter 3008 may have wires of which the number corresponds to the types of optical signals transmitted to the analysis unit 300.

The processor 3001 and the parallel-processing processor 3002 execute arithmetic processes on the waveform data stored in the storage 3004 or the RAM 3017. The analysis software 3100, which operates on the processor 3001, is similar to the analysis software 3100 shown in FIG. 50. By executing the analysis software 3100, the processor 3001 generates, through operations similar to those described above, classification information regarding the type of each analyte in the specimen.

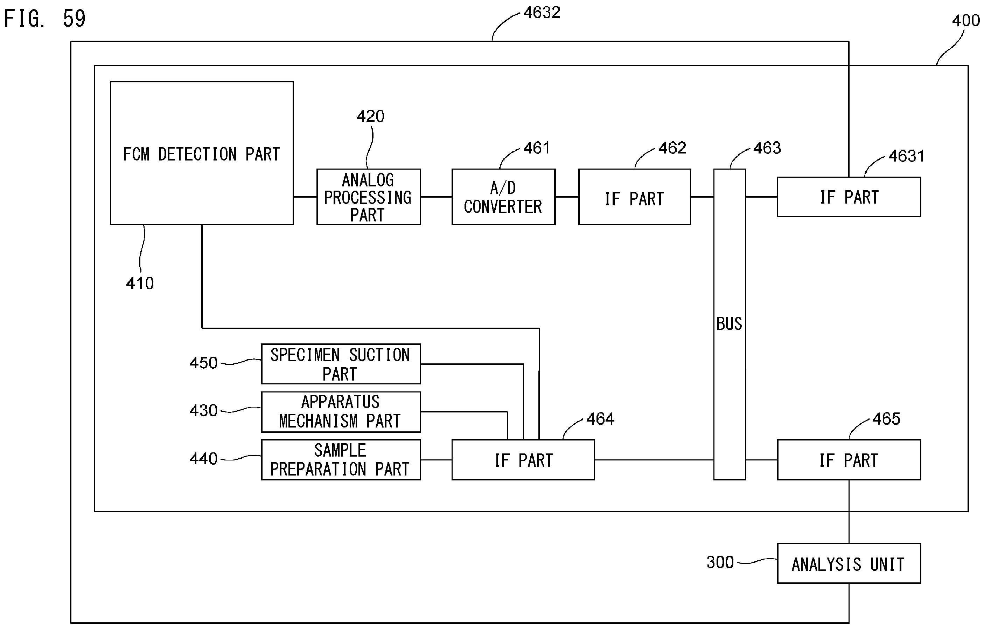

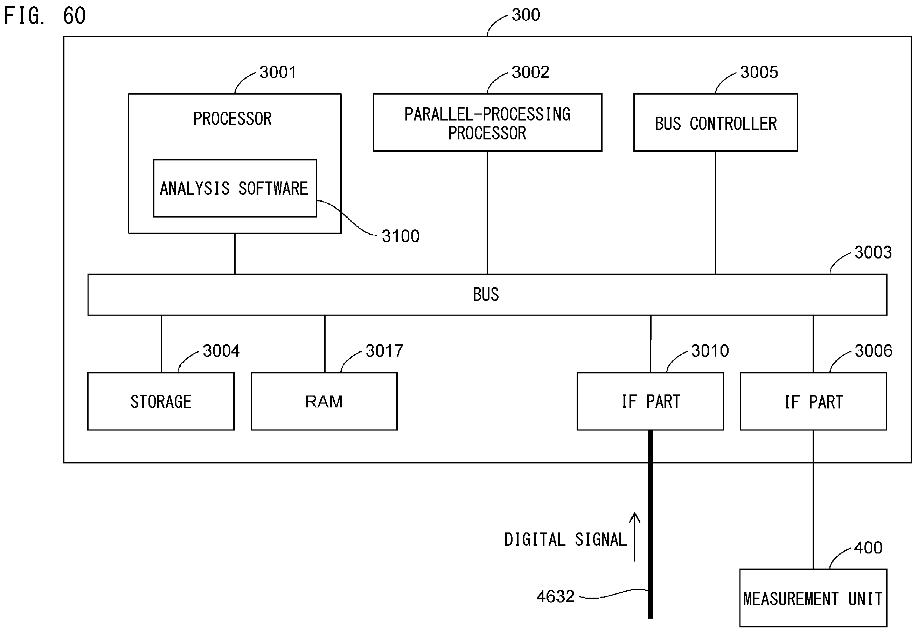

Next, with reference to FIG. 59 and FIG. 60, another configuration example of the specimen analyzer 4000 composed of the measurement unit 400 and the analysis unit 300 will be described.

FIG. 59 is a block diagram showing another configuration of the measurement unit 400.

The measurement unit 400 shown in FIG. 59 includes an IF part 4631 for transmitting waveform data generated by the A/D converter 461, to the analysis unit 300. A transmission line 4632 is connected to the IF part 4631. The other configurations and functions are similar to those of the measurement unit 400 described above.

The IF part 4631 is an interface serving as a dedicated line having a communication band of not less than 1 gigabit/second, for example. For example, the IF part 4631 is an interface according to Gigabit Ethernet, USB 3.0, or Thunderbolt 3. When the IF part 4631 is of Gigabit Ethernet, the transmission line 4632 is a LAN cable. When the IF part 4631 is of USB 3.0, the transmission line 4632 is a USB cable according to USB 3.0. The transmission line 4632 is a dedicated transmission line for transmitting digital data between the measurement unit 400 and the analysis unit 300, for example.

FIG. 60 is a block diagram showing another configuration of the analysis unit 300.

The analysis unit 300 shown in FIG. 60 includes an IF part 3010. The other configurations and functions are similar to those of the analysis unit 300 described above. The analysis unit 300 may be connected to a plurality of the measurement units 400 via a plurality of the IF parts 3010 and a plurality of the IF parts 3006.

The analysis software 3100, which operates on the processor 3001, has functions similar to those of the analysis software 3100 described above. The analysis software 3100 analyzes the type of each analyte in the specimen through operations similar to those in the related description above.

In the configuration shown in FIG. 59 and FIG. 60, the A/D converter 461 of the measurement unit 400 generates digital waveform data on the basis of each analog optical signal generated in the FCM detection part 410. The waveform data is sent to the analysis unit 300 via the IF part 462, the bus 463, the IF part 4631, and the transmission line 4632.

The measurement unit 400 and the analysis unit 300 are connected to each other in a one-to-one relationship via the transmission line 4632, for example. The transmission line 4632 in this case is a transmission line that provides no transmission of data related to an apparatus other than components (e.g., the measurement unit 400 and the analysis unit 300) forming the specimen analyzer 4000. The transmission line 4632 is a transmission line different from an intranet or the Internet, for example. Accordingly, even when waveform data generated in the measurement unit 400 is transmitted to the analysis unit 300, bottleneck in the communication speed of transmission of digital data can be avoided.

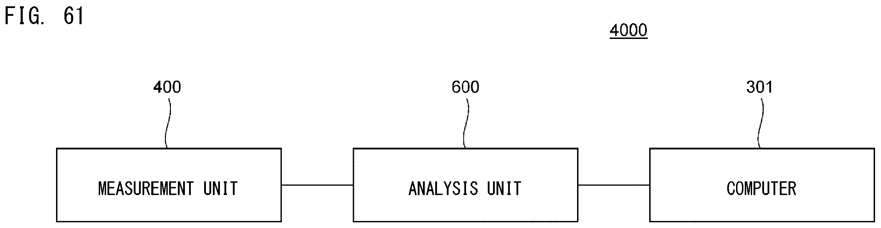

Next, with reference to FIG. 61 to FIG. 65, another configuration example of the specimen analyzer 4000 will be described.

FIG. 61 is a block diagram showing another configuration of the specimen analyzer 4000.

In the present configuration example, an analysis unit 600 is provided between the measurement unit 400 and the computer 301. That is, in the configuration in FIG. 61 to FIG. 65, the specimen analyzer 4000 includes the measurement unit 400, the computer 301, and the analysis unit 600. The analysis unit 600 analyzes the type of each measured cell. As described later, a parallel-processing processor 6002 of the present configuration example is installed to the specimen analyzer 4000 in the form of being incorporated in the analysis unit 600.

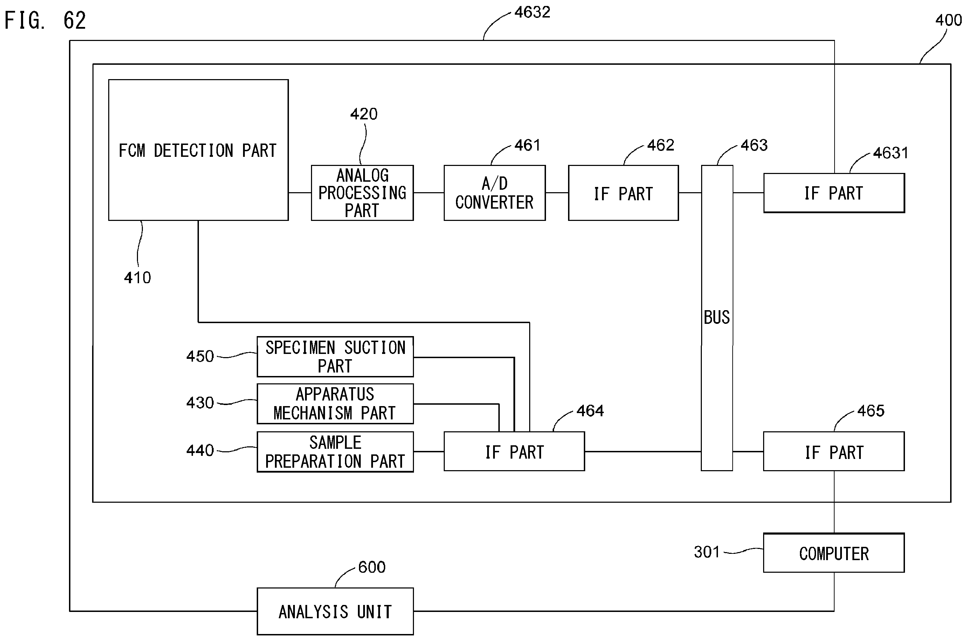

FIG. 62 is a block diagram showing another configuration of the measurement unit 400.

In the measurement unit 400 in FIG. 62, when compared with the configuration in FIG. 59, the computer 301 is connected to the IF part 465, and the analysis unit 600 is provided between the IF part 4631 and the computer 301. The analysis unit 600 is communicably connected to the IF part 4631 and the computer 301. The analysis unit 600 may be connected to a plurality of the measurement units 400. The analysis unit 600 may be connected to a plurality of the computers 301.

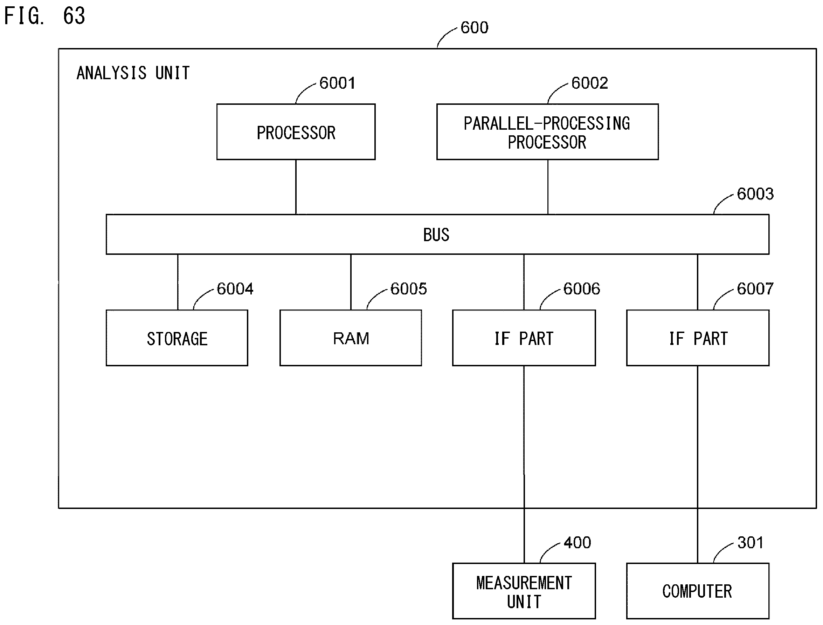

FIG. 63 is a block diagram showing a configuration of the analysis unit 600.

The analysis unit 600 includes a processor 6001, the parallel-processing processor 6002, a bus 6003, a storage 6004, a RAM 6005, and IF parts 6006, 6007. Each component of the analysis unit 600 is connected to the bus 6003.

The bus 6003 is a transmission line having a data transfer rate of not less than several hundred MB/s, for example. The bus 6003 may be a transmission line having a data transfer rate of not less than 1 GB/s. The bus 6003 performs data transfer on the basis of the PCI-Express or PCI-X standard, for example. The analysis unit 600 may be connected to a plurality of the measurement units 400 via a plurality of the IF parts 6006. When a plurality of the measurement units 400 are provided, an analysis unit 600 may be connected to each of the measurement units 400. In this case, for example, a plurality of the measurement units 400 and a plurality of the analysis units 600 are connected in a one-to-one relationship, respectively.

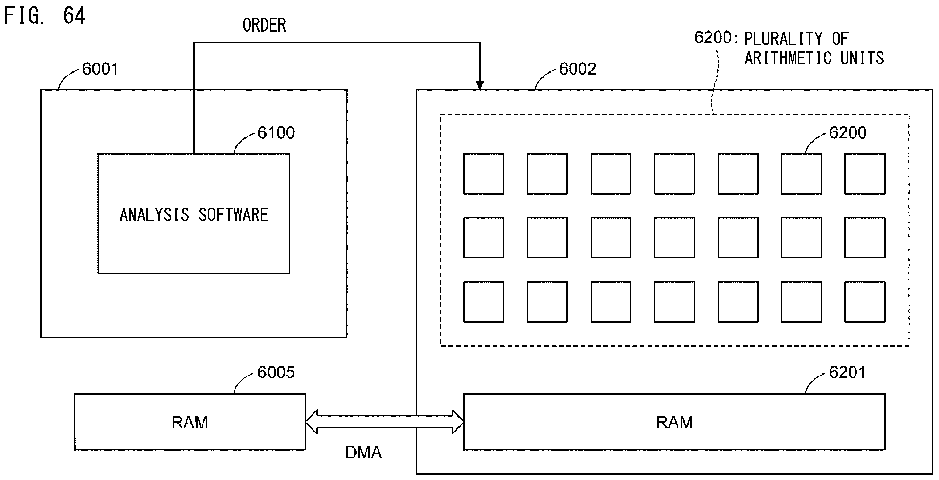

FIG. 64 shows a configuration example of the parallel-processing processor 6002 which executes arithmetic processes.

The processor 6001 and the parallel-processing processor 6002 have configurations and functions similar to those of the processor 3001 and the parallel-processing processor 3002 described above. The parallel-processing processor 6002 includes a plurality of arithmetic units 6200 and a RAM 6201. Analysis software 6100, which analyzes the type of each analyte in the specimen, operates on the processor 6001. The analysis software 6100 operating on the processor 6001 has functions similar to those of the analysis software 3100 shown in FIG. 50. The analysis software 6100 analyzes the type of the analyte in the specimen through operations similar to those described in FIG. 50. The analysis software 6100 transmits classification information of the analyte in the specimen to the computer 301 via the IF part 6007.

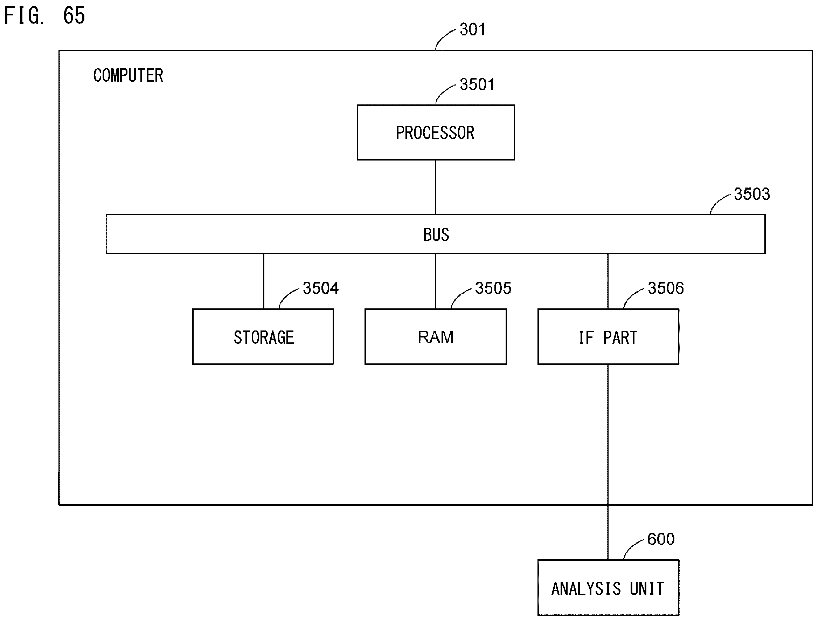

FIG. 65 is a block diagram showing a configuration of the computer 301.

The computer 301 in FIG. 65 has a configuration similar to that in which the parallel-processing processor 6002 is omitted from the analysis unit 600 in FIG. 63. The computer 301 includes a processor 3501, a bus 3503, a storage 3504, a RAM 3505, and an IF part 3506.

The analysis software 3100 need not necessarily operate on the processor 3501. The computer 301 receives, via the IF part 3506, analysis results obtained by the analysis unit 600. The IF part 3506 is of Ethernet or USB, for example. The IF part 3506 may be an interface capable of performing wireless communication.

In the configuration of FIG. 62 to FIG. 65, each analog optical signal of a cell generated in the FCM detection part 410 is converted to digital waveform data by the A/D converter 461 in the measurement unit 400. The waveform data is sent to the analysis unit 600 via the IF part 462, the bus 463, the IF part 4631, and the transmission line 4632.

As described above, the IF part 4631 is a dedicated interface that connects the measurement unit 400 and the analysis unit 600, and the IF part 4631 connects the measurement unit 400 and the analysis unit 600 in a one-to-one relationship. In other words, the transmission line 4632 is a transmission line that provides no transmission of data related to an apparatus other than components (e.g., the measurement unit 400 and the analysis unit 300) forming the specimen analyzer 4000, for example. The transmission line 4632 is a transmission line different from an intranet or the Internet. Accordingly, even when waveform data generated in the measurement unit 400 is transmitted to the analysis unit 600, bottleneck in the communication speed of transmission of the waveform data can be avoided.

In this case, steps S200 to S202 in FIG. 54 are executed by the analysis unit 600, and step S203 is executed by the computer 301.

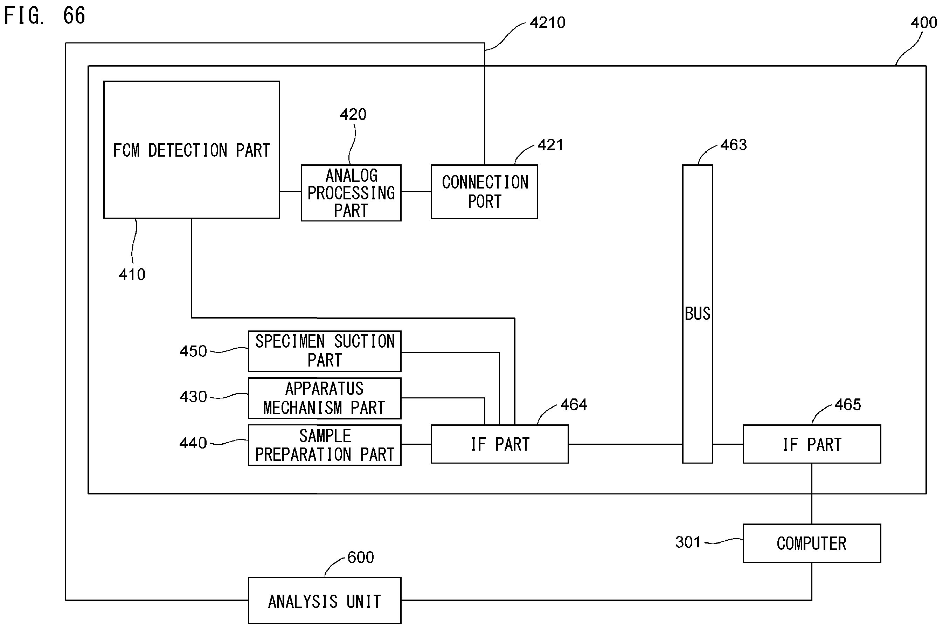

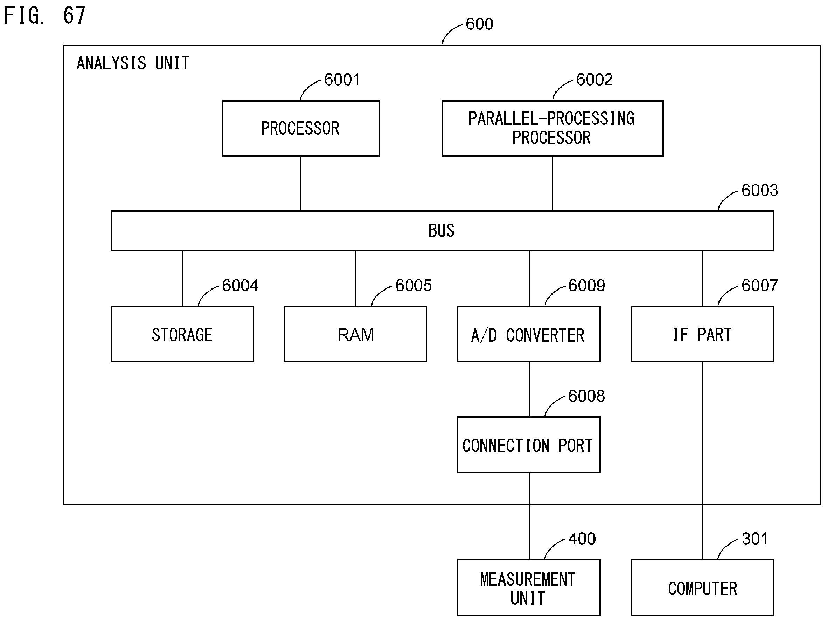

Next, with reference to FIG. 66 and FIG. 67, another configuration example of the specimen analyzer 4000 in FIG. 61 will be described. The specimen analyzer 4000 of this example includes the measurement unit 400, the computer 301, and the analysis unit 600.

In the measurement unit 400 in FIG. 66, when compared with the configuration in FIG. 57, the computer 301 is connected to the IF part 465 and the analysis unit 600 is provided between the connection port 421 and the computer 301. The analysis unit 600 is communicably connected to the connection port 421 and the computer 301. The measurement unit 400 transmits each analog optical signal to the analysis unit 600 via the connection cable 4210.

When compared with the configuration in FIG. 63, the analysis unit 600 in FIG. 67 includes a connection port 6008 and an A/D converter 6009 in place of the IF part 6006.

The analog optical signal transmitted from the measurement unit 400 via the connection cable 4210 is inputted to the A/D converter 6009 via the connection port 6008. The A/D converter 6009 generates waveform data from the optical signal through a process similar to that of the A/D converter 461.

The analysis unit 600 may be connected to a plurality of the measurement units 400 via a plurality of the connection ports 6008. When a plurality of the measurement units 400 are provided, an analysis unit 600 may be connected to each of the measurement units 400. In this case, for example, a plurality of the measurement units 400 and a plurality of the analysis units 600 are connected in a one-to-one relationship, respectively.

In the configuration in FIG. 66 and FIG. 67, in step S303 in FIG. 54, the analysis unit 600 generates waveform data on the basis of each analog optical signal transmitted from the measurement unit 400. Steps S200 to S202 in FIG. 54 are executed by the analysis unit 600, and step S203 is executed by the computer 301.

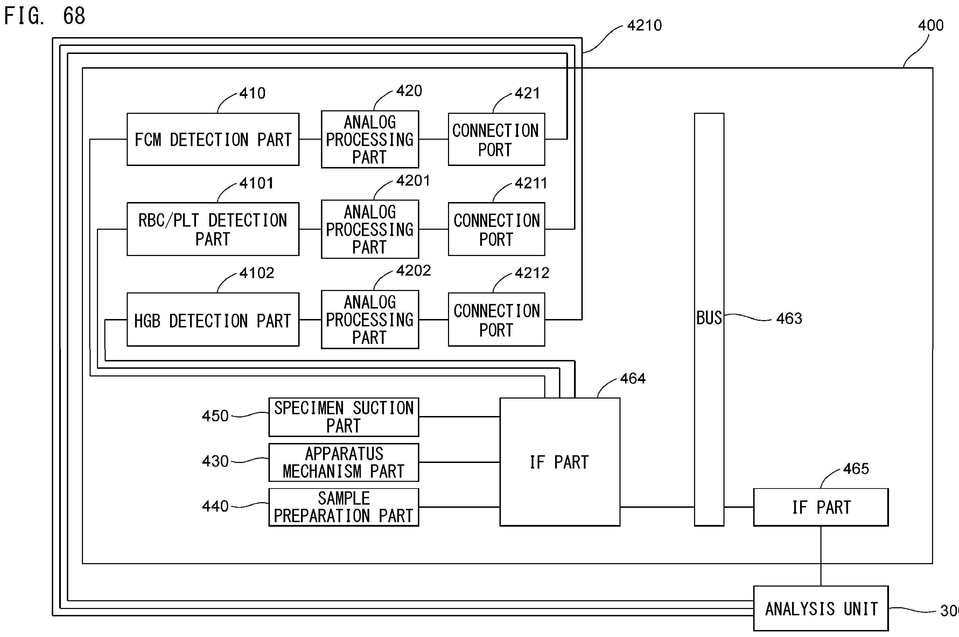

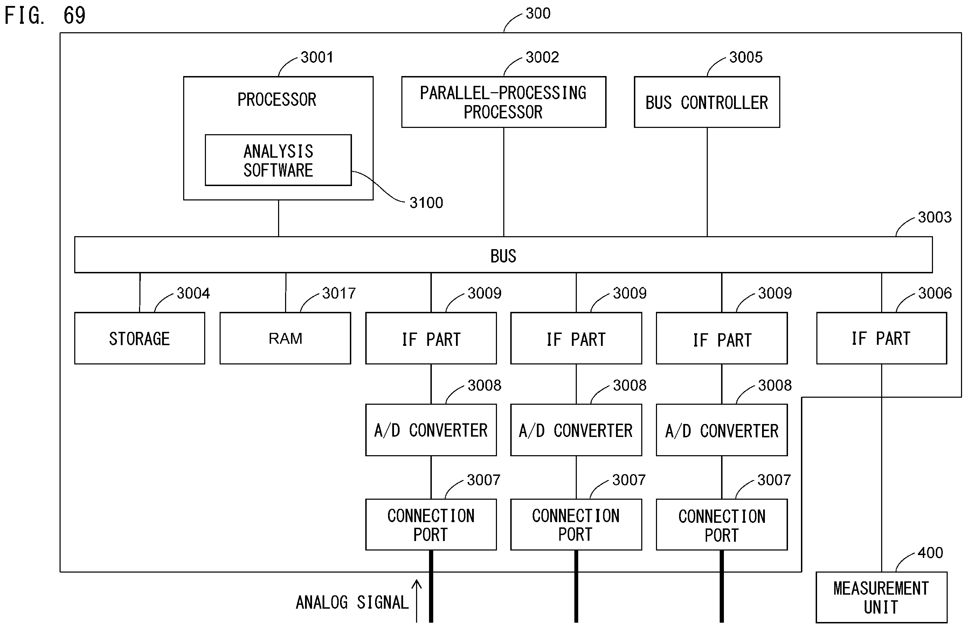

Next, with reference to FIG. 68 and FIG. 69, another configuration example of the measurement unit 400 and the analysis unit 300 of the specimen analyzer 4000 will be described.

When compared with the configuration in FIG. 27, the measurement unit 400 in FIG. 68 includes the connection ports 421, 4211, 4212 in place of the A/D converter 461, 4611, 4612 and the IF part 462. The analog optical signals obtained in the detection parts are transmitted to the analysis unit 300 via the connection cables 4210, respectively.

When compared with the configuration in FIG. 58, the analysis unit 300 in FIG. 69 includes three sets each composed of the connection port 3007, the A/D converter 3008, and the IF part 3009. The three connection ports 3007 are connected to the connection ports 421, 4211, 4212 in FIG. 68, respectively.

In the configuration in FIG. 68 and FIG. 69, in step S303 in FIG. 54, the analysis unit 300 generates waveform data on the basis of each analog optical signal transmitted from the measurement unit 400.

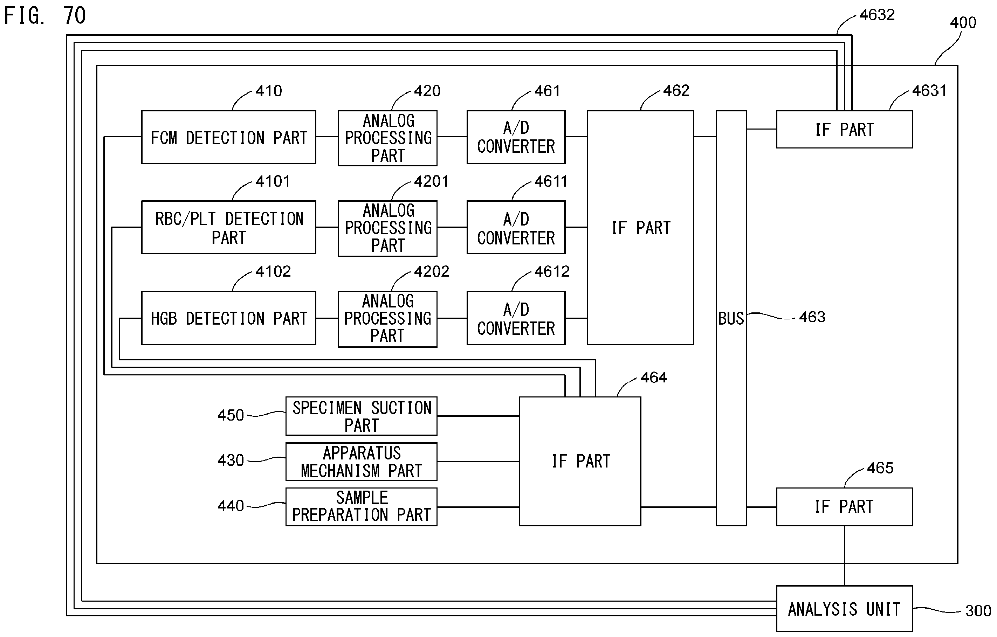

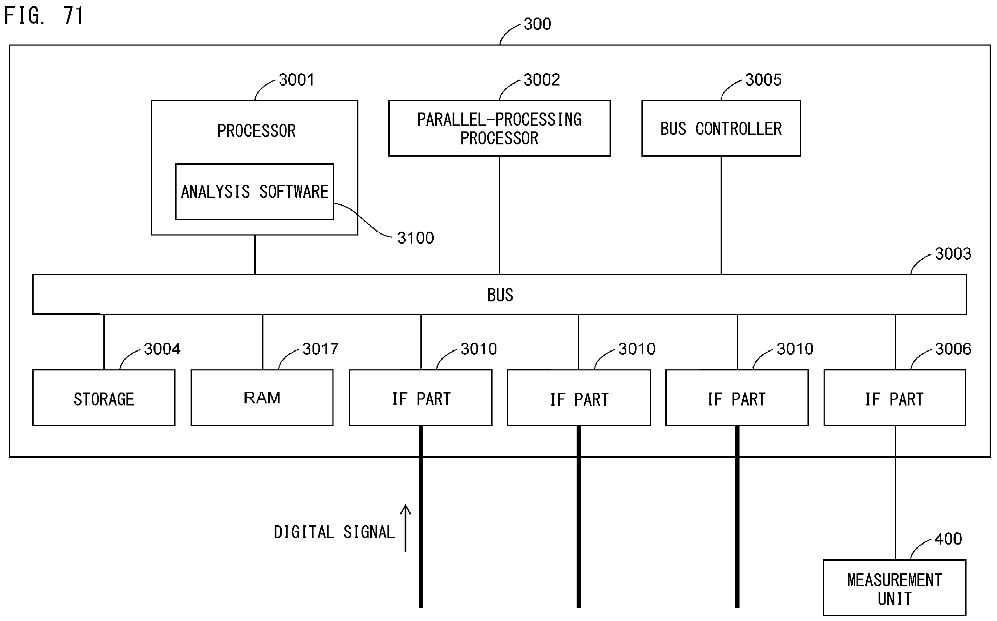

Next, with reference to FIG. 70 and FIG. 71, another configuration example of the measurement unit 400 and the analysis unit 300 of the specimen analyzer 4000 will be described.

When compared with the configuration in FIG. 27, the measurement unit 400 in FIG. 70 includes the IF part 4631. The A/D converters 461, 4611, 4612 generate waveform data on the basis of the analog optical signals obtained in the corresponding detection parts, respectively. Pieces of the waveform data corresponding to the respective detection parts are transmitted to the analysis unit 300 via the transmission lines 4632, respectively.

When compared with the configuration in FIG. 60, the analysis unit 300 in FIG. 71 includes three IF parts 3010. The three IF parts 3010 are connected to the transmission lines 4632 in FIG. 70, respectively.

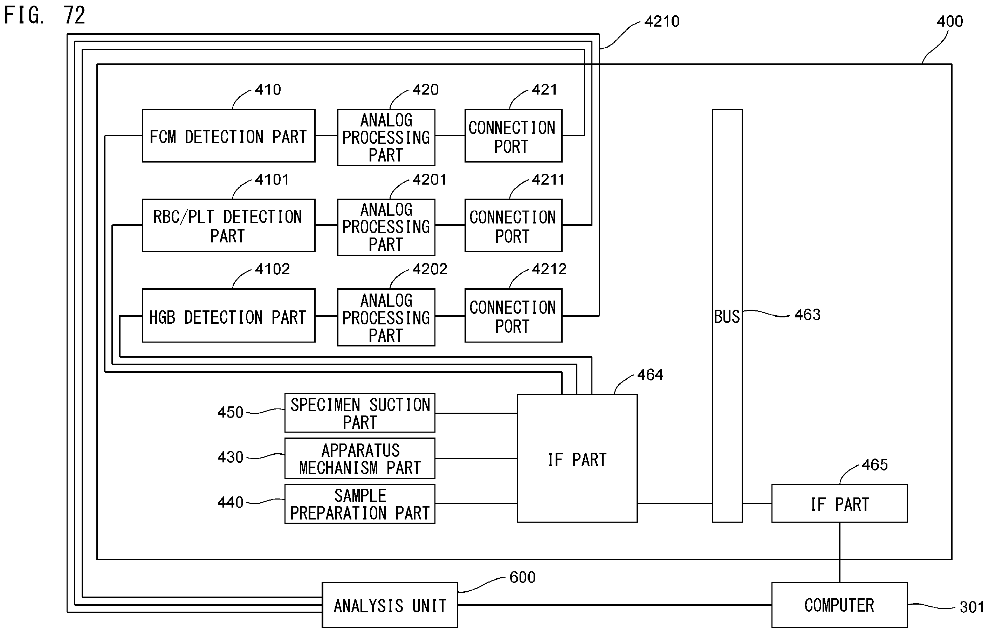

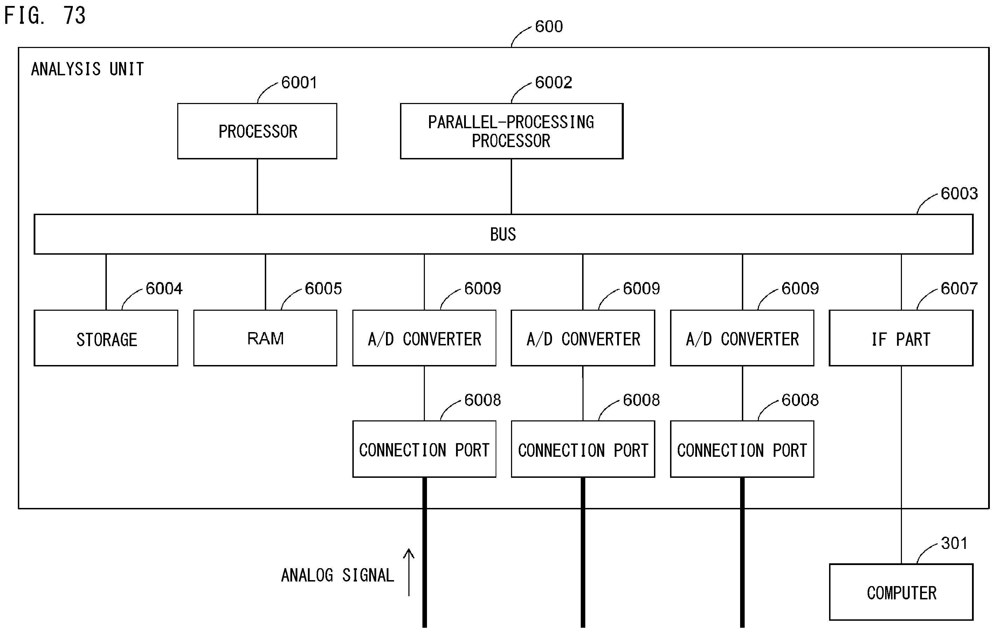

Next, with reference to FIG. 72 and FIG. 73, another configuration example of the measurement unit 400 and the analysis unit 300 of the specimen analyzer 4000 will be described.

In the measurement unit 400 in FIG. 72, when compared with the configuration in FIG. 68, the computer 301 is connected to the IF part 465, and the analysis unit 600 is disposed between the connection ports 421, 4211, 4212 and the computer 301. The analysis unit 600 is communicably connected to the connection ports 421, 4211, 4212 and the computer 301. The analysis unit 600 and the computer 301 are connected so as to be able to transmit/receive digital data to/from each other.

When compared with the configuration in FIG. 67, the analysis unit 300 in FIG. 73 includes three sets of the connection port 6008 and the A/D converter 6009. The three connection ports 6008 are connected to the connection ports 421, 4211, 4212 in FIG. 72, respectively.

Next, the data sizes of waveform data and digital data will be described.

In the present embodiment, for example, with respect to one analyte in the specimen, sampling is performed for each of an analog optical signal (FSC) based on forward scattered light, an analog optical signal (SSC) based on side scattered light, and an analog optical signal (FL) based on fluorescence.

Examples of the sampling rate include sampling at 1024 points at a 10 nanosecond interval, sampling at 128 points at an 80 nanosecond interval, sampling at 64 points at a 160 nanosecond interval, and the like. The data amount is 2 bytes per sampling, for example. With respect to each of FSC, SSC, and FL, data (in the case of the rate of 1024 points, 2 bytes×1024=2048 bytes) of an amount corresponding to the sampling rate is obtained. This data amount is the data amount per analyte in the specimen.

In a single measurement, FSC, SSC, and FL are measured with respect to at least 100 analytes, for example. In a single measurement, FSC, SSC, and FL may be measured with respect to at least 1000 analytes, for example. In a single measurement, FSC, SSC, and FL may be measured with respect to about 10000 to about 140000 analytes, for example. Therefore, when the number of analytes measured in a single measurement is 100000 and the sampling rate is 1024, the data amount of digital data of each of FSC, SSC, and FL is 2 bytes×1024×100000=204,800,000 bytes, and the data amount in total of FSC, SSC, and FL is 614,400,000 bytes.

Further, FSC, SSC, and FL are measured for each measurement channel. When the number of analytes measured in a single measurement is 100000, the sampling rate is 1024, and the number of measurement channels is 5, the data amount of each of FSC, SSC, and FL is 2 bytes×1024×100000×5=1,024,000,000 bytes, and the data amount in total of FSC, SSC, and FL is 3,072,000,000 bytes.

Thus, the volume of digital data is several hundred megabytes to several gigabytes per specimen, for example, and is at least 1 gigabyte depending on the number of analytes, the sampling rate, and the number of measurement channels.

According to the present embodiment, when digital data having a huge volume of several hundred megabytes to several gigabytes per specimen is analyzed, the analysis process using the AI algorithm 60 is concluded inside the specimen analyzer 4000 as described above, and the digital data is not transmitted, via the Internet or an intranet, to an analysis server provided outside the specimen analyzer 4000. Therefore, decrease in the throughput associated with increase in communication load caused by transmission of the digital data from the specimen analyzer 4000 to the analysis server can be avoided.

[Embodiment 7]

<Configuration of waveform data analysis system>

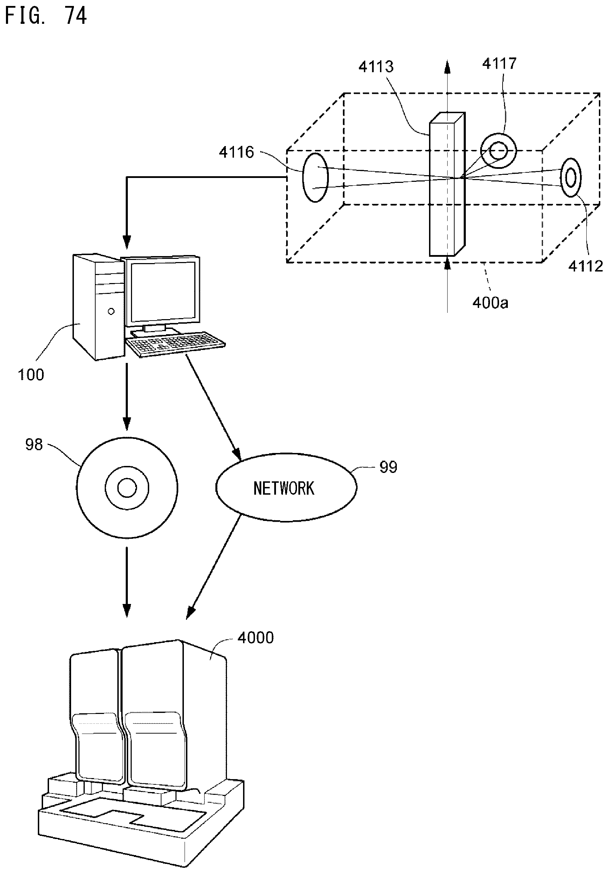

FIG. 74 schematically shows a configuration of a waveform data analysis system according to the present embodiment.

The configuration of a measurement unit 400a is similar to that of the measurement unit 400 described above. In the measurement unit 400a, a measurement sample prepared on the basis of a specimen is sent to the flow cell 4113. The light source 4111 (see FIG. 25) applies light to the measurement sample supplied to the flow cell 4113, and the light receiving elements 4116, 4121, 4122 (see FIG. 25) detect forward scattered light, side scattered light, and fluorescence generated from each analyte in the measurement sample. The measurement unit 400a generates waveform data from optical signals based on forward scattered light, side scattered light, and fluorescence outputted from the light receiving elements 4116, 4121, 4122, and transmits the generated waveform data to a deep learning apparatus 100.

The deep learning apparatus 100 is a vendor-side apparatus. The deep learning apparatus 100 receives training waveform data obtained by the measurement unit 400a. The generation method of the training waveform data has been described above. The AI algorithm 50 stored in the deep learning apparatus 100 is a deep learning algorithm. The deep learning apparatus 100 causes the AI algorithm 50 configured as a neural network before being trained, to learn by using training data, and provides the user with the AI algorithm 60 having been trained by the training data. The AI algorithm 60 configured as a learned neural network is provided to the specimen analyzer 4000 from the deep learning apparatus 100 through a storage medium 98 or a communication network 99. The storage medium 98 is a computer-readable non-transitory tangible storage medium such as a DVD-ROM or a USB memory, for example.

The deep learning apparatus 100 is implemented as a general-purpose computer, for example, and performs a deep learning process on the basis of a flowchart described later.

The specimen analyzer 4000 executes the AI analysis on waveform data that corresponds to each analyte, by using the AI algorithm 60 configured as a learned neural network.

<Hardware configuration of deep learning apparatus>

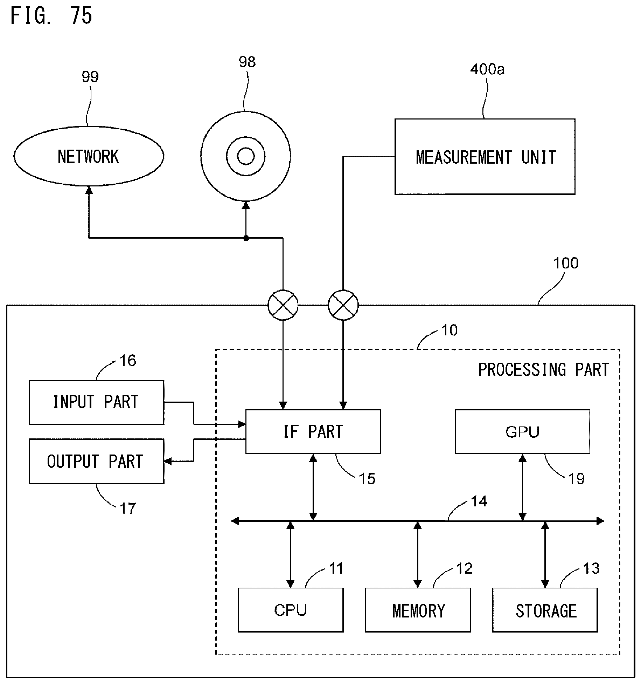

FIG. 75 is a block diagram showing a configuration of the deep learning apparatus 100.

The deep learning apparatus 100 includes a processing part 10, an input part 16, and an output part 17.

The input part 16 and the output part 17 are connected to the processing part 10 via an IF part 15. The input part 16 is an input device such as a keyboard or a mouse, for example. The output part 17 is a display device such as a liquid crystal display, for example.

The processing part 10 includes a CPU 11, a memory 12, a storage 13, a bus 14, the IF part 15, and a GPU 19.

The CPU 11 performs data processing described later. The memory 12 is used as a work area for the data processing. The storage 13 stores a program and processing data described later. The bus 14 transmits data between components. The IF part 15 performs input/output of data to/from an external apparatus. The GPU 19 functions as an accelerator that assists arithmetic processes (e.g., parallel arithmetic processes) performed by the CPU 11. That is, in the description below, the processes performed by the CPU 11 also include processes performed by the CPU 11 using the GPU 19 as an accelerator. The GPU 19 has a function equivalent to that of the parallel-processing processor 3002, 6002 described above. Instead of the GPU 19, a chip suitable for calculation in a neural network may be used. Examples of such a chip include FPGA, ASIC, and Myriad X (Intel).

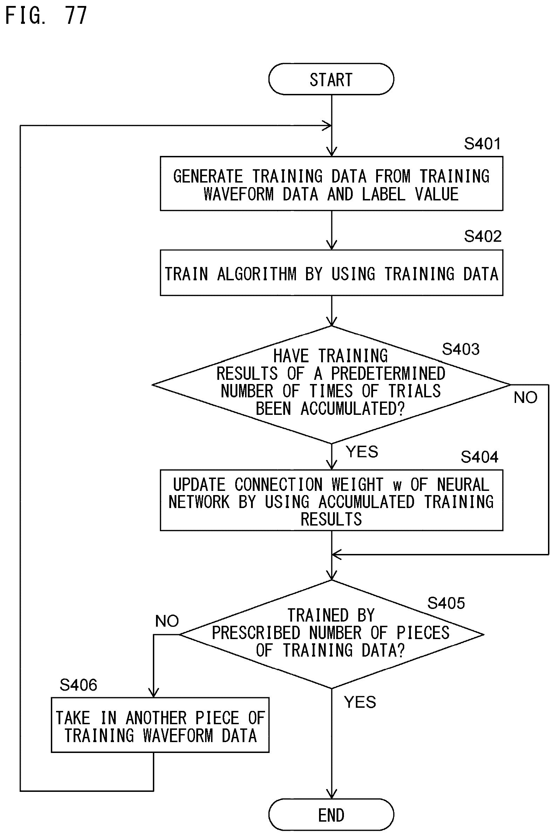

In order to perform the process of each step described later with reference to FIG. 77, the processing part 10 has previously stored, in the storage 13, a program and the AI algorithm 50 configured as a neural network before being trained according to the present embodiment, in an executable form, for example. The executable form is a form generated through conversion of a programing language by a compiler, for example. The processing part 10 uses the program stored in the storage 13, to perform a training process of the AI algorithm 50 before being trained.

In the description below, unless otherwise specified, the processes performed by the processing part 10 mean processes performed by the CPU 11 on the basis of the program and the AI algorithm 50 stored in the storage 13 or the memory 12. The CPU 11 temporarily stores necessary data (intermediate data being processed, etc.) using the memory 12 as a work area, and stores, as appropriate in the storage 13, data to be saved for a long time such as arithmetic results.

<Hardware configuration of analyzer>

The configuration of the specimen analyzer 4000 (see FIG. 74) is similar to that described above, and the specimen analyzer 4000 processes waveform data on the basis of an algorithm provided from the deep learning apparatus 100. The specimen analyzer 4000 may also have the function of the deep learning apparatus 100, to cause the AI algorithm 50 to learn by using training data. In this case, the deep learning apparatus 100 is not necessary.

In order to perform the process of each step described in the waveform data analysis process below, the specimen analyzer 4000 has previously stored, in the storage 3004 (see FIG. 26, for example) or the storage 6004 (see FIG. 63, for example), a program and the AI algorithm 60 configured as a trained neural network according to the present embodiment, in an executable form, for example. The specimen analyzer 4000 performs processes by using the program and the AI algorithm 60 stored in the storage 3004.

The AI algorithm 60 stored in the storage 3004, 6004 may be updated via a communication network. The deep learning apparatus 100 transmits the AI algorithm 60 to the specimen analyzer 4000 via a communication network (e.g., Internet, intranet). The specimen analyzer 4000 updates, by the received AI algorithm 60, the AI algorithm 60 already stored in the storage 3004, 6004.

<Function block and processing procedure>

(Deep learning process)

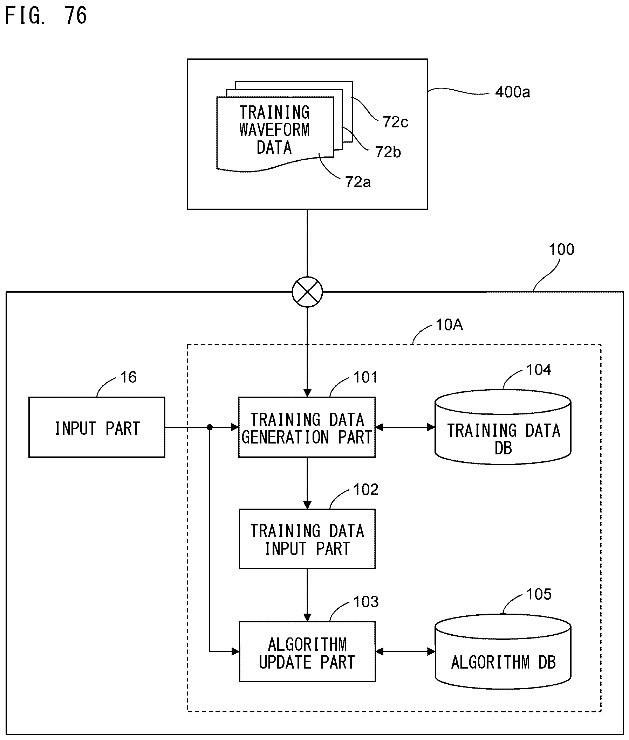

FIG. 76 is a function block diagram of the deep learning apparatus 100.

The processing part 10A of the deep learning apparatus 100 includes a training data generation part 101, a training data input part 102, and an algorithm update part 103. A program that causes a computer to execute a deep learning process is installed in the storage 13 or the memory 12 of the processing part 10 shown in FIG. 75, and this program is executed by the CPU 11 and the GPU 19, whereby each function block of the processing part 10A is realized.

A training data database (DB) 104 and an algorithm database (DB) 105 are stored in the storage 13 or the memory 12 of the processing part 10 shown in FIG. 75. Training waveform data 72a, 72b, 72c are obtained in advance by the measurement unit 400a, for example, and is stored in advance in the training data database 104. The AI algorithm 50 is stored in the algorithm database 105.

FIG. 77 is a flowchart showing a process performed by the deep learning apparatus 100.

The processes of steps S401, S404, and S406 in FIG. 77 are executed by the training data generation part 101. The process of step S402 is executed by the training data input part 102. The processes of steps S403 and S405 are executed by the algorithm update part 103.

First, the processing part 10A obtains the training waveform data 72a, 72b, 72c. The pieces of the training waveform data 72a, 72b, 72c are pieces of waveform data based on forward scattered light, side scattered light, and fluorescence, respectively. The training waveform data 72a, 72b, 72c may be obtained, for example, from the measurement unit 400a, from the storage medium 98, or via the communication network 99, through operation by the operator. When the training waveform data 72a, 72b, 72c is obtained, information regarding which cell type the training waveform data 72a, 72b, 72c indicates is also obtained. The information regarding the cell type may be associated with the training waveform data 72a, 72b, 72c, or may be inputted by the operator through the input part 16.

In step S401, the processing part 10A generates the training data 75 from the training waveform data 72a, 72b, 72c and the label value 77 as shown in FIG. 33. In step S402, the processing part 10A inputs the training data 75 to the AI algorithm 50, and obtains a trial result. The trial result is accumulated every time each of a plurality of the training data 75 is inputted to the AI algorithm 50.

In the cell type analysis method according to the present embodiment, a convolutional neural network is used, and a stochastic gradient descent method is used. Therefore, in step S403, the processing part 10A determines whether or not training results of a previously set predetermined number of times of trials have been accumulated. When the predetermined number of training results have been accumulated (S403: YES), the processing part 10A advances the process to step S404. On the other hand, when the predetermined number of training results have not been accumulated (S403: NO), the processing part 10A skips the process of step S404.

When the predetermined number of training results have been accumulated (S403: YES), the processing part 10A updates, in step S404, the connection weight w of the neural network forming the AI algorithm 50, by using the training results accumulated in step S402. In the cell type analysis method according to the present embodiment, since the stochastic gradient descent method is used, the connection weight w of the neural network is updated at the stage where the predetermined number of times of training results have been accumulated. Specifically, the process of updating the connection weight w is a process of performing calculation by the gradient descent method, represented by Formula 12 and Formula 13 described later.

In step S405, the processing part 10A determines whether or not the AI algorithm 50 has been trained by a prescribed number of pieces of training data 75. When the AI algorithm 50 has been trained by the prescribed number of pieces of training data 75 (S405: YES), the deep learning process ends. On the other hand, when the AI algorithm 50 has not been trained by the prescribed number of pieces of training data 75 (S405: NO), the processing part 10A takes in another piece of training waveform data 72a, 72b, 72c in step S406, and returns the process to step S401.

Through the processes as above, the processing part 10A trains the AI algorithm 50 and obtains the AI algorithm 60.

(Structure of neural network)

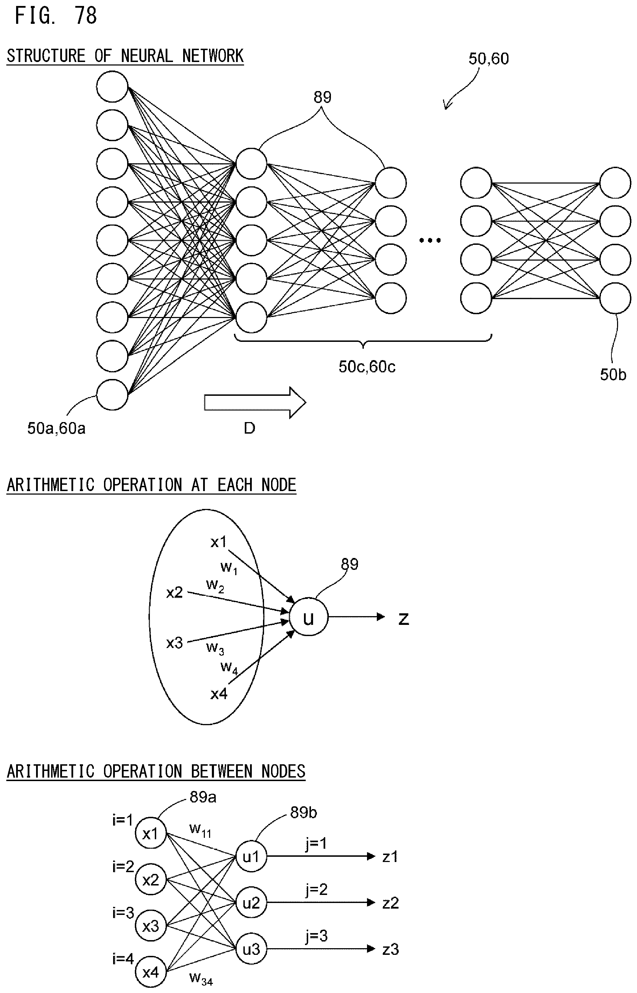

The upper part of FIG. 78 is a schematic diagram showing, as an example, a structure of a neural network forming the AI algorithm 50. As described above, a convolutional neural network is used in the present embodiment. The neural network of the AI algorithm 50 includes the input layer 50a, the output layer 50b, the middle layer 50c between the input layer 50a and the output layer 50b, and the middle layer 50c is composed of a plurality of layers. The number of layers forming the middle layer 50c is, for example, 5 or greater, preferably 50 or greater, and more preferably 100 or greater.

In the neural network of the AI algorithm 50, a plurality of nodes 89 arranged in a layered manner are connected between the layers. Accordingly, information is propagated only in one direction indicated by an arrow D in the drawing, from the input layer 50a to the output layer 50b.

(Arithmetic operation at each node)

The middle part of FIG. 78 is a schematic diagram showing arithmetic operations performed at each node 89.

Each node 89 receives a plurality of inputs, and calculates one output (z). In the case of the example shown in the middle part of FIG. 78, the node 89 receives four inputs. The total input (u) received by the node 89 is represented by Formula 2 below, for example. Here, in the present embodiment, one-dimensional matrix data is used as the training data 75 and the analysis data 85. Therefore, when variables of the arithmetic expression correspond to two-dimensional matrix data, a process of converting the variables so as to correspond to one-dimensional matrix data is performed.

[Math 2]

$$u = w_1 x_1 + w_2 x_2 + w_3 x_3 + w_4 x_4 + b \qquad \cdots \text{(Formula 2)}$$

Each input is multiplied by a different weight. In Formula 2, b is a value referred to as bias. The output (z) of the node serves as an output of a predetermined function f with respect to the total input (u) represented by Formula 2, and is represented by Formula 3 below. The function f is referred to as an activation function.

[Math 3]

$$z = f(u) \qquad \cdots \text{(Formula 3)}$$

The lower part of FIG. 78 is a schematic diagram showing arithmetic operations between nodes. In the neural network, nodes 89 that each output, with respect to the total input (u) of the corresponding node 89 represented by Formula 2, a result (z) represented by Formula 3 are arranged in a layered manner. The outputs of nodes 89 in the previous layer serve as inputs to nodes 89 in the next layer. In the example shown in the lower part of FIG. 78, the outputs of nodes 89a in the left layer serve as inputs to nodes 89b in the right layer. Each node 89b receives outputs from the nodes 89a. The connection between each node 89a and each node 89b is multiplied by a different weight. When the respective outputs from the plurality of nodes 89a are defined as x1 to x4, the inputs to the respective three nodes 89b are represented by Formula 4-1 to Formula 4-3 below.

[Math 4]

$$u_1 = w_{11} x_1 + w_{12} x_2 + w_{13} x_3 + w_{14} x_4 + b_1 \qquad \cdots \text{(Formula 4-1)}$$
$$u_2 = w_{21} x_1 + w_{22} x_2 + w_{23} x_3 + w_{24} x_4 + b_2 \qquad \cdots \text{(Formula 4-2)}$$
$$u_3 = w_{31} x_1 + w_{32} x_2 + w_{33} x_3 + w_{34} x_4 + b_3 \qquad \cdots \text{(Formula 4-3)}$$

When Formula 4-1 to Formula 4-3 are generalized, Formula 4-4 below is obtained. Here, i = 1,..., 1, and j=1,..., J. I is the total number of inputs, and J is the total number of outputs.

[Math 5]

$$u_j = \sum_{i=1}^{I} w_{ji} x_i + b_j \qquad \cdots \text{(Formula 4-4)}$$

When Formula 4-4 is applied to the activation function, an output represented by Formula 5 below is obtained.

[Math 6]

$$z_j = f(u_j) \quad (j = 1, 2, 3) \qquad \cdots \text{(Formula 5)}$$

(Activation function)

In the cell type analysis method according to the embodiment, a rectified linear unit function is used as the activation function. The rectified linear unit function is represented by Formula 6 below.

[Math 7]

$$f(u) = \max(u, 0) \qquad \cdots \text{(Formula 6)}$$

Formula 6 is a function obtained by setting u=0 to the part u<0 in the linear function with z=u. In the example shown in the lower part of FIG. 78, the output from the node of j=1 is represented by Formula 6 below.

[Math 8]

$$z_1 = \max\big((w_{11}x_1 + w_{12}x_2 + w_{13}x_3 + w_{14}x_4 + b_1), 0\big)$$

(Neural network learning)

If a function expressed by use of a neural network is defined as y(x:w), the function y(x:w) varies when a parameter w of the neural network is varied. Adjusting the function y(x:w) such that the neural network selects a more suitable parameter w with respect to the input x is referred to as neural network training/learning. It is assumed that a plurality of pairs of inputs and outputs of a function expressed by use of a neural network are given. When a desirable output for an input x is defined as d, the pairs of the input/output are given as {(x1,d1), (x2,d2),..., (xn,dn)}. The set of pairs expressed as (x,d) is referred to as training data. Specifically, as shown in FIG. 33, the set of the waveform data 72a, 72b, 72c is the training data 75.

The neural network learning means adjusting the weight w such that, with respect to any input/output pair (xn,dn), the output y(xn:w) of the neural network when given the input xn becomes close to the output dn as much as possible, as shown in the Formula below.

[Math 9]

$$y(x_n : w) \approx d_n$$

An error function is a measure for measuring closeness between the training data and the function expressed by use of the neural network. The error function is also referred to as a loss function. An error function E(w) used in the cell type analysis method according to the embodiment is represented by Formula 7 below. Formula 7 is referred to as cross entropy.

[Math 10]

$$E(w) = -\sum_{n=1}^{N} \sum_{k=1}^{K} d_{nk} \log y_k(x_n : w) \qquad \cdots \text{(Formula 7)}$$

A method for calculating the cross entropy of Formula 7 will be described. In the output layer 50b of the neural network used in the cell type analysis method according to the embodiment, i.e., in the last layer of the neural network, an activation function for classifying the input x into a finite number of classes in accordance with the contents, is used. The activation function is referred to as a softmax function, and is represented by Formula 8 below. It is assumed that, in the output layer 50b, nodes are arranged by the same number as the number of classes k. It is assumed that the total input u of each node k (k=1,..., K) of an output layer L is given as $u_k^{(L)}$ from the outputs of the previous layer L-1. Accordingly, the output of the k-th node in the output layer is represented by Formula 8 below.

[Math 11]

$$y_k \equiv z_k^{(L)} = \frac{\exp\left(u_k^{(L)}\right)}{\sum_{j=1}^{K} \exp\left(u_j^{(L)}\right)} \qquad \cdots \text{(Formula 8)}$$

Formula 8 is the softmax function. The sum of output y1,..., yK determined by Formula 8 is always 1.

When each class is expressed as C1,..., CK, output yK of node k in the output layer L (i.e., uk$^{(L)}$) represents the probability at which the given input x belongs to class CK. The input x is classified to a class at which the probability represented by Formula 9 below is highest.

[Math 12]

$$p(C_k|x) = y_k = z_k^{(L)} \qquad \cdots \text{(Formula 9)}$$

In the neural network learning, a function expressed by the neural network is considered as a model of the posterior probability of each class. The likelihood of the weight w with respect to the training data is evaluated under such a probability model, and a weight w that maximizes the likelihood is selected.

It is assumed that the target output by the softmax function of Formula 8 is 1 only when the output is a correct class, and otherwise, the target output is 0. When the target output is expressed in a vector form dn=[dn1,..., dnK], if, for example, the correct class of input xn is C3, only target output dn3 becomes 1, and the other target outputs become 0. When coding is performed in this manner, the posterior distribution is represented by Formula 10 below.

[Math 13]

$$p(d|x) = \prod_{k=1}^{K} p(C_k|x)^{d_k} \qquad \cdots \text{(Formula 10)}$$

Likelihood L(w) of the weight w with respect to the training data {(xn,dn)}(n=1,..., N) is represented by Formula 11 below. When the logarithm of the likelihood L(w) is taken and the sign is inverted, the error function of Formula 7 is derived.

[Math 14]

$$L(w) = \prod_{n=1}^{N} p(d_n|x_n : w)$$
$$= \prod_{n=1}^{N} \prod_{k=1}^{K} p(C_k|x_n)^{d_{nk}}$$
$$= \prod_{n=1}^{N} \prod_{k=1}^{K} \left(y_k(x : w)\right)^{d_{nk}} \qquad \cdots \text{(Formula 11)}$$

Learning means minimizing the error function E(w) calculated on the basis of the training data, with respect to the parameter w of the neural network. In the cell type analysis method according to the embodiment, the error function E(w) is represented by Formula 7.

Minimizing the error function E(w) with respect to the parameter w has the same meaning as finding a local minimum point of the error function E(w). The parameter w is a weight of connection between nodes. The local minimum point of the weight w is obtained by iterative calculation of repeatedly updating the parameter w from an arbitrary initial value used as a starting point. An example of such calculation is the gradient descent method.

In the gradient descent method, a vector represented by Formula 12 below is used.

[Math 15]

$$\nabla E = \frac{\partial E}{\partial w} = \left[\frac{\partial E}{\partial w_1}, \dots, \frac{\partial E}{\partial w_M}\right]^T \quad \cdots \text{(Formula 12)}$$

In the gradient descent method, a process of moving the value of the current parameter w in the negative gradient direction (i.e., -VE) is repeated many times. When the current weight is $w^{(t)}$ and the weight after the moving is $w^{(t+1)}$, the arithmetic operation according to the gradient descent method is represented by Formula 13 below. The value t means the number of times the parameter w is moved.
[Math 16]

$$w^{(t+1)} = w^{(t)} - \epsilon\nabla E \quad \cdots \text{(Formula 13)}$$

The symbol used in Formula 13 and shown in Formula 14 below is a constant that determines the magnitude of the update amount of the parameter w, and is referred to as a learning coefficient.
[Math 17]

$$\epsilon \quad \cdots \text{(Formula 14)}$$

As a result of repetition of the arithmetic operation represented by Formula 13, an error function $E(w^{(t)})$ decreases in association with increase of the value t, and the parameter w reaches a local minimum point.

It should be noted that the arithmetic operation according to Formula 13 may be performed on all of the training data (n=1,..., N), or may be performed on only a part of the training data. The gradient descent method performed on only a part of the training data is referred to as a stochastic gradient descent method. In the cell type analysis method according to the embodiment, the stochastic gradient descent method is used.

[Effects of embodiments]

The measurement unit 400 includes: a first reagent container storing a first reagent, a second reagent container storing a second reagent, a first reaction chamber 440a configured to prepare a first measurement sample on the basis of a first specimen and a first reagent; a second reaction chamber 440a configured to prepare a second measurement sample on the basis of the first reagent and a second specimen different from the first specimen; a third reaction chamber 440b, 440c, 440d configured to prepare a third measurement sample on the basis of the first specimen and a second reagent; and the FCM detection part 410 (optical detection part) for obtaining a first optical signal from the first measurement sample, for obtaining a second optical signal from the second measurement sample, and for obtaining a third optical signal from the third measurement sample. The analysis unit 300 analyzes first data that corresponds to the first optical signal, analyzes second data that corresponds to the second optical signal, and analyzes third data that corresponds to the third optical signal. The analysis unit 300: executes analysis of a first measurement item with respect to the first measurement sample, through the AI analysis (first analysis operation) of processing the first data according to the artificial intelligence algorithm; executes analysis of a second measurement item different from the first measurement item with respect to the first measurement sample, through the calculation processing analysis (second analysis operation) of processing a first representative value obtained from the first data, the first representative value corresponding to a feature of the analyte; and executes analysis with respect to the third measurement sample, through the calculation processing analysis (third analysis operation) of processing a second representative value obtained from the third data, the second representative value corresponding to a feature of the analyte. Herein, the second analysis operation and the third analysis operation do not use the AI algorithm, and the first reaction chamber 440a and the second reaction chamber 440a execute, in parallel, preparation of the first measurement sample and the second measurement sample.

When the measurement unit 400 is configured as shown in FIG. 29, the first reagent is used, for example, in order to classify white blood cells in a specimen, and more specifically, in order to classify cells in the specimen into neutrophils, lymphocytes, monocytes, and eosinophils. The first reagent is the WDF hemolytic agent and the WDF staining liquid, for example. The first measurement sample is the WDF measurement sample, for example. The first sample preparation part is the reaction chamber 440a of the WDF channel, for example. The second reagent is a hemolytic agent and a staining liquid of a measurement channel other than the WDF channel, for example. The second measurement sample is a measurement sample of a measurement channel other than the WDF channel, for example. The second sample preparation part is the reaction chamber 440b, 440c, 440d of a measurement channel other than the WDF channel, for example.

The first optical signal and the second optical signal are analog signals outputted from light receiving elements on the basis of forward scattered light, side scattered light, and fluorescence. The first optical signal and the second optical signal are each a signal that has a region corresponding to each of analytes (e.g., cell) in the specimen, and that reflects the presence of the analyte in the specimen. The first data and the second data are respectively pieces of digital data that correspond to the intensity of the first optical signal and the second optical signal based on light generated from each analyte (e.g., cell). The first data and the second data are pieces of waveform data generated so as to correspond to the regions of the first optical signal and the second optical signal, respectively. In other words, the first data and the second data respectively correspond to the first optical signal and the second optical signal obtained while the analyte passes through the application position of light by the light source 4111.

The first measurement item is an item that is analyzed on the basis of measurement in the WDF channel, for example. An example of the first measurement item is an item regarding white blood cells, for example. The second measurement item is an item that is analyzed on the basis of a measurement channel other than the WDF channel, for example. Examples of the second measurement item include an item regarding platelets and an item regarding reticulocytes, for example.

The first, second, and third analysis operations are each an operation that determines the type of an analyte. The first representative value that corresponds to a feature of an analyte is a value (see FIG. 3), such as the peak value, the area, or the width, obtained from the first data (waveform data) that corresponds to the analyte, for example. The second representative value that corresponds to a feature of the analyte is a value (see FIG. 3), such as the peak value, the area, or the width, obtained from the second data (waveform data) that corresponds to the analyte, for example.

According to this configuration, the analysis process performed on data that corresponds to the optical signal obtained from the first and second specimen is apportioned between the first analysis operation (AI analysis) according to the artificial intelligence algorithm, and the second and third analysis operations (calculation processing analysis) of processing the first and second representative values that each correspond to a feature of the analyte. Therefore, when compared with a case where the data corresponding to the optical signal is all analyzed by using only the artificial intelligence algorithm, the load on the analysis unit 300 being a computer that processes the data can be reduced.

Analysis of the second measurement item with respect to the first measurement sample is executed through at least the second analysis operation (calculation processing analysis) of processing the first representative value that corresponds to a feature of the analyte. Accordingly, for example, even when analysis of the second measurement item is difficult to be performed through the second analysis operation, a highly accurate analysis can be performed through the first analysis operation as appropriate.

Since the plurality of first sample preparation parts are provided, a plurality of first measurement samples can be prepared in parallel on the basis of a plurality of specimens. Accordingly, the throughput of specimen analysis can be improved.

The representative value of the waveform data (each of the first data and the second data) that can be a target of the calculation processing analysis is specified on the basis of the magnitude of the waveform data (the corresponding first data and second data).

Specifically, a representative value, such as the peak value, the area, or the width, of the waveform data is specified on the basis of the magnitude of the waveform data. Accordingly, the representative value can be smoothly specified.

The first optical signal and the second optical signal each have a region that corresponds to each of analytes in the specimen. The analysis unit 300 specifies a first representative value and a second representative value that can be targets of the calculation processing analysis, on the basis of waveform data (the first data and the second data) that corresponds to each of regions of the first optical signal and the second optical signal. Since the optical signal includes regions that correspond to the respective analytes, a representative value such as the peak value, the area, or the width that corresponds to each analyte can be smoothly specified on the basis of the waveform data that corresponds to each region of the first optical signal and the second optical signal.

The first optical signal has a region that corresponds to each of analytes in the specimen. The analysis unit 300 inputs waveform data (the first data) that corresponds to each of the regions of the first optical signal, into the artificial intelligence algorithm. Since the first optical signal includes regions that correspond to the respective analytes, the AI analysis can be smoothly executed by inputting waveform data that corresponds to each region of the first optical signal, into the artificial intelligence algorithm.

As described above, when the first optical signal and the second optical signal each have a region that corresponds to each of analytes in the specimen, the measurement unit 400 obtains waveform data (the first data and the second data) on the basis of a signal that is greater than a predetermined threshold and that corresponds to the intensity of each of the first optical signal and the second optical signal, as shown in the drawing in the upper part of FIG. 3. According to this configuration, waveform data that corresponds to each of the analytes can be appropriately obtained.

As shown in FIG. 37, in analysis of a WDF measurement sample (the first measurement sample) according to the artificial intelligence algorithm, the analysis unit 300 performs classification as to whether or not a cell in the specimen corresponds to at least either of a nucleated red blood cell and a basophil. Usually, classification of a nucleated red blood cell and a basophil needs to be performed in a WNR channel. Regarding this, according to the AI analysis (the first analysis operation) using the artificial intelligence algorithm, classification of a nucleated red blood cell and a basophil can be accurately performed on the basis of the WDF measurement sample (the first measurement sample) prepared so as to classify white blood cells.

As shown in FIG. 37, the analysis unit 300 classifies cells in a specimen into nucleated red blood cells and basophils through the AI analysis (the first analysis operation) on the basis of waveform data (the first data) obtained from a WDF measurement sample (the first measurement sample). Further, the analysis unit 300 classifies cells in the specimen into neutrophils, lymphocytes, monocytes, and eosinophils through the calculation processing analysis (the second analysis operation) on the basis of waveform data (the first data) obtained from the WDF measurement sample (the first measurement sample). While nucleated red blood cells and basophils of which highly accurate classification is difficult to be realized by the calculation processing analysis are classified through the AI analysis, neutrophils, lymphocytes, monocytes, and eosinophils of which highly accurate classification can be realized merely by the calculation processing analysis are classified through the calculation processing analysis. Therefore, when compared with a case where all classifications are executed through the AI analysis, the load on the analysis unit 300 can be reduced.

The plurality of reaction chambers 440a (the first sample preparation parts) of the WDF channel execute, in parallel, preparation of a plurality of WDF measurement samples (the first measurement samples) that respectively correspond to a plurality of different specimens. According to this configuration, the throughput of the specimen analysis can be improved.

As shown in FIG. 37, the analysis unit 300 executes, through the AI analysis (the first analysis operation), analysis of measurement items (the first measurement item) regarding nucleated red blood cells and basophils with respect to a WDF measurement sample (the first measurement sample). Further, the analysis unit 300 analyzes measurement items (the second measurement item) regarding lymphocytes, monocytes, eosinophils, and neutrophils by determining, through the calculation processing analysis (the second analysis operation), the type of an analyte that does not correspond to a measurement item (the first measurement item) regarding nucleated red blood cells and basophils as a result of the AI analysis (the first analysis operation). According to this configuration, even when five classifications of white blood cells are difficult to be performed only through the calculation processing analysis based on the WDF measurement sample, five classifications of white blood cells can be performed on the basis of the WDF measurement sample since both of the AI analysis and the calculation processing analysis are executed.

As shown in FIG. 38, the analysis unit 300 executes, through the calculation processing analysis (the second analysis operation), analysis of measurement items (the second measurement item) regarding lymphocytes, monocytes, and eosinophils with respect to a WDF measurement sample (the first measurement sample). Further, the analysis unit 300 analyzes measurement items (the first measurement item) regarding neutrophils and basophils by determining, through the AI analysis (the first analysis operation), the type of an analyte that does not correspond to the above second measurement item as a result of the calculation processing analysis (the second analysis operation). In this configuration as well, since both of the AI analysis and the calculation processing analysis are executed, five classifications of white blood cells can be performed on the basis of a WDF measurement sample.

Various modifications can be made as appropriate to the embodiments of the present disclosure, without departing from the scope of the technological idea defined by the claims.

## Claims

1. A specimen analyzer (4000) for analyzing an analyte in a specimen, the specimen analyzer comprising:

    a measurement unit (400) including

        a first reagent container storing a first reagent,
        a second reagent container storing a second reagent,
        a first reaction chamber (440a) configured to prepare a first measurement sample on the basis of a first specimen and the first reagent,
        a second reaction chamber (440a) configured to prepare a second measurement sample on the basis of the first reagent and a second specimen different from the first specimen,
        a third reaction chamber (440b, 440c, 440d) configured to prepare a third measurement sample on the basis of the first specimen and a second reagent, and
        an optical detection part (410, 470) configured to obtain a first optical signal (80a, 80b, 80c) from the first measurement sample, obtain a second optical signal (80a, 80b, 80c) from the second measurement sample, and obtain a third optical signal (80a, 80b, 80c) from the third measurement sample; and

an analysis unit (300) configured to analyze first data (82a, 82b, 82c) corresponding to the first optical signal (80a, 80b, 80c), analyze second data (82a, 82b, 82c) corresponding to the second optical signal (80a, 80b, 80c), and analyze third data (82a, 82b, 82c) corresponding to the third optical signal (80a, 80b, 80c), wherein the analysis unit (300) is configured to

execute analysis of a first measurement item with respect to the first measurement sample, through a first analysis operation of processing the first data (82a, 82b, 82c) according to an artificial intelligence, AI, algorithm (60),
execute analysis of a second measurement item different from the first measurement item with respect to the first measurement sample, through a second analysis operation of processing a first representative value obtained from the first data (82a, 82b, 82c), the first representative value corresponding to a feature of the analyte, and
execute analysis with respect to the third measurement sample, through a third analysis operation of processing a second representative value obtained from the third data (82a, 82b, 82c), the second representative value corresponding to a feature of the analyte,

the second analysis operation and the third analysis operation do not use the AI algorithm, and
the first reaction chamber (440a) and the second reaction chamber (440a) execute, in parallel, preparation of the first measurement sample and the second measurement sample.

2. The specimen analyzer (4000) of claim 1, wherein
in the second analysis operation, the analysis unit (300) is configured to specify the first representative value on the basis of the first data (82a, 82b, 82c) and processes the specified first representative value.

3. The specimen analyzer (4000) of claim 2, wherein
in the second analysis operation, the analysis unit (300) is configured to specify the first representative value on the basis of a magnitude of the first data (82a, 82b, 82c) .

4. The specimen analyzer (4000) of claim 2 or 3, wherein

the first optical signal (80a, 80b, 80c) has a region corresponding to each of analytes in the first specimen, and
the analysis unit (300) is configured to specify the first representative value on the basis of the first data (82a, 82b, 82c) corresponding to each of the regions of the first optical signal (80a, 80b, 80c).

5. The specimen analyzer (4000) of claim 4, wherein
the analysis unit (300) is configured to specify, as the first representative value, a peak value in the region of the first data (82a, 82b, 82c).

6. The specimen analyzer (4000) of any one of claims 1 to 5, wherein

the first optical signal (80a, 80b, 80c) has a region corresponding to each of analytes in the first specimen, and
in the first analysis operation, the analysis unit (300) is configured to input, to the AI algorithm, the first data (82a, 82b, 82c) corresponding to each of the regions of the first optical signal (80a, 80b, 80c).

7. The specimen analyzer (4000) of any one of claims 1 to 6, wherein
the measurement unit (400) is configured to obtain the first data (82a, 82b, 82c) on the basis of a signal that is greater than a predetermined threshold and corresponding to intensity of the first optical signal (80a, 80b, 80c).

8. The specimen analyzer (4000) of claim 1, wherein

the measurement unit (400) is configured to obtain the first representative value on the basis of the first optical signal (80a, 80b, 80c), and
in the second analysis operation, the analysis unit (300) is configured to process the first representative value obtained by the measurement unit (400).

9. The specimen analyzer (4000) of any one of claims 1 to 8, wherein
the first optical signal (80a, 80b, 80c) is a signal that reflects presence of an analyte in the first specimen.

**10.** The specimen analyzer (4000) of any one of claims 1 to 9, wherein
the optical detection part (410, 470)

> includes a light source, a flow cell, and a photodetector, and is configured to
> apply light to the flow cell, and
> detect light generated from analytes in the first and second specimens flowing in the flow cell.

**11.** The specimen analyzer (4000) of claim 10, wherein
the first data (82a, 82b, 82c) and the second data (82a, 82b, 82c) respectively correspond to the first optical signal (80a, 80b, 80c) and the second optical signal (80a, 80b, 80c) obtained while the analyte passes through an application position of the light.

**12.** The specimen analyzer (4000) of any one of claims 1 to 11, wherein
the analysis unit (300) is configured to analyze the first data (82a, 82b, 82c) through a convolution operation according to the AI algorithm.

**13.** The specimen analyzer (4000) of any one of claims 1 to 12, wherein
the analysis unit (300) is configured to analyze the first data (82a, 82b, 82c) through a matrix operation according to the AI algorithm.

**14.** The specimen analyzer (4000) of claim 13, wherein
the analysis unit (300) is configured to execute the matrix operation according to the AI algorithm, through parallel processing performed by a parallel-processing processor.

**15.** The specimen analyzer (4000) of claim 14, wherein
the analysis unit (300) is configured to execute the first analysis operation by the parallel-processing processor and executes the second analysis operation and the third analysis operation by a host processor of the parallel-processing processor.

**16.** The specimen analyzer (4000) of any one of claims 1 to 15, wherein
the AI algorithm is a deep learning algorithm.

**17.** The specimen analyzer (4000) of any one of claims 1 to 16, wherein

> the first reaction chamber (440a) is configured to prepare the first measurement sample on the basis of the first specimen and the first reagent that is used in order to classify white blood cells in the first specimen, and
> the second reaction chamber (440a) is configured to prepare the second measurement sample on the basis of the second specimen and the first reagent that is used in order to classify white blood cells in the second specimen.

**18.** The specimen analyzer (4000) of claim 17, wherein
the first reaction chamber (440a) is configured to prepare the first measurement sample on the basis of the first specimen and the first reagent that is used in order to classify cells in the first specimen into neutrophils, lymphocytes, monocytes, and eosinophils.

**19.** The specimen analyzer (4000) of claim 17 or 18, wherein
in analysis of the first measurement sample according to the AI algorithm, the analysis unit (300) is configured to perform classification as to whether or not a cell in the first specimen corresponds to at least either of a nucleated red blood cell and a basophil.

**20.** The specimen analyzer (4000) of any one of claims 17 to 19, wherein
the analysis unit (300) is configured to

> classify cells in the first specimen into nucleated red blood cells and basophils through the first analysis operation on the basis of the first data (82a, 82b, 82c) obtained from the first measurement sample, and
> classify cells in the first specimen into neutrophils, lymphocytes, monocytes, and eosinophils through the second analysis operation on the basis of the first data (82a, 82b, 82c) obtained from the first measurement sample.

**21.** The specimen analyzer (4000) of any one of claims 1 to 20, wherein

the analysis unit (300) is configured to execute, through the first analysis operation, analysis of the first measurement item with respect to the first measurement sample, and analyzes the second measurement item by determining, through the second analysis operation, a type of an analyte that has not correspond to the first measurement item as a result of the first analysis operation.

22. The specimen analyzer (4000) of any one of claims 1 to 21, wherein

the analysis unit (300) is configured to execute, through the second analysis operation, analysis of the second measurement item with respect to the first measurement sample, and analyzes the first measurement item by determining, through the first analysis operation, a type of an analyte that has not correspond to the second measurement item as a result of the second analysis operation.

23. A specimen analysis method for analyzing an analyte in a specimen, the specimen analysis method comprising:

preparing (S301) a first measurement sample on the basis of a first specimen and a first reagent, preparing a second measurement sample on the basis of the first reagent and a second specimen different from the first specimen, and preparing a third measurement sample on the basis of the first specimen and a second reagent;
obtaining (S1, S11, S121, S131, S302) a first optical signal (80a, 80b, 80c) from the first measurement sample, obtaining a second optical signal (80a, 80b, 80c) from the second measurement sample, and obtaining a third optical signal (80a, 80b, 80c) from the third measurement sample; and
analyzing (S2, S3, S14, S16, S71, S74, S81, S84, S91, S95, S122, S124, S132, S134, S201, S202) first data (82a, 82b, 82c) corresponding to the first optical signal (80a, 80b, 80c), analyzing second data (82a, 82b, 82c) corresponding to the second optical signal (80a, 80b, 80c), and analyzing third data (82a, 82b, 82c) corresponding to the third optical signal (80a, 80b, 80c), wherein
the analyzing (S2, S3, S14, S16, S71, S74, S81, S84, S91, S95, S122, S124, S132, S134, S201, S202) includes

executing analysis of a first measurement item with respect to the first measurement sample, through a first analysis operation of processing the first data (82a, 82b, 82c) according to an artificial intelligence, AI, algorithm (60),
executing analysis of a second measurement item different from the first measurement item with respect to the first measurement sample, through a second analysis operation of processing a first representative value obtained from the first data (82a, 82b, 82c), the first representative value corresponding to a feature of the analyte, and
executing analysis with respect to the third measurement sample, through a third analysis operation of processing a second representative value obtained from the third data (82a, 82b, 82c), the second representative value corresponding to a feature of the analyte,

the second analysis operation and the third analysis operation do not use the AI algorithm, and
preparation of the first measurement sample and preparation of the second measurement sample is executed in parallel.

24. A program for causing a computer (300, 600, 3001, 3002, 6001, 6002) to execute a process according to claim 23 on a device according to claim 1.

**Patentansprüche**

1. Probenanalysegerät (4000) zum Analysieren eines Analyten in einer Probe, wobei das Probenanalysegerät Folgendes umfasst:

eine Messeinheit (400) einschließlich

eines ersten Reagenzienbehälters, der ein erstes Reagenz lagert,
eines zweiten Reagenzienbehälters, der ein zweites Reagenz lagert,
einer ersten Reaktionskammer (440a), die so konfiguriert ist, dass sie eine erste Messprobe auf der Grundlage einer ersten Probe und des ersten Reagenzes herstellt,
einer zweiten Reaktionskammer (440a), die so konfiguriert ist, dass sie eine zweite Messprobe auf der Grundlage des ersten Reagenzes und einer zweiten, von der ersten Probe verschiedenen Probe herstellt,
einer dritten Reaktionskammer (440b, 440c, 440d), die so konfiguriert ist, dass sie eine dritte Messprobe auf der Grundlage der ersten Probe und eines zweiten Reagenzes herstellt, und

eines optischen Detektionsteils (410, 470), das so konfiguriert ist, dass es ein erstes optisches Signal (80a, 80b, 80c) von der ersten Messprobe erhält, ein zweites optisches Signal (80a, 80b, 80c) von der zweiten Messprobe erhält und ein drittes optisches Signal (80a, 80b, 80c) von der dritten Messprobe erhält; und

eine Analyseeinheit (300), die so konfiguriert ist, dass sie erste Daten (82a, 82b, 82c), die dem ersten optischen Signal (80a, 80b, 80c) entsprechen, analysiert, zweite Daten (82a, 82b, 82c), die dem zweiten optischen Signal (80a, 80b, 80c) entsprechen, analysiert und dritte Daten (82a, 82b, 82c), die dem dritten optischen Signal (80a, 80b, 80c) entsprechen, analysiert, wobei die Analyseeinheit (300) so konfiguriert ist, dass sie

eine Analyse eines ersten Messelements in Bezug auf die erste Messprobe durch eine erste Analyseoperation der Verarbeitung der ersten Daten (82a, 82b, 82c) gemäß einem Algorithmus der künstlichen Intelligenz, KI, (60) ausführt,
eine Analyse eines zweiten Messelements, das sich von dem ersten Messelement unterscheidet, in Bezug auf die erste Messprobe durch eine zweite Analyseoperation der Verarbeitung eines ersten repräsentativen Wertes, der aus den ersten Daten (82a, 82b, 82c) erhalten wird, ausführt, wobei der erste repräsentative Wert einem Merkmal des Analyten entspricht, und
eine Analyse in Bezug auf die dritte Messprobe durch eine dritte Analyseoperation der Verarbeitung eines zweiten repräsentativen Wertes, der aus den dritten Daten (82a, 82b, 82c) erhalten wird, ausführt, wobei der zweite repräsentative Wert einem Merkmal des Analyten entspricht,

wobei die zweite Analyseoperation und die dritte Analyseoperation nicht den KI-Algorithmus verwenden, und wobei die erste Reaktionskammer (440a) und die zweite Reaktionskammer (440a) parallel die Herstellung der ersten Messprobe und der zweiten Messprobe ausführen.

2.  Probenanalysegerät (4000) nach Anspruch 1, wobei
in der zweiten Analyseoperation die Analyseeinheit (300) so konfiguriert ist, dass sie den ersten repräsentativen Wert auf der Grundlage der ersten Daten (82a, 82b, 82c) spezifiziert und den spezifizierten ersten repräsentativen Wert verarbeitet.

3.  Probenanalysegerät (4000) nach Anspruch 2, wobei
in der zweiten Analyseoperation die Analyseeinheit (300) so konfiguriert ist, dass sie den ersten repräsentativen Wert auf der Grundlage einer Größe der ersten Daten (82a, 82b, 82c) spezifiziert.

4.  Probenanalysegerät (4000) nach Anspruch 2 oder 3, wobei

das erste optische Signal (80a, 80b, 80c) einen Bereich aufweist, der jedem der Analyten in der ersten Probe entspricht, und
die Analyseeinheit (300) so konfiguriert ist, dass sie den ersten repräsentativen Wert auf der Grundlage der ersten Daten (82a, 82b, 82c) entsprechend jedem der Bereiche des ersten optischen Signals (80a, 80b, 80c) spezifiziert.

5.  Probenanalysegerät (4000) nach Anspruch 4, wobei
die Analyseeinheit (300) so konfiguriert ist, dass sie als ersten repräsentativen Wert einen Spitzenwert in dem Bereich der ersten Daten (82a, 82b, 82c) spezifiziert.

6.  Probenanalysegerät (4000) nach einem der Ansprüche 1 bis 5, wobei

das erste optische Signal (80a, 80b, 80c) einen Bereich aufweist, der jedem der Analyten in der ersten Probe entspricht, und
die Analyseeinheit (300) so konfiguriert ist, dass sie in der ersten Analyseoperation die ersten Daten (82a, 82b, 82c), die jedem der Bereiche des ersten optischen Signals (80a, 80b, 80c) entsprechen, in den KI-Algorithmus eingibt.

7.  Probenanalysegerät (4000) nach einem der Ansprüche 1 bis 6, wobei
die Messeinheit (400) so konfiguriert ist, dass sie die ersten Daten (82a, 82b, 82c) auf der Grundlage eines Signals erhält, das größer als ein vorbestimmter Schwellenwert ist und der Intensität des ersten optischen Signals (80a, 80b, 80c) entspricht.

8. Probenanalysegerät (4000) nach Anspruch 1, wobei

die Messeinheit (400) so konfiguriert ist, dass sie den ersten repräsentativen Wert auf der Grundlage des ersten optischen Signals (80a, 80b, 80c) erhält, und
die Analyseeinheit (300) so konfiguriert ist, dass sie in der zweiten Analyseoperation den von der Messeinheit (400) erhaltenen ersten repräsentativen Wert verarbeitet.

9. Probenanalysegerät (4000) nach einem der Ansprüche 1 bis 8, wobei
das erste optische Signal (80a, 80b, 80c) ein Signal ist, das das Vorhandensein eines Analyten in der ersten Probe widerspiegelt.

10. Probenanalysegerät (4000) nach einem der Ansprüche 1 bis 9, wobei
das optische Detektionsteil (410, 470)

eine Lichtquelle, eine Durchflusszelle und einen Fotodetektor einschließt und so konfiguriert ist, dass
Licht an der Durchflusszelle anwendet, und
das von den Analyten in der ersten und der zweiten Probe, die in der Durchflusszelle fließen, erzeugte Licht detektiert.

11. Probenanalysegerät (4000) nach Anspruch 10, wobei
die ersten Daten (82a, 82b, 82c) und die zweiten Daten (82a, 82b, 82c) dem ersten optischen Signal (80a, 80b, 80c) bzw. dem zweiten optischen Signal (80a, 80b, 80c) entsprechen, die erhalten werden, während der Analyt eine Anwendungsposition des Lichts durchläuft.

12. Probenanalysegerät (4000) nach einem der Ansprüche 1 bis 11, wobei
die Analyseeinheit (300) so konfiguriert ist, dass sie die ersten Daten (82a, 82b, 82c) durch eine Faltungsoperation gemäß dem KI-Algorithmus analysiert.

13. Probenanalysegerät (4000) nach einem der Ansprüche 1 bis 12, wobei
die Analyseeinheit (300) so konfiguriert ist, dass sie die ersten Daten (82a, 82b, 82c) durch eine Matrixoperation gemäß dem KI-Algorithmus analysiert.

14. Probenanalysegerät (4000) nach Anspruch 13, wobei
die Analyseeinheit (300) so konfiguriert ist, dass sie die Matrixoperation gemäß dem KI-Algorithmus durch parallele Verarbeitung, die von einem Parallelverarbeitungsprozessor durchgeführt wird, ausführt.

15. Probenanalysegerät (4000) nach Anspruch 14, wobei
die Analyseeinheit (300) so konfiguriert ist, dass sie die erste Analyseoperation durch den Parallelverarbeitungsprozessor ausführt und die zweite Analyseoperation und die dritte Analyseoperation durch einen Host-Prozessor des Parallelverarbeitungsprozessors ausführt.

16. Probenanalysegerät (4000) nach einem der Ansprüche 1 bis 15, wobei
der KI-Algorithmus ein Deep-Learning-Algorithmus ist.

17. Probenanalysegerät (4000) nach einem der Ansprüche 1 bis 16, wobei

die erste Reaktionskammer (440a) so konfiguriert ist, dass sie die erste Messprobe auf der Grundlage der ersten Probe und des ersten Reagenzes, das verwendet wird, um weiße Blutzellen in der ersten Probe zu klassifizieren, herstellt, und
die zweite Reaktionskammer (440a) so konfiguriert ist, dass sie die zweite Messprobe auf der Grundlage der zweiten Probe und des ersten Reagenzes, das verwendet wird, um weiße Blutkörperchen in der zweiten Probe zu klassifizieren, herstellt.

18. Probenanalysegerät (4000) nach Anspruch 17, wobei
die erste Reaktionskammer (440a) so konfiguriert ist, dass sie die erste Messprobe auf der Grundlage der ersten Probe und des ersten Reagenzes herstellt, das verwendet wird, um Zellen in der ersten Probe in Neutrophile, Lymphozyten, Monozyten und Eosinophile zu klassifizieren.

19. Probenanalysegerät (4000) nach Anspruch 17 oder 18, wobei
bei der Analyse der ersten Messprobe gemäß dem KI-Algorithmus die Analyseeinheit (300) so konfiguriert ist, dass sie eine Klassifizierung dahingehend durchführt, ob eine Zelle in der ersten Probe mindestens einem kernhaltigen roten Blutkörperchen oder einem Basophil entspricht oder nicht.

20. Probenanalysegerät (4000) nach einem der Ansprüche 17 bis 19, wobei

    die Analyseeinheit (300) so konfiguriert ist, dass sie
    Zellen in der ersten Probe in kernhaltige rote Blutkörperchen und Basophile durch die erste Analyseoperation auf der Grundlage der von der ersten Messprobe erhaltenen ersten Daten (82a, 82b, 82c) klassifiziert, und
    Zellen in der ersten Probe in Neutrophile, Lymphozyten, Monozyten und Eosinophile durch die zweite Analyseoperation auf der Grundlage der aus der ersten Messprobe erhaltenen ersten Daten (82a, 82b, 82c) klassifiziert.

21. Probenanalysegerät (4000) nach einem der Ansprüche 1 bis 20, wobei
die Analyseeinheit (300) so konfiguriert ist, dass sie durch die erste Analyseoperation eine Analyse des ersten Messelements in Bezug auf die erste Messprobe ausführt und das zweite Messelement analysiert, indem sie durch die zweite Analyseoperation einen Typ eines Analyten bestimmt, der als Ergebnis der ersten Analyseoperation nicht dem ersten Messelement entsprochen hat.

22. Probenanalysegerät (4000) nach einem der Ansprüche 1 bis 21, wobei
die Analyseeinheit (300) so konfiguriert ist, dass sie durch die zweite Analyseoperation eine Analyse des zweiten Messelements in Bezug auf die erste Messprobe ausführt und das erste Messelement analysiert, indem sie durch die erste Analyseoperation einen Typ eines Analyten bestimmt, der als Ergebnis der zweiten Analyseoperation nicht dem zweiten Messelement entsprochen hat.

23. Probenanalyseverfahren zum Analysieren eines Analyten in einer Probe, wobei das Probenanalyseverfahren Folgendes umfasst:

    Herstellen (S301) einer ersten Messprobe auf der Grundlage einer ersten Probe und eines ersten Reagenzes, Herstellen einer zweiten Messprobe auf der Grundlage des ersten Reagenzes und einer zweiten, von der ersten Probe verschiedenen Probe, und Herstellen einer dritten Messprobe auf der Grundlage der ersten Probe und eines zweiten Reagenzes;
    Erhalten (S1, S11, S121, S131, S302) eines ersten optischen Signals (80a, 80b, 80c) von der ersten Messprobe, Erhalten eines zweiten optischen Signals (80a, 80b, 80c) von der zweiten Messprobe, und Erhalten eines dritten optischen Signals (80a, 80b, 80c) von der dritten Messprobe; und
    Analysieren (S2, S3, S14, S16, S71, S74, S81, S84, S91, S95, S122, S124, S132, S134, S201, S202) erster Daten (82a, 82b, 82c) entsprechend dem ersten optischen Signal (80a, 80b, 80c), Analysieren zweiter Daten (82a, 82b, 82c) entsprechend dem zweiten optischen Signal (80a, 80b, 80c), und Analysieren dritter Daten (82a, 82b, 82c) entsprechend dem dritten optischen Signal (80a, 80b, 80c), wobei
    das Analysieren (S2, S3, S14, S16, S71, S74, S81, S84, S91, S95, S122, S124, S132, S134, S201, S202) Folgendes einschließt

        Ausführen einer Analyse eines ersten Messelements in Bezug auf die erste Messprobe durch eine erste Analyseoperation der Verarbeitung der ersten Daten (82a, 82b, 82c) gemäß einem Algorithmus der künstlichen Intelligenz, KI, (60),
        Ausführen einer Analyse eines zweiten Messelements, das sich von dem ersten Messelement unterscheidet, in Bezug auf die erste Messprobe durch eine zweite Analyseoperation der Verarbeitung eines ersten repräsentativen Wertes, der aus den ersten Daten (82a, 82b, 82c) erhalten wird, wobei der erste repräsentative Wert einem Merkmal des Analyten entspricht, und
        Ausführen einer Analyse in Bezug auf die dritte Messprobe durch eine dritte Analyseoperation der Verarbeitung eines zweiten repräsentativen Wertes, der aus den dritten Daten (82a, 82b, 82c) erhalten wird, wobei der zweite repräsentative Wert einem Merkmal des Analyten entspricht,

    wobei die zweite Analyseoperation und die dritte Analyseoperation nicht den KI-Algorithmus verwenden, und
    wobei die Herstellung der ersten Messprobe und die Herstellung der zweiten Messprobe parallel ausgeführt werden.

**24.** Programm, das einen Computer (300, 600, 3001, 3002, 6001, 6002) veranlasst, einen Prozess nach Anspruch 23 auf einer Vorrichtung nach Anspruch 1 auszuführen.

**Revendications**

**1.** Analyseur d'échantillon (4000) pour analyser un analyte dans un échantillon, l'analyseur d'échantillon comprenant :

une unité de mesure (400) incluant

un premier conteneur de réactif stockant un premier réactif,
un second conteneur de réactif stockant un second réactif,
une première chambre de réaction (440a) configurée pour préparer un premier échantillon de mesure sur la base d'un premier échantillon et du premier réactif,
une deuxième chambre de réaction (440a) configurée pour préparer un deuxième échantillon de mesure sur la base du premier réactif et d'un second échantillon différent du premier échantillon,
une troisième chambre de réaction (440b, 440c, 440d) configurée pour préparer un troisième échantillon de mesure sur la base du premier échantillon et d'un second réactif, et
une partie de détection optique (410, 470) configurée pour obtenir un premier signal optique (80a, 80b, 80c) du premier échantillon de mesure, obtenir un deuxième signal optique (80a, 80b, 80c) du deuxième échantillon de mesure et obtenir un troisième signal optique (80a, 80b, 80c) du troisième échantillon de mesure ; et

une unité d'analyse (300) configurée pour analyser des premières données (82a, 82b, 82c) correspondant au premier signal optique (80a, 80b, 80c), analyser des deuxièmes données (82a, 82b, 82c) correspondant au deuxième signal optique (80a, 80b, 80c), et analyser des troisièmes données (82a, 82b, 82c) correspondant au troisième signal optique (80a, 80b, 80c), dans lequel
l'unité d'analyse (300) est configurée pour

exécuter une analyse d'un premier élément de mesure par rapport au premier échantillon de mesure, par une première opération d'analyse consistant à traiter les premières données (82a, 82b, 82c) selon un algorithme d'intelligence artificielle (AI) (60),
exécuter une analyse d'un second élément de mesure différent du premier élément de mesure par rapport au premier échantillon de mesure, par une deuxième opération d'analyse consistant à traiter une première valeur représentative obtenue à partir des premières données (82a, 82b, 82c), la première valeur représentative correspondant à une caractéristique de l'analyte, et
exécuter une analyse par rapport au troisième échantillon de mesure, par une troisième opération d'analyse consistant à traiter une seconde valeur représentative obtenue à partir des troisièmes données (82a, 82b, 82c), la seconde valeur représentative correspondant à une caractéristique de l'analyte,

la deuxième opération d'analyse et la troisième opération d'analyse n'utilisent pas l'algorithme d'AI, et
la première chambre de réaction (440a) et la deuxième chambre de réaction (440a) exécutent, en parallèle, la préparation du premier échantillon de mesure et du deuxième échantillon de mesure.

**2.** Analyseur d'échantillon (4000) selon la revendication 1, dans lequel
dans la deuxième opération d'analyse, l'unité d'analyse (300) est configurée pour spécifier la première valeur représentative sur la base des premières données (82a, 82b, 82c) et traite la première valeur représentative spécifiée.

**3.** Analyseur d'échantillon (4000) selon la revendication 2, dans lequel
dans la deuxième opération d'analyse, l'unité d'analyse (300) est configurée pour spécifier la première valeur représentative sur la base d'une amplitude des premières données (82a, 82b, 82c).

**4.** Analyseur d'échantillon (4000) selon la revendication 2 ou la revendication 3, dans lequel

le premier signal optique (80a, 80b, 80c) présente une région correspondant à chacun des analytes du premier échantillon, et
l'unité d'analyse (300) est configurée pour spécifier la première valeur représentative sur la base des premières données (82a, 82b, 82c) correspondant à chacune des régions du premier signal optique (80a, 80b, 80c).

**5.** Analyseur d'échantillon (4000) selon la revendication 4, dans lequel
l'unité d'analyse (300) est configurée pour spécifier, en tant que première valeur représentative, une valeur de crête dans la région des premières données (82a, 82b, 82c).

**6.** Analyseur d'échantillon (4000) selon l'une quelconque des revendications 1 à 5, dans lequel

le premier signal optique (80a, 80b, 80c) présente une région correspondant à chacun des analytes du premier échantillon, et
dans la première opération d'analyse, l'unité d'analyse (300) est configurée pour transmettre à l'algorithme d'AI les premières données (82a, 82b, 82c) correspondant à chacune des régions du premier signal optique (80a, 80b, 80c).

**7.** Analyseur d'échantillon (4000) selon l'une quelconque des revendications 1 à 6, dans lequel
l'unité de mesure (400) est configurée pour obtenir les premières données (82a, 82b, 82c) sur la base d'un signal supérieur à un seuil prédéterminé et correspondant à une intensité du premier signal optique (80a, 80b, 80c).

**8.** Analyseur d'échantillon (4000) selon la revendication 1, dans lequel

l'unité de mesure (400) est configurée pour obtenir la première valeur représentative sur la base du premier signal optique (80a, 80b, 80c), et
dans la deuxième opération d'analyse, l'unité d'analyse (300) est configurée pour traiter la première valeur représentative obtenue par l'unité de mesure (400).

**9.** Analyseur d'échantillon (4000) selon l'une quelconque des revendications 1 à 8, dans lequel
le premier signal optique (80a, 80b, 80c) est un signal qui reflète la présence d'un analyte dans le premier échantillon.

**10.** Analyseur d'échantillon (4000) selon l'une quelconque des revendications 1 à 9, dans lequel
la partie de détection optique (410, 470)

inclut une source de lumière, une cuve à circulation et un photodétecteur, et
est configurée pour appliquer de la lumière sur la cuve à circulation, et
détecter de la lumière générée par des analytes dans les premier et deuxième échantillons circulant dans la cuve à circulation.

**11.** Analyseur d'échantillon (4000) selon la revendication 10, dans lequel
les premières données (82a, 82b, 82c) et les deuxièmes données (82a, 82b, 82c) correspondent respectivement au premier signal optique (80a, 80b, 80c) et au deuxième signal optique (80a, 80b, 80c) obtenus lorsque l'analyte passe à travers une position d'application de la lumière.

**12.** Analyseur d'échantillon (4000) selon l'une quelconque des revendications 1 à 11, dans lequel
l'unité d'analyse (300) est configurée pour analyser les premières données (82a, 82b, 82c) par une opération de convolution selon l'algorithme d'AI.

**13.** Analyseur d'échantillon (4000) selon l'une quelconque des revendications 1 à 12, dans lequel
l'unité d'analyse (300) est configurée pour analyser les premières données (82a, 82b, 82c) par une opération matricielle selon l'algorithme d'AI.

**14.** Analyseur d'échantillon (4000) selon la revendication 13, dans lequel
l'unité d'analyse (300) est configurée pour exécuter l'opération matricielle selon l'algorithme d'AI, par un traitement parallèle effectué par un processeur de traitement parallèle.

**15.** Analyseur d'échantillon (4000) selon la revendication 14, dans lequel
l'unité d'analyse (300) est configurée pour exécuter la première opération d'analyse par le processeur de traitement parallèle et exécuter la deuxième opération d'analyse et la troisième opération d'analyse par un processeur hôte du processeur de traitement parallèle.

**16.** Analyseur d'échantillon (4000) selon l'une quelconque des revendications 1 à 15, dans lequel
l'algorithme d'AI est un algorithme d'apprentissage profond.

**17.** Analyseur d'échantillon (4000) selon l'une quelconque des revendications 1 à 16, dans lequel

la première chambre de réaction (440a) est configurée pour préparer le premier échantillon de mesure sur la base du premier échantillon et du premier réactif utilisé pour classer des globules blancs du premier échantillon, et la deuxième chambre de réaction (440a) est configurée pour préparer le deuxième échantillon de mesure sur la base du second échantillon et du premier réactif utilisé pour classer des globules blancs du second échantillon.

**18.** Analyseur d'échantillon (4000) selon la revendication 17, dans lequel
la première chambre de réaction (440a) est configurée pour préparer le premier échantillon de mesure sur la base du premier échantillon et du premier réactif utilisé pour classer des cellules dans le premier échantillon en neutrophiles, lymphocytes, monocytes et éosinophiles.

**19.** Analyseur d'échantillon (4000) selon la revendication 17 ou la revendication 18, dans lequel
dans l'analyse du premier échantillon de mesure selon
l'algorithme d'AI, l'unité d'analyse (300) est configurée pour effectuer une classification de manière à déterminer si une cellule du premier échantillon correspond ou non à au moins soit un globule rouge nucléé soit un basophile.

**20.** Analyseur d'échantillon (4000) selon l'une quelconque des revendications 17 à 19, dans lequel

l'unité d'analyse (300) est configurée pour
classer des cellules du premier échantillon en globules rouges nucléés et en basophiles par la première opération d'analyse sur la base des premières données (82a, 82b, 82c) obtenues à partir du premier échantillon de mesure, et
classer des cellules du premier échantillon en neutrophiles, lymphocytes, monocytes et éosinophiles par la deuxième opération d'analyse sur la base des premières données (82a, 82b, 82c) obtenues à partir du premier échantillon de mesure.

**21.** Analyseur d'échantillon (4000) selon l'une quelconque des revendications 1 à 20, dans lequel
l'unité d'analyse (300) est configurée pour exécuter, par la première opération d'analyse, l'analyse du premier élément de mesure par rapport au premier échantillon de mesure, et analyse le second élément de mesure en déterminant, par la deuxième opération d'analyse, un type d'un analyte qui n'a pas correspondu au premier élément de mesure à la suite de la première opération d'analyse.

**22.** Analyseur d'échantillon (4000) selon l'une quelconque des revendications 1 à 21, dans lequel
l'unité d'analyse (300) est configurée pour exécuter, par la deuxième opération d'analyse, l'analyse du second élément de mesure par rapport au premier échantillon de mesure, et analyse le premier élément de mesure en déterminant, par la première opération d'analyse, un type d'un analyte qui n'a pas correspondu au second élément de mesure à la suite de la deuxième opération d'analyse.

**23.** Procédé d'analyse d'échantillon pour analyser un analyte dans un échantillon, le procédé d'analyse d'échantillon comprenant :

la préparation (S301) d'un premier échantillon de mesure sur la base d'un premier échantillon et d'un premier réactif, la préparation d'un deuxième échantillon de mesure sur la base du premier réactif et d'un second échantillon différent du premier échantillon, et la préparation d'un troisième échantillon de mesure sur la base du premier échantillon et d'un second réactif ;
l'obtention (S1, S11, S121, S131, S302) d'un premier signal optique (80a, 80b, 80c) à partir du premier échantillon de mesure, l'obtention d'un deuxième signal optique (80a, 80b, 80c) à partir du deuxième échantillon de mesure, et l'obtention d'un troisième signal optique (80a, 80b, 80c) à partir du troisième échantillon de mesure ; et
l'analyse (S2, S3, S14, S16, S71, S74, S81, S84, S91, S95, S122, S124, S132, S134, S201, S202) des premières données (82a, 82b, 82c) correspondant au premier signal optique (80a, 80b, 80c), l'analyse des deuxièmes données (82a, 82b, 82c) correspondant au deuxième signal optique (80a, 80b, 80c), et l'analyse des troisièmes données (82a, 82b, 82c) correspondant au troisième signal optique (80a, 80b, 80c), dans lequel
l'analyse (S2, S3, S14, S16, S71, S74, S81, S84, S91, S95, S122, S124, S132, S134, S201, S202) inclut

l'exécution d'une analyse d'un premier élément de mesure par rapport au premier échantillon de mesure, par une première opération d'analyse consistant à traiter les premières données (82a, 82b, 82c) selon un algorithme d'intelligence artificielle (AI) (60),

l'exécution d'une analyse d'un second élément de mesure différent du premier élément de mesure par rapport au premier échantillon de mesure, par une deuxième opération d'analyse consistant à traiter une première valeur représentative obtenue à partir des premières données (82a, 82b, 82c), la première valeur représentative correspondant à une caractéristique de l'analyte, et

l'exécution d'une analyse par rapport au troisième échantillon de mesure, par une troisième opération d'analyse consistant à traiter une seconde valeur représentative obtenue à partir des troisièmes données (82a, 82b, 82c), la seconde valeur représentative correspondant à une caractéristique de l'analyte,

la deuxième opération d'analyse et la troisième opération d'analyse n'utilisent pas l'algorithme d'AI, et

la préparation du premier échantillon de mesure et la préparation du deuxième échantillon de mesure sont exécutées en parallèle.

24. Programme pour amener un ordinateur (300, 600, 3001, 3002, 6001, 6002) à exécuter un processus selon la revendication 23 sur un dispositif selon la revendication 1.

# FIG. 1

CONFIGURATION EXAMPLE OF EMBODIMENT 1

4000

400    300

MODIFICATION OF CONFIGURATION OF EMBODIMENT 1

400

4000

300

FIG. 2

CALCULATION PROCESSING ANALYSIS

FSC

SSC

FL

REPRESENTATIVE
VALUE

FLP

SC

SSCP

CLASSIFICATION
AND ANALYSIS

AI ANALYSIS

FSC

SSC

FL

WAVEFORM DATA

60

AI ALGORITHM

CLASSIFICATION
AND ANALYSIS

EP 4 246 123 B1

FIG. 3

PEAK VALUE

WIDTH

AREA

FIG. 4

FIG. 5

```
          ┌─────────────┐
          │    START    │
          └─────────────┘
                 │
                 ▼
  ┌──────────────────────────────────┐  S1
  │   OBTAIN OPTICAL SIGNAL AND OBTAIN │
  │   WAVEFORM DATA FROM OPTICAL SIGNAL│
  └──────────────────────────────────┘
                 │
                 ▼
  ┌──────────────────────────────────┐  S2
  │   EXECUTE AI ANALYSIS ON FIRST DATA│
  └──────────────────────────────────┘
                 │
                 ▼
  ┌──────────────────────────────────┐  S3
  │ EXECUTE CALCULATION PROCESSING ANALYSIS│
  │           ON SECOND DATA           │
  └──────────────────────────────────┘
                 │
                 ▼
  ┌──────────────────────────────────┐  S4
  │      PROVIDE ANALYSIS RESULT       │
  └──────────────────────────────────┘
                 │
                 ▼
          ┌─────────────┐
          │     END     │
          └─────────────┘
```

# FIG. 6

EXAMPLE IN WHICH ANALYSIS IS EXECUTED
ON THE BASIS OF RULE

EMBODIMENT 2

```
        ( START )
            │
            ▼
┌───────────────────────────────┐ S11
│  OBTAIN OPTICAL SIGNAL AND     │
│  OBTAIN WAVEFORM DATA FROM     │
│  OPTICAL SIGNAL                │
└───────────────────────────────┘
            │
            ▼
┌───────────────────────────────┐ S12
│ SPECIFY WAVEFORM DATA ON THE   │
│ BASIS OF RULE                  │
└───────────────────────────────┘
            │
            ▼
         S13
    ╱ IS THERE WAVEFORM ╲
NO ╱  DATA OF AI ANALYSIS ╲
  ◄    TARGET?           
    ╲                   ╱
     ╲                 ╱
          │ YES
          ▼
┌───────────────────────────────┐ S14
│         AI ANALYSIS           │
└───────────────────────────────┘
          │
          ▼
        S15
    ╱ IS THERE ╲
   ╱ WAVEFORM DATA OF ╲    NO
  ╱ CALCULATION PROCESSING ╲──►
   ╲ ANALYSIS TARGET?     ╱
    ╲                   ╱
       │ YES
       ▼
┌───────────────────────────────┐ S16
│  CALCULATION PROCESSING        │
│  ANALYSIS                      │
└───────────────────────────────┘
          │
          ▼
┌───────────────────────────────┐ S17
│     PROVIDE ANALYSIS RESULT    │
└───────────────────────────────┘
          │
          ▼
       ( END )
```

## FIG. 7

EXAMPLE IN WHICH ANALYSIS IS EXECUTED
IN ACCORDANCE WITH MEASUREMENT ITEM                    EMBODIMENT 3

FIG. 8

OPERATION MODE SETTING

| MEASUREMENT ITEM | AI | CALCULATION PROCESSING |
|---|---|---|
| NRBC# | ☑ | ☐ |
| NRBC% | ☑ | ☐ |
| NEUT# | ☐ | ☑ |
| LYMPH# | ☐ | ☑ |
| MONO# | ☐ | ☑ |
| BASO# | ☑ | ☐ |
| EO# | ☐ | ☑ |
| NEUT% | ☐ | ☑ |
| LYMPH% | ☐ | ☑ |
| MONO% | ☐ | ☑ |
| BASO% | ☑ | ☐ |
| EO% | ☐ | ☑ |

SET

# FIG. 9

EXAMPLE IN WHICH ANALYSIS IS EXECUTED
IN ACCORDANCE WITH MEASUREMENT ORDER

START

OBTAIN OPTICAL SIGNAL AND OBTAIN
WAVEFORM DATA FROM OPTICAL SIGNAL — S11

SPECIFY WAVEFORM DATA ON THE BASIS OF
MEASUREMENT ORDER — S31

WAVEFORM DATA
OF MEASUREMENT ORDER OF AI
ANALYSIS TARGET? — S32

NO

YES

AI ANALYSIS — S14

ANALYSIS ACCORDING TO
CALCULATION PROCESSING — S16

PROVIDE ANALYSIS RESULT — S17

END

FIG. 10

OPERATION MODE SETTING

| SPECIMEN NUMBER | AI ANALYSIS MODE | CALCULATION PROCESSING ANALYSIS MODE |
|---|---|---|
| 0000099266 | ☑ | ☐ |
| 0000099267 | ☐ | ☑ |
| 0000099268 | ☐ | ☑ |
| 0000099269 | ☑ | ☐ |
| 0000099270 | ☐ | ☑ |
| 0000099271 | ☐ | ☑ |
| 0000099272 | ☐ | ☑ |
| 0000099273 | ☑ | ☐ |
| 0000099274 | ☐ | ☑ |
| 0000099275 | ☐ | ☑ |
| 0000099276 | ☑ | ☐ |
| 0000099277 | ☐ | ☑ |

SET

FIG. 11

EXAMPLE IN WHICH ANALYSIS IS EXECUTED IN
ACCORDANCE WITH ANALYSIS MODE OF APPARATUS

START

OBTAIN OPTICAL SIGNAL AND OBTAIN
WAVEFORM DATA FROM OPTICAL SIGNAL — S11

SPECIFY WAVEFORM DATA ON THE BASIS OF
ANALYSIS MODE OF APPARATUS — S41

WAVEFORM DATA OF AI
ANALYSIS TARGET? — S42

NO

YES

AI ANALYSIS — S14

CALCULATION PROCESSING ANALYSIS — S16

PROVIDE ANALYSIS RESULT — S17

END

FIG. 12

| OPERATION MODE SETTING |
| --- |

☑ AI ANALYSIS MODE

☐ CALCULATION PROCESSING ANALYSIS MODE

| SET |
| --- |

# FIG. 13

EXAMPLE IN WHICH ANALYSIS IS EXECUTED IN
ACCORDANCE WITH TYPE OF MEASUREMENT ORDER

```
                    ┌──────────────┐
                    │    START     │
                    └──────────────┘
                           │
                           ▼
        ┌──────────────────────────────────┐  S11
        │   OBTAIN OPTICAL SIGNAL AND OBTAIN│
        │  WAVEFORM DATA FROM OPTICAL SIGNAL│
        └──────────────────────────────────┘
                           │
                           ▼
        ┌──────────────────────────────────┐  S51
        │  SPECIFY WAVEFORM DATA ON THE BASIS OF│
        │      TYPE OF MEASUREMENT ORDER    │
        └──────────────────────────────────┘
                           │
                           ▼
                        S52
              ◇ WAVEFORM DATA OF AI ◇───── NO ──┐
                ANALYSIS TARGET?                │
                    │ YES                       │
                    ▼  S14                       ▼  S16
        ┌──────────────────┐       ┌──────────────────────────────┐
        │    AI ANALYSIS    │       │ CALCULATION PROCESSING ANALYSIS│
        └──────────────────┘       └──────────────────────────────┘
                    │                       │
                    ▼◄──────────────────────┘
        ┌──────────────────────────────────┐  S17
        │       PROVIDE ANALYSIS RESULT     │
        └──────────────────────────────────┘
                           │
                           ▼
                    ┌──────────────┐
                    │     END      │
                    └──────────────┘
```

FIG. 14

OPERATION MODE SETTING

| MEASUREMENT ORDER TYPE | AI ANALYSIS MODE | CALCULATION PROCESSING ANALYSIS MODE |
|---|---|---|
| Normal | ☐ | ☑ |
| Rerun | ☑ | ☐ |
| Reflex | ☑ | ☐ |

SET

# FIG. 15

EXAMPLE IN WHICH ANALYSIS IS EXECUTED IN ACCORDANCE
WITH MEASUREMENT ITEM AND TYPE OF MEASUREMENT ORDER

START

S11 OBTAIN OPTICAL SIGNAL AND OBTAIN WAVEFORM DATA FROM OPTICAL SIGNAL

S61 SPECIFY WAVEFORM DATA ON THE BASIS OF MEASUREMENT ITEM AND TYPE OF MEASUREMENT ORDER

S13 IS THERE WAVEFORM DATA OF AI ANALYSIS TARGET?

NO

YES

S14 AI ANALYSIS

S15 IS THERE WAVEFORM DATA OF CALCULATION PROCESSING ANALYSIS TARGET?

NO

YES

S16 CALCULATION PROCESSING ANALYSIS

S17 PROVIDE ANALYSIS RESULT

END

# FIG. 16

OPERATION MODE SETTING

| MEASUREMENT ITEM | AI ANALYSIS | CALCULATION PROCESSING ANALYSIS |
|---|---|---|
| NRBC# | ☑ | ☐ |
| NRBC% | ☑ | ☐ |
| NEUT# | ☐ | ☑ |
| LYMPH# | ☐ | ☑ |
| MONO# | ☐ | ☑ |
| BASO# | ☑ | ☐ |
| EO# | ☐ | ☑ |
| NEUT% | ☐ | ☑ |
| LYMPH% | ☐ | ☑ |
| MONO% | ☐ | ☑ |
| BASO% | ☑ | ☐ |
| EO% | ☐ | ☑ |

| MEASUREMENT ORDER TYPE | AI ANALYSIS MODE | CALCULATION PROCESSING ANALYSIS MODE |
|---|---|---|
| Normal | ☐ | ☑ |
| Rerun | ☑ | ☐ |
| Reflex | ☑ | ☐ |

SET

# FIG. 17

EXAMPLE IN WHICH WHETHER OR NOT AI ANALYSIS IS NECESSARY IS DETERMINED
ON THE BASIS OF FLAG ACCORDING TO CALCULATION PROCESSING ANALYSIS

FIG. 18

| OPERATION MODE SETTING | |
|---|---|

| FLAG | AI ANALYSIS |
|---|---|
| ABNORMALITY IN DIFFERENTIATION OF WHITE BLOOD CELL | ☐ |
| DECREASE OF NEUTROPHIL | ☐ |
| INCREASE OF NEUTROPHIL | ☐ |
| DECREASE OF LYMPHOCYTE | ☐ |
| INCREASE OF LYMPHOCYTE | ☐ |
| INCREASE OF MONOCYTE | ☐ |
| INCREASE OF EOSINOPHIL | ☐ |
| INCREASE OF BASOPHIL | ☐ |
| DECREASE OF WHITE BLOOD CELL | ☐ |
| INCREASE OF WHITE BLOOD CELL | ☐ |
| EMERGENCE OF NUCLEATED RED BLOOD CELL | ☐ |
| EMERGENCE OF IMMATURE GRANULOCYTE | ☐ |
| BLAST/ABNORMAL LYMPHOCYTE | ☑ |
| BLAST | ☑ |
| ABNORMAL LYMPHOCYTE | ☑ |
| ATYPICAL LYMPHOCYTE | ☑ |

SET

FIG. 19

EXAMPLE IN WHICH AI ANALYSIS IS EXECUTED WITH RESPECT TO
SPECIFIC ANALYTE CLASSIFIED IN CALCULATION PROCESSING ANALYSIS

START

OBTAIN OPTICAL SIGNAL AND OBTAIN
WAVEFORM DATA FROM OPTICAL SIGNAL — S11

CALCULATION PROCESSING ANALYSIS — S81

REFER TO RULE FOR SELECTING ANALYSIS
OPERATION — S82

SPECIFY WAVEFORM DATA THAT CORRESPONDS
TO ANALYTE CLASSIFIED AS SPECIFIC
CLASSIFICATION — S83

EXECUTE AI ANALYSIS ON SPECIFIED
WAVEFORM DATA — S84

PROVIDE ANALYSIS RESULT — S17

END

FIG. 20

| OPERATION MODE SETTING | | |
| --- | --- | --- |

| TYPE | AI ANALYSIS |
| --- | --- |
| MONOCYTE | ☑ |
| LYMPHOCYTE | ☑ |
| NEUTROPHIL | ☐ |
| EOSINOPHIL | ☐ |

SET

FIG. 21

CLASSIFY INTO MONOCYTE AND LYMPHOCYTE
THROUGH CALCULATION PROCESSING ANALYSIS

FLP

SSCP

WAVEFORM DATA

AI ANALYSIS

# FIG. 22

EXAMPLE IN WHICH AI ANALYSIS IS EXECUTED WHEN SPECIFIC
CLASSIFICATION HAS BEEN PERFORMED IN CALCULATION PROCESSING ANALYSIS

```
                    ( START )
                        |
                        v
        ┌───────────────────────────────┐  S11
        │   OBTAIN OPTICAL SIGNAL AND OBTAIN │
        │   WAVEFORM DATA FROM OPTICAL SIGNAL │
        └───────────────────────────────┘
                        |
                        v
        ┌───────────────────────────────┐  S91
        │   CALCULATION PROCESSING ANALYSIS │
        └───────────────────────────────┘
                        |
                        v
        ┌───────────────────────────────┐  S92
        │   REFER TO RULE FOR SELECTING ANALYSIS │
        │              OPERATION              │
        └───────────────────────────────┘
                        |
                        v
                      S93
                  ╱ HAS SPECIFIC ╲
               ╱   CLASSIFICATION BEEN   ╲  NO
               ╲      DETECTED?         ╱ ───────┐
                  ╲               ╱              │
                      YES                        │
                        |                        │
                        v                        │
        ┌───────────────────────────────┐  S94   │
        │  SPECIFY WAVEFORM DATA THAT CORRESPONDS │
        │   TO ANALYTE CLASSIFIED AS SPECIFIC │
        │            CLASSIFICATION            │
        └───────────────────────────────┘        │
                        |                         │
                        v                         │
        ┌───────────────────────────────┐  S95    │
        │   EXECUTE AI ANALYSIS ON SPECIFIED │     │
        │           WAVEFORM DATA          │      │
        └───────────────────────────────┘        │
                        |  <──────────────────────┘
                        v
        ┌───────────────────────────────┐  S17
        │       PROVIDE ANALYSIS RESULT       │
        └───────────────────────────────┘
                        |
                        v
                    (  END  )
```

FIG. 23

| | | | |
|---|---|---|---|
| | OPERATION MODE SETTING | | |

| TYPE | AI ANALYSIS |
|---|---|
| BLAST | ☑ |
| ABNORMAL LYMPHOCYTE | ☑ |
| ATYPICAL LYMPHOCYTE | ☑ |
| IMMATURE GRANULOCYTE | ☐ |

SET

FIG. 24

EMBODIMENT 4

EP 4 246 123 B1

FIG. 25

FIG. 26

EP 4 246 123 B1

FIG. 27

FIG. 28

FIG. 29

# FIG. 30

EXAMPLE IN WHICH ANALYSIS IS EXECUTED IN
ACCORDANCE WITH MEASUREMENT CHANNEL

```
                    ┌──────────────┐
                    │    START     │
                    └──────────────┘
                           │
                           ▼
        ┌──────────────────────────────────────┐
        │   OBTAIN OPTICAL SIGNAL AND OBTAIN     │  S11
        │   WAVEFORM DATA FROM OPTICAL SIGNAL    │
        └──────────────────────────────────────┘
                           │
                           ▼
        ┌──────────────────────────────────────┐
        │   SPECIFY WAVEFORM DATA ON THE BASIS OF │  S101
        │          MEASUREMENT CHANNEL           │
        └──────────────────────────────────────┘
                           │
                           ▼
                    IS THERE WAVEFORM          S13
             NO     DATA OF AI ANALYSIS
        ◀───────         TARGET?
                           │
                          YES
                           │
                           ▼
        ┌──────────────────────────────────────┐
        │              AI ANALYSIS               │  S14
        └──────────────────────────────────────┘
                           │
                           ▼
                       IS THERE               S15
                   WAVEFORM DATA OF
                 CALCULATION PROCESSING    NO
                   ANALYSIS TARGET?      ───────▶
                           │
                          YES
                           │
                           ▼
        ┌──────────────────────────────────────┐
        │      CALCULATION PROCESSING ANALYSIS   │  S16
        └──────────────────────────────────────┘
                           │
                           ▼
        ┌──────────────────────────────────────┐
        │          PROVIDE ANALYSIS RESULT       │  S17
        └──────────────────────────────────────┘
                           │
                           ▼
                    ┌──────────────┐
                    │     END      │
                    └──────────────┘
```

FIG. 31

OPERATION MODE SETTING

| MEASUREMENT CHANNEL TYPE | AI ANALYSIS | CALCULATION PROCESSING ANALYSIS |
|---|---|---|
| WDF | ☑ | ☐ |
| RET | ☐ | ☑ |
| WNR | ☐ | ☑ |
| PLT-F | ☐ | ☑ |
| WPC | ☐ | ☑ |

SET

FIG. 32

POINT WHERE SIGNAL LEVEL IS NOT LESS
THAN PREDETERMINED THRESHOLD

ANALOG SIGNAL

START
POINT

END
POINT

SAMPLING

DIGITAL SIGNAL
(SAMPLING RATE: EXAMPLE OF 1024 POINTS AT 10 NANOSECOND INTERVAL)

INDEX     INDEX     INDEX      INDEX

| 1 | 10 | 15 | ·· | 12 | 7 | 2 | 9 | 13 | ·· | 10 | 9 | 3 | 8 | ·· | 12 | 10 | ·· | N | 6 | ·· | 12 | 9 |

t=0ns 10ns    10240ns   0ns 10ns    10240ns   0ns     10240ns

WAVEFORM DATA    WAVEFORM DATA    WAVEFORM DATA     WAVEFORM DATA

# FIG. 33

DIGITAL SIGNAL OF FORWARD SCATTERED LIGHT

| 10 | 10 | 100 | 200 | 100 | ··· |

← 70a

DIGITAL SIGNAL OF SIDE SCATTERED LIGHT

| 10 | 50 | 200 | 100 | 50 | 10 | ··· |

← 70b

DIGITAL SIGNAL OF FLUORESCENCE

| 10 | 10 | 100 | 200 | 100 | 50 | 10 | ··· |

← 70c

FIG. 34

| CELL TYPE | LABEL VALUE |
|---|---|
| NOT APPLICABLE (NONE) | 0 |
| NEUTROPHIL (NEUT) | 1 |
| LYMPHOCYTE (LYMPH) | 2 |
| MONOCYTE (MONO) | 3 |
| EOSINOPHIL (EO) | 4 |
| BASOPHIL (BASO) | 5 |
| IMMATURE GRANULOCYTE (IG) | 6 |
| ABNORMAL CELL (BLOOD-DERIVED CELL OTHER THAN FIVE CLASSIFICATIONS) | 7 |

# FIG. 35

DIGITAL SIGNAL OF FORWARD SCATTERED LIGHT

| 10 | 10 | 100 | 200 | 100 | ⋯ |

80a

DIGITAL SIGNAL OF SIDE SCATTERED LIGHT

| 10 | 50 | 200 | 100 | 50 | 10 | ⋯ |

80b

DIGITAL SIGNAL OF FLUORESCENCE

| 10 | 10 | 100 | 200 | 100 | 50 | 10 | ⋯ |

80c

<u>85</u>

t=0ns          10240ns

| 10 | 10 | ⋯ | 200 | 100 |

82a

t=0ns          10240ns

| 50 | 200 | ⋯ | 50 | 10 |

82b

t=0ns          10240ns

| 100 | 200 | ⋯ | 50 | 10 |

82c

60

60a    60c    60b

82

| (NEUTROPHIL) | 90% |
| (LYMPHOCYTE) | 5% |

84          83

NEUTROPHIL ← 1 ←

FIG. 36

EXAMPLE IN WHICH AI ANALYSIS IS EXECUTED
ON WAVEFORM DATA OBTAINED IN WDF CHANNEL

```
                        START

          OBTAIN OPTICAL SIGNAL OF WDF CHANNEL AND    S111
          OBTAIN WAVEFORM DATA FROM OPTICAL SIGNAL

                     AI ANALYSIS                      S112

                 PROVIDE ANALYSIS RESULT              S113

                         END
```

FIG. 37

EXAMPLE IN WHICH, ON THE BASIS OF WAVEFORM DATA OBTAINED IN WDF CHANNEL, NUCLEATED RED BLOOD CELL AND BASOPHIL ARE CLASSIFIED THROUGH AI ANALYSIS AND OTHERS ARE CLASSIFIED THROUGH CALCULATION PROCESSING ANALYSIS

```
                    ┌──────────────┐
                    │    START     │
                    └──────────────┘
                           │
                           ▼
        ┌─────────────────────────────────────────┐   S121
        │ OBTAIN OPTICAL SIGNAL OF WDF CHANNEL AND │
        │ OBTAIN WAVEFORM DATA FROM OPTICAL SIGNAL │
        └─────────────────────────────────────────┘
                           │
                           ▼
        ┌─────────────────────────────────────────┐   S122
        │              AI ANALYSIS                 │
        └─────────────────────────────────────────┘
                           │
                           ▼
        ┌─────────────────────────────────────────┐   S123
        │ SPECIFY WAVEFORM DATA THAT CORRESPONDS TO CELL │
        │ CLASSIFIED AS NEITHER NUCLEATED RED BLOOD CELL │
        │                NOR BASOPHIL              │
        └─────────────────────────────────────────┘
                           │
                           ▼
        ┌─────────────────────────────────────────┐   S124
        │   EXECUTE CALCULATION PROCESSING ANALYSIS │
        │        ON SPECIFIED WAVEFORM DATA        │
        └─────────────────────────────────────────┘
                           │
                           ▼
        ┌─────────────────────────────────────────┐   S125
        │         PROVIDE ANALYSIS RESULT          │
        └─────────────────────────────────────────┘
                           │
                           ▼
                    ┌──────────────┐
                    │     END      │
                    └──────────────┘
```

FIG. 38

EXAMPLE IN WHICH AI ANALYSIS IS EXECUTED WITH RESPECT TO
NEUTROPHIL/BASOPHIL SPECIFIED THROUGH CALCULATION PROCESSING
ANALYSIS IN WDF CHANNEL

START

OBTAIN OPTICAL SIGNAL OF WDF CHANNEL AND
OBTAIN WAVEFORM DATA FROM OPTICAL SIGNAL
S131

CALCULATION PROCESSING ANALYSIS
S132

SPECIFY WAVEFORM DATA THAT CORRESPONDS TO CELLS BELOW:

(1) CELL CLASSIFIED AS NONE OF LYMPHOCYTE, MONOCYTE,
    EOSINOPHIL, AND NEUTROPHIL OR BASOPHIL
(2) CELL CLASSIFIED AS NEUTROPHIL OR BASOPHIL
S133

EXECUTE AI ANALYSIS ON SPECIFIED
WAVEFORM DATA
S134

PROVIDE ANALYSIS RESULT
S135

END

FIG. 39

EP 4 246 123 B1

FIG. 40

# FIG. 41

EXAMPLE IN WHICH, IN COAGULATION APPARATUS, NORMAL ANALYSIS IS
PERFORMED THROUGH CALCULATION PROCESSING AND PRESENCE OR ABSENCE
OF NONSPECIFIC REACTION IS ANALYZED THROUGH AI

```
                    START

OBTAIN OPTICAL SIGNAL AND OBTAIN          S141
COAGULATION WAVEFORM DATA FROM OPTICAL
              SIGNAL

    CALCULATION PROCESSING ANALYSIS      S142

    ANALYZE PRESENCE OR ABSENCE OF       S143
    NONSPECIFIC REACTION THROUGH AI

         PROVIDE ANALYSIS RESULT         S144

                     END
```

FIG. 42

EMBODIMENT 6

4000

400

FCM DETECTION PART
410

420 ANALOG PROCESSING PART

461 A/D CONVERTER

300 ANALYSIS UNIT

301 COMPUTER

450 SPECIMEN SUCTION PART

430 APPARATUS MECHANISM PART

440 SAMPLE PREPARATION PART

FIG. 43

FCM DETECTION PART — 410
SPECIMEN SUCTION PART — 450
APPARATUS MECHANISM PART — 430
SAMPLE PREPARATION PART — 440

300

PROCESSOR — 3001
ANALYSIS SOFTWARE — 3100
PARALLEL-PROCESSING PROCESSOR — 3002
BUS CONTROLLER — 3005

BUS — 3003

STORAGE — 3004
RAM — 3017
IF PART — 464
IF PART — 462
IF PART — 3006

A/D CONVERTER — 461
COMPUTER — 301

EP 4 246 123 B1

FIG. 44

FCM DETECTION PART — 410

ANALOG PROCESSING PART — 420

A/D CONVERTER — 462

RBC/PLT DETECTION PART — 4101

ANALOG PROCESSING PART — 4201

A/D CONVERTER — 4611

HGB DETECTION PART — 4102

ANALOG PROCESSING PART — 4202

A/D CONVERTER — 4612

ANALYSIS UNIT — 300

SPECIMEN SUCTION PART — 450

APPARATUS MECHANISM PART — 430

SAMPLE PREPARATION PART — 440

COMPUTER — 301

4000  400

EP 4 246 123 B1

FIG. 45

3200: PLURALITY OF ARITHMETIC UNITS

3002

3200

3201

RAM

FIG. 46

FIG. 47

FIG. 48

FIG. 49

PARALLEL-PROCESSING PROCESSOR — 3400 / 3002

4000  400  300

3001 — PROCESSOR
ANALYSIS SOFTWARE — 3100

467 — IF PART

3005 — BUS CONTROLLER

3003

BUS

3004 — STORAGE

3017 — RAM

464 — IF PART

462 — IF PART

3006 — IF PART

410 — FCM DETECTION PART

450 — SPECIMEN SUCTION PART

430 — APPARATUS MECHANISM PART

440 — SAMPLE PREPARATION PART

461 — A/D CONVERTER

301 — COMPUTER

FIG. 50

# FIG. 51

<u>CALCULATION OF PRODUCT OF MATRIX</u>

$$C_{ij} = \sum_{k=1}^{n} a_{ik} \times b_{kj}$$

```
for(int i=0; i<n; i++){
    for(int j=0; j<n; j++){
        for(int k=0; k<n; k++){
            c[ i ][ j ] += a[ i ][ k ]*b[ k ][ j ]
} } }
```

<u>CALCULATION PROCESSING BY PARALLEL-PROCESSING PROCESSOR</u>

```
for(int k=0; k<n; k++){
c[0][0] += a[0][k]*b[k][0] }
```
```
for(int k=0; k<n; k++){
c[0][1] += a[0][k]*b[k][1] }
```
...

```
for(int k=0; k<n; k++){
c[1][0] += a[1][k]*b[k][0] }
```
```
for(int k=0; k<n; k++){
c[1][1] += a[1][k]*b[k][1] }
```
...

⋮                                        ⋮

FIG. 52

```
for(int k=0; k<n; k++){
c[0][0] += a[0][k]*b[k][0] }
```

```
for(int k=0; k<n; k++){
c[0][1] += a[0][k]*b[k][1] }
```
...

```
for(int k=0; k<n; k++){
c[1][0] += a[1][k]*b[k][0] }
```

```
for(int k=0; k<n; k++){
c[1][1] += a[1][k]*b[k][1] }
```
...

3002

3200: PLURALITY OF ARITHMETIC UNITS

3200

3201

RAM

EP 4 246 123 B1

# FIG. 53

EP 4 246 123 B1

WAVEFORM DATA AND FILTER

FSC

WAVEFORM DATA

| 1 | 1 | 1 | 10 | 30 | 60 | 70 | 60 | 55 | 40 | ... |

FILTER

① | 1 | 0 | 1 |

② | 1 | 1 | 0 |

③ | 1 | 0 | 0 |

...

MATRIX OPERATION BETWEEN WAVEFORM DATA AND FILTER

① | 1 | 0 | 1 |

$\otimes$

| 1 | 1 | 1 | 10 | 30 | 60 | 70 | 60 | 55 | 40 | ... |

① | 1 | 0 | 1 |

$\otimes$

| 1 | 1 | 1 | 10 | 30 | 60 | 70 | 60 | 55 | 40 | ... |

# FIG. 54

ANALYSIS UNIT 300          MEASUREMENT UNIT 400

( START )              ( START )

S200
| MEASUREMENT INSTRUCTION |

S300
| SUCTION AND DISCHARGE SPECIMEN |

S301
| PREPARE MEASUREMENT SAMPLE |

S302
| MEASURE BY DETECTION PART |

S303
| GENERATE WAVEFORM DATA |

( END )

S201
| CLASSIFY ANALYTE IN SPECIMEN |

S202
| ANALYZE CLASSIFICATION RESULT |

S203
| OUTPUT TEST RESULT OF SPECIMEN |

( END )

FIG. 55

```
┌─┬─────────────────────────┬─┐
│ │   CLASSIFY ANALYTE IN   │ │  S201
│ │        SPECIMEN         │ │
└─┴─────────────────────────┴─┘
              │
              ▼
┌─────────────────────────────┐
│    TRANSFER WAVEFORM DATA TO │  S2010
│ PARALLEL-PROCESSING PROCESSOR│
└─────────────────────────────┘
              │
              ▼
┌─┬─────────────────────────┬─┐
│ │ EXECUTE PARALLEL PROCESSING│  S2011
└─┴─────────────────────────┴─┘
              │
              ▼
┌─────────────────────────────┐
│  RECEIVE ARITHMETIC RESULT FROM│  S2012
│ PARALLEL-PROCESSING PROCESSOR│
└─────────────────────────────┘
              │
              ▼
┌─────────────────────────────┐
│    GENERATE ANALYSIS RESULT  │  S2013
└─────────────────────────────┘
              │
              ▼
         (  RETURN  )
```

FIG. 56

```
┌─────────────────────────────────┐
│ │EXECUTE PARALLEL PROCESSING│ │  S2011
└─────────────────────────────────┘
                  │
                  ▼
┌─────────────────────────────────┐
│ ASSIGN ARITHMETIC PROCESSES TO ARITHMETIC │  S2100
│ UNITS OF PARALLEL-PROCESSING PROCESSOR │
└─────────────────────────────────┘
                  │
                  ▼
┌─────────────────────────────────┐
│ ARITHMETIC UNITS ASSIGNED WITH ARITHMETIC │  S2101
│ PROCESSES EXECUTE ARITHMETIC OPERATIONS │
│ IN PARALLEL │
└─────────────────────────────────┘
                  │
                  ▼
┌─────────────────────────────────┐
│ TRANSFER ARITHMETIC RESULT TO RAM │  S2102
└─────────────────────────────────┘
                  │
                  ▼
           ╭─────────────╮
           │   RETURN     │
           ╰─────────────╯
```

FIG. 57

FCM DETECTION PART

ANALOG PROCESSING PART — 420

CONNECTION PORT — 421

— 410

— 4210

400

BUS — 463

SPECIMEN SUCTION PART — 450

APPARATUS MECHANISM PART — 430

SAMPLE PREPARATION PART — 440

IF PART — 464

IF PART — 465

ANALYSIS UNIT — 300

EP 4 246 123 B1

FIG. 58

FIG. 59

FCM DETECTION PART 410

ANALOG PROCESSING PART 420

A/D CONVERTER 461

IF PART 462

BUS 463

IF PART 4631

SPECIMEN SUCTION PART 450

APPARATUS MECHANISM PART 430

SAMPLE PREPARATION PART 440

IF PART 464

IF PART 465

4632

400

ANALYSIS UNIT 300

FIG. 60

FIG. 61

4000

400

600

301

| MEASUREMENT UNIT | ANALYSIS UNIT | COMPUTER |

EP 4 246 123 B1

FIG. 62

FCM DETECTION PART — 410

ANALOG PROCESSING PART — 420

A/D CONVERTER — 461

IF PART — 462

BUS — 463

IF PART — 4631

4632

400

SPECIMEN SUCTION PART — 450

APPARATUS MECHANISM PART — 430

SAMPLE PREPARATION PART — 440

IF PART — 464

IF PART — 465

COMPUTER — 301

ANALYSIS UNIT — 600

EP 4 246 123 B1

FIG. 63

ANALYSIS UNIT 600

PROCESSOR 6001

PARALLEL-PROCESSING PROCESSOR 6002

BUS 6003

STORAGE 6004

RAM 6005

IF PART 6006

IF PART 6007

MEASUREMENT UNIT 400

COMPUTER 301

EP 4 246 123 B1

FIG. 64

ORDER

6001

6100

ANALYSIS SOFTWARE

6002

6200: PLURALITY OF
ARITHMETIC UNITS

6200

6005

RAM

DMA

6201

RAM

EP 4 246 123 B1

FIG. 65

FIG. 66

FCM DETECTION PART 410

ANALOG PROCESSING PART 420

CONNECTION PORT 421

4210

BUS 463

400

SPECIMEN SUCTION PART 450

APPARATUS MECHANISM PART 430

SAMPLE PREPARATION PART 440

IF PART 464

IF PART 465

COMPUTER 301

ANALYSIS UNIT 600

EP 4 246 123 B1

FIG. 67

ANALYSIS UNIT 600

PROCESSOR 6001

PARALLEL-PROCESSING PROCESSOR 6002

BUS 6003

STORAGE 6004

RAM 6005

A/D CONVERTER 6009

IF PART 6007

CONNECTION PORT 6008

MEASUREMENT UNIT 400

COMPUTER 301

EP 4 246 123 B1

FIG. 68

EP 4 246 123 B1

FIG. 69

FIG. 70

EP 4 246 123 B1

FIG. 71

300

PROCESSOR 3001

ANALYSIS SOFTWARE 3100

PARALLEL-PROCESSING PROCESSOR 3002

BUS CONTROLLER 3005

BUS 3003

STORAGE 3004

RAM 3017

IF PART 3010

IF PART 3010

IF PART 3010

IF PART 3006

DIGITAL SIGNAL

MEASUREMENT UNIT 400

EP 4 246 123 B1

FIG. 72

FIG. 73

ANALYSIS UNIT — 600

PROCESSOR — 6001

PARALLEL-PROCESSING PROCESSOR — 6002

BUS — 6003

STORAGE — 6004

RAM — 6005

A/D CONVERTER — 6009

A/D CONVERTER — 6009

A/D CONVERTER — 6009

IF PART — 6007

CONNECTION PORT — 6008

CONNECTION PORT — 6008

CONNECTION PORT — 6008

ANALOG SIGNAL

COMPUTER — 301

EP 4 246 123 B1

FIG. 74

FIG. 75

FIG. 76

FIG. 77

# FIG. 78

STRUCTURE OF NEURAL NETWORK

ARITHMETIC OPERATION AT EACH NODE

ARITHMETIC OPERATION BETWEEN NODES

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018203568 A

- US 2022003745 A1